# EUROPEAN PATENT SPECIFICATION

(11) **EP 3 033 325 B1**
(45) Date of publication and mention of the grant of the patent: **04.12.2019**
(21) Application number: 14829948.0
(22) Date of filing: 22.07.2014
(51) Int. Cl.: C07C 229/12, A61K 47/18, A61K 47/24, A61K 47/28, A61K 48/00, A61K 9/50, C07H 21/02, C12N 15/11, C12N 15/88

(54) **COMPOSITIONS AND METHODS FOR DELIVERING MESSENGER RNA**
ZUSAMMENSETZUNGEN UND VERFAHREN ZUR ABGABE VON BOTEN-RNA
COMPOSITIONS ET PROCÉDÉS POUR L'ADMINISTRATION D'ARN MESSAGER

(30) Priority: 23.07.2013 US 201361857573 P; 24.02.2014 US 201461943856 P
(43) Date of publication of application: 22.06.2016
(73) Proprietor: Arbutus Biopharma Corporation, Burnaby, BC V5J 5J8 (CA)
(72) Inventor: HEYES, James, Burnaby, British Columbia V5J 5J8 (CA); PALMER, Lorne R., Burnaby, British Columbia V5J 5J8 (CA); REID, Stephen P., Burnaby, British Columbia V5J 5J8 (CA); YAWORSKI, Edward D., Burnaby, British Columbia V5J 5J8 (CA); MACLACHLAN, Ian, Burnaby, British Columbia V5J 5J8 (CA); WOOD, Mark, Burnaby, British Columbia V5J 5J8 (CA); MARTIN, Alan D., Burnaby, British Columbia V5J 5J8 (CA)
(74) Representative: Müller-Boré & Partner Patentanwälte PartG mbB
(86) International application number: PCT/IB2014/063289
(87) International publication number: WO 2015/011633

(56) References cited:
- WO-A1-2011/140627
- WO-A1-2011/141705
- WO-A1-2011/143230
- WO-A1-2012/170930
- WO-A1-2013/126803
- WO-A1-2013/126803
- WO-A1-2013/149140
- WO-A1-2013/185069
- WO-A1-2014/089486
- CA-A1- 2 859 387
- US-A1- 2012 172 411
- US-A1- 2012 172 411
- US-A1- 2012 183 581

## Description

Some diseases in human beings are caused by the absence, or impairment, of a functional protein in a cell type where the protein is normally present and active. The functional protein can be completely or partially absent due, for example, to transcriptional inactivity of the encoding gene, or due to the presence of a mutation in the encoding gene that renders the protein completely or partially non-functional.

Examples of human diseases that are caused by complete or partial inactivation of a protein include X-linked severe combined immunodeficiency (X-SCID), and adrenoleukodystrophy (X-ALD). X-SCID is caused by one or more mutations in the gene encoding the common gamma chain protein that is a component of the receptors for several interleukins that are involved in the development and maturation of B and T cells within the immune system. X-ALD is caused by one or more mutations in a peroxisomal membrane transporter protein gene called *ABCD1*. Individuals afflicted with X-ALD have very high levels of long chain fatty acids in tissues throughout the body, which causes a variety of symptoms that may lead to mental impairment or death.

Attempts have been made to use gene therapy to treat some diseases caused by the absence, or impairment, of a functional protein in a cell type where the protein is normally present and active. Gene therapy typically involves introduction of a vector that includes a gene encoding a functional form of the affected protein, into a diseased person, and expression of the functional protein to treat the disease. So far gene therapy has met with limited success.

Thus, there is a continuing need for compositions and methods for expressing a functional form of a protein within a human being who suffers from a disease caused by the complete or partial absence of the functional protein.

US 2012/172411 A1 and WO 2011/141705 A1 disclose compositions and methods for the delivery of therapeutic agents to cells.

WO 2011/143230 A1 describes methods and compositions for delivery of active agents.

WO 2011/140627 A1 discloses nucleic acid-containing lipid particles and related methods.

In accordance with the foregoing, the present invention provides compositions and methods that can be used to express one or more mRNA molecules in a living cell (e.g., cells within a human body). The mRNA molecules encode one or more polypeptides that is/are expressed within the living cells. In some embodiments, the polypeptides are expressed within a diseased organism (e.g., mammal, such as a human being), and expression of the polypeptide ameliorates one or more symptoms of the disease. The compositions and methods of the invention are particularly useful for treating human diseases caused by the absence, or reduced levels, of a functional polypeptide within the human body.

The present invention and embodiments thereof are defined in the claims.

The methods and compositions of the invention can be used, for example, to treat any disease that is caused, at least in part, by the absence of a polypeptide, or the reduced level of a polypeptide, or the expression of a non-functional (or partially functional, or aberrantly functional) form of a polypeptide, in a cell, tissue, and/or organ of a human body.

Other objects, features, and advantages of the present invention will be apparent to one of skill in the art from the following detailed description and figures.
FIGURE 1 shows a cryo TEM image of a population of nucleic acid-lipid particles of the present invention that include mRNA encapsulated within the interior portion of the lipid particles. The particles each have an electron dense core.
FIGURE 2 illustrates a cross-section of a particle in accordance with one or more embodiments.

The nucleic acid-lipid particles, methods, and pharmaceutical formulations, described herein advantageously provide significant new compositions and methods for expressing proteins in a mammalian organism, such as a human being. Embodiments of the present invention can be administered, for example, once per day, once per week, or once every several weeks (*e.g*., once every two, three, four, five or six weeks), or once per month, or once per year. Encapsulation of mRNA within lipid particles confers one or more advantages, such as protecting the mRNA from nuclease degradation in the bloodstream, allowing preferential accumulation of the mRNA in target tissue and providing a means of mRNA entry into the cellular cytoplasm where the mRNA can express the encoded protein.

As used herein, the following terms have the meanings ascribed to them unless specified otherwise.

An "effective amount" or "therapeutically effective amount" of a therapeutic nucleic acid such as an mRNA is an amount sufficient to produce the desired effect, *e.g*., mRNA-directed expression of an amount of a protein that causes a desirable biological effect in the organism within which the protein is expressed. For example, in some embodiments, the expressed protein is an active form of a protein that is normally expressed in a cell type within the body, and the therapeutically effective amount of the mRNA is an amount that produces an amount of the encoded protein that is at least 50% (e.g., at least 60%, or at least 70%, or at least 80%, or at least 90%) of the amount of the protein that is normally expressed in the cell type of a healthy individual. Suitable assays for measuring the expression of an mRNA or protein include, but are not limited to dot blots, Northern blots, *in situ* hybridization, ELISA, immunoprecipitation, enzyme function, as well as phenotypic assays known to those of skill in the art.

By "decrease," "decreasing," "reduce," or "reducing" of an immune response by an mRNA is intended to mean a detectable decrease of an immune response to a given mRNA (*e.g.,* a modified mRNA). The amount of decrease of an immune response by a modified mRNA may be determined relative to the level of an immune response in the presence of an unmodified mRNA. A detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified mRNA. A decrease in the immune response to mRNA is typically measured by a decrease in cytokine production (*e.g.,* IFNγ, IFNα, TNFα, IL-6, or IL-12) by a responder cell *in vitro* or a decrease in cytokine production in the sera of a mammalian subject after administration of the mRNA.

"Substantial identity" refers to a sequence that hybridizes to a reference sequence under stringent conditions, or to a sequence that has a specified percent identity over a specified region of a reference sequence.

The phrase "stringent hybridization conditions" refers to conditions under which a nucleic acid will hybridize to its target sequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization.

Exemplary stringent hybridization conditions can be as follows: 50% formamide, 5x SSC, and 1% SDS, incubating at 42°C, or, 5x SSC, 1% SDS, incubating at 65°C, with wash in 0.2x SSC, and 0.1% SDS at 65°C. For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C-95°C for 30 sec. to 2 min., an annealing phase lasting 30 sec. to 2 min., and an extension phase of about 72°C for 1 to 2 min. Protocols and guidelines for low and high stringency amplification reactions are provided, *e.g.,* in Innis et al., PCR Protocols, A Guide to Methods and Applications, Academic Press, Inc. N.Y. (1990).

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous references, *e.g.,* Current Protocols in Molecular Biology, Ausubel et al., eds.

The terms "substantially identical" or "substantial identity," in the context of two or more nucleic acids, refer to two or more sequences or subsequences that are the same or have a specified percentage of nucleotides that are the same (*i.e.,* at least about 60%, preferably at least about 65%, 70%, 75%, 80%, 85%, 90%, or 95% identity over a specified region), when compared and aligned for maximum correspondence over a comparison window, or designated region as measured using one of the following sequence comparison algorithms or by manual alignment and visual inspection. This definition, when the context indicates, also refers analogously to the complement of a sequence. Preferably, the substantial identity exists over a region that is at least about 5, 10, 15, 20, 25, 30, 35, 40, 45, 50, 55, or 60 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of a number of contiguous positions selected from the group consisting of from about 5 to about 60, usually about 10 to about 45, more usually about 15 to about 30, in which a sequence may be compared to a reference sequence of the same number of contiguous positions after the two sequences are optimally aligned. Methods of alignment of sequences for comparison are well known in the art. Optimal alignment of sequences for comparison can be conducted, *e.g.,* by the local homology algorithm of Smith and Waterman, Adv. Appl. Math., 2:482 (1981), by the homology alignment algorithm of Needleman and Wunsch, J. Mol. Biol., 48:443 (1970), by the search for similarity method of Pearson and Lipman, Proc. Natl. Acad. Sci. USA, 85:2444 (1988), by computerized implementations of these algorithms (GAP, BESALDHIT, FASTA, and ALDHASTA in the Wisconsin Genetics Software Package, Genetics Computer Group, 575 Science Dr., Madison, WI), or by manual alignment and visual inspection (*see, e.g.,* Current Protocols in Molecular Biology, Ausubel et al., eds. (1995 supplement)).

Non-limiting examples of algorithms that are suitable for determining percent sequence identity and sequence similarity are the BLAST and BLAST 2.0 algorithms, which are described in Altschul et al., Nuc. Acids Res., 25:3389-3402 (1977) and Altschul et al., J. Mol. Biol., 215:403-410 (1990), respectively. BLAST and BLAST 2.0 are used, with the parameters described herein, to determine percent sequence identity for the nucleic acids of the invention. Software for performing BLAST analyses is publicly available through the National Center for Biotechnology Information (http://www.ncbi.nlm.nih.gov/). Another example is a global alignment algorithm for determining percent sequence identity such as the Needleman-Wunsch algorithm for aligning protein or nucleotide (*e.g*., RNA) sequences.

The BLAST algorithm also performs a statistical analysis of the similarity between two sequences (*see, e.g.,* Karlin and Altschul, Proc. Natl. Acad. Sci. USA, 90:5873-5787 (1993)). One measure of similarity provided by the BLAST algorithm is the smallest sum probability (P(N)), which provides an indication of the probability by which a match between two nucleotide sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "nucleic acid" as used herein refers to a polymer containing at least two deoxyribonucleotides or ribonucleotides in either single- or double-stranded form and includes DNA and RNA. DNA may be in the form of, *e.g*., antisense molecules, plasmid DNA, pre-condensed DNA, a PCR product, vectors (e.g., P1, PAC, BAC, YAC, artificial chromosomes), expression cassettes, chimeric sequences, chromosomal DNA, or derivatives and combinations of these groups. RNA may be in the form of small interfering RNA (siRNA), Dicer-substrate dsRNA, small hairpin RNA (shRNA), asymmetrical interfering RNA (aiRNA), microRNA (miRNA), mRNA, tRNA, rRNA, tRNA, viral RNA (vRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (*e.g*., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)).

"Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (*e.g.,* DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

The term "lipid particle" includes a lipid formulation that can be used to deliver a therapeutic mRNA to a target site of interest (*e.g*., cell, tissue, organ, and the like). In preferred embodiments, the lipid particle of the invention is a nucleic acid-lipid particle, which is typically formed from a cationic lipid, a non-cationic lipid (e.g., a phospholipid), a conjugated lipid that prevents aggregation of the particle (e.g., a PEG-lipid), and optionally cholesterol. Typically, the therapeutic mRNA may be encapsulated in the lipid portion of the particle, thereby protecting it from enzymatic degradation.

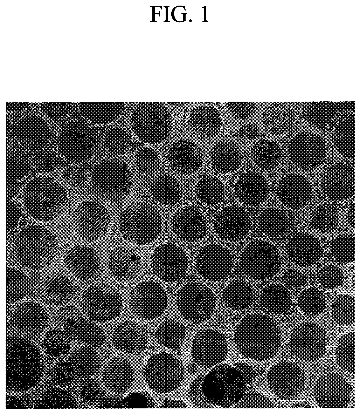

The term "electron dense core", when used to describe a lipid particle of the present invention, refers to the dark appearance of the interior portion of a lipid particle when visualized using cryo transmission electron microscopy ("cryoTEM"). An example of a lipid particle of the present invention having an electron dense core is shown in FIGURE 1. Some lipid particles of the present invention have an electron dense core and lack a lipid bilayer structure. Some lipid particles of the present invention have an electron dense core, lack a lipid bilayer structure, and have an inverse Hexagonal or Cubic phase structure. While not wishing to be bound by theory, it is thought that the non-bilayer lipid packing provides a 3 - dimensional network of lipid cylinders with water and nucleic on the inside, i.e., essentially, a lipid droplet interpenetrated with aqueous channels containg the nucleic acid.

As used herein, the term "SNALP" refers to a stable nucleic acid-lipid particle. A SNALP is a particle made from lipids (*e.g.,* a cationic lipid, a non-cationic lipid, and a conjugated lipid that prevents aggregation of the particle), wherein the nucleic acid (*e.g.,* mRNA) is fully encapsulated within the lipid. In certain instances, SNALP are extremely useful for systemic applications, as they can exhibit extended circulation lifetimes following intravenous (i.v.) injection, they can accumulate at distal sites (*e.g*., sites physically separated from the administration site), and they can mediate mRNA expression at these distal sites. The nucleic acid may be complexed with a condensing agent and encapsulated within a SNALP as set forth in PCT Publication No. WO 00/03683.

The lipid particles of the invention (e.g., SNALP) typically have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, and are substantially non-toxic. In addition, nucleic acids, when present in the lipid particles of the present invention, are resistant in aqueous solution to degradation with a nuclease. Nucleic acid-lipid particles and their method of preparation are disclosed in, *e.g.,* U.S. Patent Publication Nos. 20040142025 and 20070042031.

As used herein, "lipid encapsulated" can refer to a lipid particle that provides a therapeutic mRNA with full encapsulation, partial encapsulation, or both. In a preferred embodiment, the mRNA is fully encapsulated in the lipid particle (*e.g.,* to form a SNALP or other nucleic acid-lipid particle).

The term "lipid conjugate" refers to a conjugated lipid that inhibits aggregation of lipid particles. Such lipid conjugates include, but are not limited to, PEG-lipid conjugates such as, *e.g.,* PEG coupled to dialkyloxypropyls (*e.g.,* PEG-DAA conjugates), PEG coupled to diacylglycerols (*e.g*., PEG-DAG conjugates), PEG coupled to cholesterol, PEG coupled to phosphatidylethanolamines, and PEG conjugated to ceramides (*see, e.g.,* U.S. Patent No. 5,885,613), cationic PEG lipids, polyoxazoline (POZ)-lipid conjugates, polyamide oligomers (*e.g*., ATTA-lipid conjugates), and mixtures thereof. Additional examples of POZ-lipid conjugates are described in PCT Publication No. WO 2010/006282. PEG or POZ can be conjugated directly to the lipid or may be linked to the lipid via a linker moiety. Any linker moiety suitable for coupling the PEG or the POZ to a lipid can be used including, *e.g*., non-ester containing linker moieties and ester-containing linker moieties. In certain preferred embodiments, non-ester containing linker moieties, such as amides or carbamates, are used.

The term "amphipathic lipid" refers, in part, to any suitable material wherein the hydrophobic portion of the lipid material orients into a hydrophobic phase, while the hydrophilic portion orients toward the aqueous phase. Hydrophilic characteristics derive from the presence of polar or charged groups such as carbohydrates, phosphate, carboxylic, sulfato, amino, sulfhydryl, nitro, hydroxyl, and other like groups. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). Examples of amphipathic compounds include, but are not limited to, phospholipids, aminolipids, and sphingolipids.

Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine. Other compounds lacking in phosphorus, such as sphingolipid, glycosphingolipid families, diacylglycerols, and β-acyloxyacids, are also within the group designated as amphipathic lipids. Additionally, the amphipathic lipids described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides, and diacylglycerols.

The term "non-cationic lipid" refers to any amphipathic lipid as well as any other neutral lipid or anionic lipid.

The term "anionic lipid" refers to any lipid that is negatively charged at physiological pH. These lipids include, but are not limited to, phosphatidylglycerols, cardiolipins, diacylphosphatidylserines, diacylphosphatidic acids, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

The term "hydrophobic lipid" refers to compounds having apolar groups that include, but are not limited to, long-chain saturated and unsaturated aliphatic hydrocarbon groups and such groups optionally substituted by one or more aromatic, cycloaliphatic, or heterocyclic group(s). Suitable examples include, but are not limited to, diacylglycerol, dialkylglycerol, N-N-dialkylamino, 1,2-diacyloxy-3-aminopropane, and 1,2-dialkyl-3-aminopropane.

The terms "cationic lipid" and "amino lipid" are used interchangeably herein to include those lipids and salts thereof having one, two, three, or more fatty acid or fatty alkyl chains and a pH-titratable amino head group (*e.g*., an alkylamino or dialkylamino head group). The cationic lipid is typically protonated (*i.e.,* positively charged) at a pH below the pKₐ of the cationic lipid and is substantially neutral at a pH above the pKₐ. The cationic lipids of the invention may also be termed titratable cationic lipids. In some embodiments, the cationic lipids comprise: a protonatable tertiary amine (*e.g*., pH-titratable) head group; C₁₈ alkyl chains, wherein each alkyl chain independently has 0 to 3 (*e.g.,* 0, 1, 2, or 3) double bonds; and ether, ester, or ketal linkages between the head group and alkyl chains. General cationic lipids include DSDMA, DODMA, DLinDMA, DLenDMA, γ-DLenDMA, DLin-K-DMA, DLin-K-C2-DMA (also known as DLin-C2K-DMA, XTC2, and C2K), DLin-K-C3-DMA, DLin-K-C4-DMA, DLen-C2K-DMA, γ-DLen-C2K-DMA, DLin-M-C2-DMA (also known as MC2), DLin-M-C3-DMA (also known as MC3) and (DLin-MP-DMA)(also known as 1-B11).

The term "alkylamino" includes a group of formula -N(H)R, wherein R is an alkyl as defined herein.

The term "dialkylamino" includes a group of formula -NR₂, wherein each R is independently an alkyl as defined herein.

The term "salts" includes any anionic and cationic complex, such as the complex formed between a cationic lipid and one or more anions. Non-limiting examples of anions include inorganic and organic anions, *e.g*., hydride, fluoride, chloride, bromide, iodide, oxalate (*e.g*., hemioxalate), phosphate, phosphonate, hydrogen phosphate, dihydrogen phosphate, oxide, carbonate, bicarbonate, nitrate, nitrite, nitride, bisulfite, sulfide, sulfite, bisulfate, sulfate, thiosulfate, hydrogen sulfate, borate, formate, acetate, benzoate, citrate, tartrate, lactate, acrylate, polyacrylate, fumarate, maleate, itaconate, glycolate, gluconate, malate, mandelate, tiglate, ascorbate, salicylate, polymethacrylate, perchlorate, chlorate, chlorite, hypochlorite, bromate, hypobromite, iodate, an alkylsulfonate, an arylsulfonate, arsenate, arsenite, chromate, dichromate, cyanide, cyanate, thiocyanate, hydroxide, peroxide, permanganate, and mixtures thereof. In particular embodiments, the salts of the cationic lipids disclosed herein are crystalline salts.

The term "alkyl" includes a straight chain or branched, noncyclic or cyclic, saturated aliphatic hydrocarbon containing from 1 to 24 carbon atoms. Representative saturated straight chain alkyls include, but are not limited to, methyl, ethyl, *n*-propyl, *n*-butyl, *n*-pentyl, *n*-hexyl, and the like, while saturated branched alkyls include, without limitation, isopropyl, *sec*-butyl, isobutyl, *tert*-butyl, isopentyl, and the like. Representative saturated cyclic alkyls include, but are not limited to, cyclopropyl, cyclobutyl, cyclopentyl, cyclohexyl, and the like, while unsaturated cyclic alkyls include, without limitation, cyclopentenyl, cyclohexenyl, and the like.

The term "alkenyl" includes an alkyl, as defined above, containing at least one double bond between adjacent carbon atoms. Alkenyls include both *cis* and *trans* isomers. Representative straight chain and branched alkenyls include, but are not limited to, ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

The term "alkynyl" includes any alkyl or alkenyl, as defined above, which additionally contains at least one triple bond between adjacent carbons. Representative straight chain and branched alkynyls include, without limitation, acetylenyl, propynyl, 1-butynyl, 2-butynyl, 1-pentynyl, 2-pentynyl, 3-methyl-1 butynyl, and the like.

The term "acyl" includes any alkyl, alkenyl, or alkynyl wherein the carbon at the point of attachment is substituted with an oxo group, as defined below. The following are non-limiting examples of acyl groups: -C(=O)alkyl, -C(=O)alkenyl, and -C(=O)alkynyl.

The term "heterocycle" includes a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include, but are not limited to, heteroaryls as defined below, as well as morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" mean that, when substituted, at least one hydrogen atom is replaced with a substituent. In the case of an oxo substituent (=O), two hydrogen atoms are replaced. In this regard, substituents include, but are not limited to, oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, R^{x} and R^{y} are the same or different and are independently hydrogen, alkyl, or heterocycle, and each of the alkyl and heterocycle substituents may be further substituted with one or more of oxo, halogen, -OH, -CN, alkyl, -OR^{x}, heterocycle, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}. The term "optionally substituted," when used before a list of substituents, means that each of the substituents in the list may be optionally substituted as described herein.

The term "halogen" includes fluoro, chloro, bromo, and iodo.

The term "fusogenic" refers to the ability of a lipid particle, such as a SNALP, to fuse with the membranes of a cell. The membranes can be either the plasma membrane or membranes surrounding organelles, *e.g.,* endosome, nucleus, *etc.*

As used herein, the term "aqueous solution" refers to a composition comprising in whole, or in part, water.

As used herein, the term "organic lipid solution" refers to a composition comprising in whole, or in part, an organic solvent having a lipid.

"Distal site," as used herein, refers to a physically separated site, which is not limited to an adjacent capillary bed, but includes sites broadly distributed throughout an organism.

"Serum-stable" in relation to nucleic acid-lipid particles such as SNALP means that the particle is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of lipid particles that leads to a broad biodistribution of an active agent such as an mRNA within an organism. Some techniques of administration can lead to the systemic delivery of certain agents, but not others. Systemic delivery means that a useful, preferably therapeutic, amount of an agent is exposed to most parts of the body. To obtain broad biodistribution generally requires a blood lifetime such that the agent is not rapidly degraded or cleared (such as by first pass organs (liver, lung, *etc*.) or by rapid, nonspecific cell binding) before reaching a disease site distal to the site of administration. Systemic delivery of lipid particles can be by any means known in the art including, for example, intravenous, subcutaneous, and intraperitoneal. In a preferred embodiment, systemic delivery of lipid particles is by intravenous delivery.

"Local delivery," as used herein, refers to delivery of an active agent such as an mRNA directly to a target site within an organism. For example, an agent can be locally delivered by direct injection into a disease site, other target site, or a target organ such as the liver, heart, pancreas, kidney, and the like.

The term "mammal" refers to any mammalian species such as a human, mouse, rat, dog, cat, hamster, guinea pig, rabbit, livestock, and the like.

When used herein to describe the ratio of lipid:mRNA, the term "lipid" refers to the total lipid in the particle.

Unless stated otherwise herein, the term "about", when used in connection with a value or range of values, means plus or minus 5% of the stated value or range of values.

In one aspect, the present invention provides nucleic acid-lipid particles that each include a lipid particle comprising a cationic lipid, a non-cationic lipid; and an mRNA molecule encapsulated within the lipid particle. Typically, a population of mRNA molecules is encapsulated within the lipid particle. The lipid particles typically include an outer layer defining an interior portion, wherein the mRNA molecule(s) is located within the interior portion. The mRNA molecule(s) is typically completely encapsulated within the lipid particle. The lipid particles can be spherical or non-spherical. The lipid particles can have an electron dense core when visualized using cryo TEM. Typically, the electron dense core is mainly composed of lipid, although aqueous material may be present in an amount that is less than the amount of the lipid.

In one embodiment, the present invention provides a lipid particle comprising a PEG lipid, a non-cationic lipid, a tetra alkyl cationic lipid, and an mRNA; wherein the lipid particle has an electron dense core and the mRNA is encapsulated within the electron dense core.

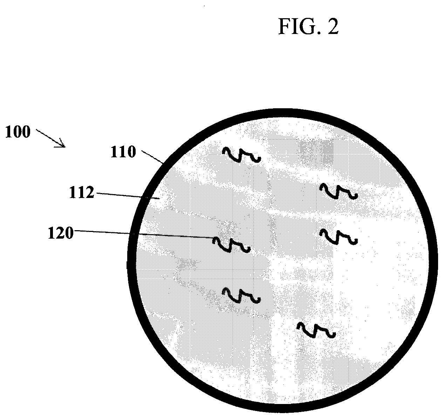

FIG. 2 shows a particle 100 having an outer lipid layer 110 that encapsulates an electron dense core 112 having mRNA molecules 120 therein. The particle is delivered to a mammal and used to deliver the mRNA molecules to living cells within the mammal.

In some embodiments of the invention the lipid particles include (a) a lipid particle comprising a cationic lipid, a PEG-lipid, and a phospholipid; and (b) an mRNA molecule, wherein the mRNA molecule is encapsulated within the lipid particle. The lipid particles can optionally include cholesterol. The mRNA can be completely or partially encapsulated within the lipid particle.

The formation of the particle 100 includes, in one or more embodiments, disposing a lipid into a first fluid, such as ethanol, disposing mRNA into a second fluid, such as an aqueous buffer, and mixing the first and second fluids under controlled conditions to form particle 100. The resulting particle 100 includes an electron dense core within the lipid particle when viewed by Cryo Transmission Electron Microscopy.

### mRNA

mRNA useful in the practice of the present invention may comprise at least one, two, three, four, five, six, seven, eight, nine, ten, or more modified nucleotides such as 2'OMe nucleotides. Preferably, uridine and/or guanosine nucleotides in the mRNA are modified with 2'OMe nucleotides. In some embodiments, the mRNA may further comprise modified (*e.g.,* 2'OMe-modified) adenosine and/or modified (*e.g.,* 2'OMe-modified) cytosine nucleotides.

In one aspect, the present invention provides a nucleic acid-lipid particle (*e.g*., SNALP) that includes an mRNA. The nucleic acid-lipid particles (*e.g*., SNALP) typically comprise one or more (*e.g.,* a cocktail) mRNA(s), a cationic lipid, and a non-cationic lipid. In certain instances, the nucleic acid-lipid particles (*e.g*., SNALP) further comprise a conjugated lipid that inhibits aggregation of particles. Preferably, the nucleic acid-lipid particles (*e.g.,* SNALP) comprise one or more (*e.g.,* a cocktail) mRNAs, a cationic lipid, a non-cationic lipid, and a conjugated lipid that inhibits aggregation of particles.

In some embodiments, the mRNA(s) are fully encapsulated in the nucleic acid-lipid particle (*e.g*., SNALP). With respect to formulations comprising an mRNA cocktail, the different types of mRNA species present in the cocktail (*e.g*., mRNA having different sequences) may be co-encapsulated in the same particle, or each type of mRNA species present in the cocktail may be encapsulated in a separate particle. The mRNA cocktail may be formulated in the particles described herein using a mixture of two or more individual mRNAs (each having a unique sequence) at identical, similar, or different concentrations or molar ratios. In one embodiment, a cocktail of mRNAs (corresponding to a plurality of mRNAs with different sequences) is formulated using identical, similar, or different concentrations or molar ratios of each mRNA species, and the different types of mRNAs are co-encapsulated in the same particle. In another embodiment, each type of mRNA species present in the cocktail is encapsulated in different particles at identical, similar, or different mRNA concentrations or molar ratios, and the particles thus formed (each containing a different mRNA payload) are administered separately (*e.g*., at different times in accordance with a therapeutic regimen), or are combined and administered together as a single unit dose (*e.g*., with a pharmaceutically acceptable carrier). The particles described herein are serum-stable, are resistant to nuclease degradation, and are substantially non-toxic to mammals such as humans.

The cationic lipid in the nucleic acid-lipid particles of the invention (*e.g*., SNALP) is the cationic lipid of the present invention described herein. A general cationic lipid is selected from the group consisting of 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (γ-DLenDMA), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), dilinoleylmethyl-3-dimethylaminopropionate (DLin-M-C2-DMA), (6Z,9Z,28Z,31Z)-heptatriaconta-6,9,28,31-tetraen-19-yl 4-(dimethylamino)butanoate (DLin-M-C3-DMA), salts thereof, and mixtures thereof.

The non-cationic lipid in the nucleic acid-lipid particles of the present invention (*e.g*., SNALP) may comprise, *e.g*., one or more anionic lipids and/or neutral lipids. In some embodiments, the non-cationic lipid comprises one of the following neutral lipid components: (1) a mixture of a phospholipid and cholesterol or a derivative thereof; (2) cholesterol or a derivative thereof; or (3) a phospholipid. In certain preferred embodiments, the phospholipid comprises dipalmitoylphosphatidylcholine (DPPC), distearoylphosphatidylcholine (DSPC), or a mixture thereof. In a particularly preferred embodiment, the non-cationic lipid is a mixture of DPPC and cholesterol.

The lipid conjugate in the nucleic acid-lipid particles of the invention (*e.g*., SNALP) inhibits aggregation of particles and may comprise, *e.g*., one or more of the lipid conjugates described herein. In one particular embodiment, the lipid conjugate comprises a PEG-lipid conjugate. Examples of PEG-lipid conjugates include, but are not limited to, PEG-DAG conjugates, PEG-DAA conjugates, and mixtures thereof. In certain embodiments, the PEG-DAA conjugate in the lipid particle may comprise a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, a PEG-distearyloxypropyl (C₁₈) conjugate, or mixtures thereof. In another embodiment, the lipid conjugate comprises a POZ-lipid conjugate such as a POZ-DAA conjugate.

In some embodiments, the present invention provides nucleic acid-lipid particles (*e.g*., SNALP) comprising: (a) one or more (*e.g.,* a cocktail) mRNA molecule(s) that each encode a protein; (b) one or more cationic lipids or salts thereof comprising from about 50 mol % to about 85 mol % of the total lipid present in the particle; (c) one or more non-cationic lipids comprising from about 13 mol % to about 49.5 mol % of the total lipid present in the particle; and (d) one or more conjugated lipids that inhibit aggregation of particles comprising from about 0.5 mol % to about 2 mol % of the total lipid present in the particle.

In one aspect of this embodiment, the nucleic acid-lipid particle comprises: (a) one or more (*e.g.,* a cocktail) mRNA molecule(s) that each encode a protein; (b) a cationic lipid or a salt thereof comprising from about 52 mol % to about 62 mol % of the total lipid present in the particle; (c) a mixture of a phospholipid and cholesterol or a derivative thereof comprising from about 36 mol % to about 47 mol % of the total lipid present in the particle; and (d) a PEG-lipid conjugate comprising from about 1 mol % to about 2 mol % of the total lipid present in the particle. This embodiment of nucleic acid-lipid particle is generally referred to herein as the "1:57" formulation. In one particular embodiment, the 1:57 formulation is a four-component system comprising about 1.4 mol % PEG-lipid conjugate (*e.g*., PEG2000-C-DMA), about 57.1 mol % cationic lipid or a salt thereof, about 7.1 mol % DPPC (or DSPC), and about 34.3 mol % cholesterol (or derivative thereof).

In another aspect of this embodiment, the nucleic acid-lipid particle comprises: (a) one or more (*e.g*., a cocktail) mRNA molecule(s) that each encode a protein; (b) a cationic lipid or a salt thereof comprising from about 56.5 mol % to about 66.5 mol % of the total lipid present in the particle; (c) cholesterol or a derivative thereof comprising from about 31.5 mol % to about 42.5 mol % of the total lipid present in the particle; and (d) a PEG-lipid conjugate comprising from about 1 mol % to about 2 mol % of the total lipid present in the particle. This embodiment of nucleic acid-lipid particle is generally referred to herein as the "1:62" formulation. In one particular embodiment, the 1:62 formulation is a three-component system which is phospholipid-free and comprises about 1.5 mol % PEG-lipid conjugate (*e.g*., PEG2000-C-DMA), about 61.5 mol % cationic lipid or a salt thereof, and about 36.9 mol % cholesterol (or derivative thereof).

Additional embodiments related to the 1:57 and 1:62 formulations are described in PCT Publication No. WO 09/127060 and published US patent application publication number US 2011/0071208 A1.

In other embodiments, the present invention provides nucleic acid-lipid particles (*e.g.*, SNALP) comprising: (a) one or more (*e.g.,* a cocktail) mRNA molecule(s) that each encode a protein; (b) one or more cationic lipids or salts thereof comprising from about 2 mol % to about 50 mol % of the total lipid present in the particle; (c) one or more non-cationic lipids comprising from about 5 mol % to about 90 mol % of the total lipid present in the particle; and (d) one or more conjugated lipids that inhibit aggregation of particles comprising from about 0.5 mol % to about 20 mol % of the total lipid present in the particle.

In one aspect of this embodiment, the nucleic acid-lipid particle comprises: (a) one or more (*e.g.,* a cocktail) mRNA molecule(s) that each encode a protein; (b) a cationic lipid or a salt thereof comprising from about 30 mol % to about 50 mol % of the total lipid present in the particle; (c) a mixture of a phospholipid and cholesterol or a derivative thereof comprising from about 47 mol % to about 69 mol % of the total lipid present in the particle; and (d) a PEG-lipid conjugate comprising from about 1 mol % to about 3 mol % of the total lipid present in the particle. This embodiment of nucleic acid-lipid particle is generally referred to herein as the "2:40" formulation. In one particular embodiment, the 2:40 formulation is a four-component system which comprises about 2 mol % PEG-lipid conjugate (*e.g*., PEG2000-C-DMA), about 40 mol % cationic lipid or a salt thereof, about 10 mol % DPPC (or DSPC), and about 48 mol % cholesterol (or derivative thereof).

In further embodiments, the present invention provides nucleic acid-lipid particles (*e.g.,* SNALP) comprising: (a) one or more (*e.g.,* a cocktail) mRNA molecule(s) that each encode a protein; (b) one or more cationic lipids or salts thereof comprising from about 50 mol % to about 65 mol % of the total lipid present in the particle; (c) one or more non-cationic lipids comprising from about 25 mol % to about 45 mol % of the total lipid present in the particle; and (d) one or more conjugated lipids that inhibit aggregation of particles comprising from about 5 mol % to about 10 mol % of the total lipid present in the particle.

In one aspect of this embodiment, the nucleic acid-lipid particle comprises: (a) one or more (*e.g.,* a cocktail) mRNA molecule(s) that each encode a protein; (b) a cationic lipid or a salt thereof comprising from about 50 mol % to about 60 mol % of the total lipid present in the particle; (c) a mixture of a phospholipid and cholesterol or a derivative thereof comprising from about 35 mol % to about 45 mol % of the total lipid present in the particle; and (d) a PEG-lipid conjugate comprising from about 5 mol % to about 10 mol % of the total lipid present in the particle. This embodiment of nucleic acid-lipid particle is generally referred to herein as the "7:54" formulation. In certain instances, the non-cationic lipid mixture in the 7:54 formulation comprises: (i) a phospholipid of from about 5 mol % to about 10 mol % of the total lipid present in the particle; and (ii) cholesterol or a derivative thereof of from about 25 mol % to about 35 mol % of the total lipid present in the particle. In one particular embodiment, the 7:54 formulation is a four-component system which comprises about 7 mol % PEG-lipid conjugate (*e.g*., PEG750-C-DMA), about 54 mol % cationic lipid or a salt thereof, about 7 mol % DPPC (or DSPC), and about 32 mol % cholesterol (or derivative thereof).

In another aspect of this embodiment, the nucleic acid-lipid particle comprises: (a) one or more (*e.g*., a cocktail) mRNA molecule(s) that each encode a protein; (b) a cationic lipid or a salt thereof comprising from about 55 mol % to about 65 mol % of the total lipid present in the particle; (c) cholesterol or a derivative thereof comprising from about 30 mol % to about 40 mol % of the total lipid present in the particle; and (d) a PEG-lipid conjugate comprising from about 5 mol % to about 10 mol % of the total lipid present in the particle. This embodiment of nucleic acid-lipid particle is generally referred to herein as the "7:58" formulation. In one particular embodiment, the 7:58 formulation is a three-component system which is phospholipid-free and comprises about 7 mol % PEG-lipid conjugate (*e.g.*, PEG750-C-DMA), about 58 mol % cationic lipid or a salt thereof, and about 35 mol % cholesterol (or derivative thereof).

Additional embodiments related to the 7:54 and 7:58 formulations are described in published US patent application publication number US 2011/0076335 A1.

The present invention also provides pharmaceutical compositions comprising a nucleic acid-lipid particle such as a SNALP and a pharmaceutically acceptable carrier.

The nucleic acid-lipid particles of the present invention (*e.g*., SNALP) are useful for the therapeutic delivery of mRNAs that express one or more proteins (such as full length proteins, or biologically active fragments of full length proteins). In some embodiments, a cocktail of mRNAs that express different proteins is formulated into the same or different nucleic acid-lipid particles, and the particles are administered to a mammal (*e.g*., a human) requiring such treatment. In certain instances, a therapeutically effective amount of the nucleic acid-lipid particles can be administered to the mammal.

In certain embodiments, the present invention provides a lipid particle for use in a method for introducing one or more mRNA molecules into a cell by contacting the cell with a nucleic acid-lipid particle described herein (*e.g*., a SNALP formulation). In one particular embodiment, the cell is a reticuloendothelial cell (*e.g*., monocyte or macrophage), fibroblast cell, endothelial cell, or platelet cell.

In some embodiments, the nucleic acid-lipid particles described herein (*e.g*., SNALP) are administered by one of the following routes of administration: oral, intranasal, intravenous, intraperitoneal, intramuscular, intra-articular, intralesional, intratracheal, subcutaneous, and intradermal. In particular embodiments, the nucleic acid-lipid particles are administered systemically, *e.g*., via enteral or parenteral routes of administration.

In particular embodiments, the nucleic acid-lipid particles of the invention (*e.g*., SNALP) can preferentially deliver a payload such as an mRNA to the liver as compared to other tissues.

In certain aspects, the present invention provides a lipid particle for use in methods for expressing a protein in a mammal (*e.g*., human) in need thereof, the method comprising administering to the mammal a therapeutically effective amount of a nucleic acid-lipid particle (*e.g*., a SNALP formulation) comprising one or more mRNAs that encode one or more proteins under conditions that enable expression of the protein(s) in the mammal. For example, in embodiments in which the mRNA encodes a protein that is normally expressed in a healthy mammalian subject, the level of expression of the protein encoded by the mRNA encapsulated within the SNALP is at least 10%, or at least 20%, or at least 30%, or at least 40%, or at least 50%, or at least 60%, or at least 70%, or at least 80%, or at least 90%, or at least 100%, or greater than 100%, of the level of the protein that is normally expressed in a healthy mammalian subject.

In other aspects, the present invention provides a lipid particle for use in methods for treating, preventing, reducing the risk or likelihood of developing (*e.g*., reducing the susceptibility to), delaying the onset of, and/or ameliorating one or more symptoms associated with a disease in a mammal (*e.g*., human) in need thereof, wherein the disease is caused (at least in part) by reduced or aberrant expression of a protein. The methods each include the step of administering to the mammal a therapeutically effective amount of a nucleic acid-lipid particle (*e.g.,* a SNALP formulation) comprising one or more mRNA molecules that encode a protein that is absent, or present at reduced levels, within the treated subject.

mRNA molecules useful in the present invention may be chemically modified or unmodified. Typically mRNA molecules are chemically modified in order to reduce their ability to induce the innate immune response of a cell into which the mRNA is introduced.

### Modifications to mRNA

mRNA used in the practice of the present invention can include one, two, or more than two nucleoside modifications. In some embodiments, the modified mRNA exhibits reduced degradation in a cell into which the mRNA is introduced, relative to a corresponding unmodified mRNA.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihy drouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In specific embodiments, a modified nucleoside is 5'-0-(1-Thiophosphate)-Adenosine, 5'-0-(1-Thiophosphate)-Cytidine, 5'-0-(1-Thiophosphate)-Guanosine, 5'-0-( 1 -Thiophosphate)-Uridine or 5'-0-(1-Thiophosphate)-Pseudouridine. The α-thio substituted phosphate moiety is provided to confer stability to RNA polymers through the unnatural phosphorothioate backbone linkages. Phosphorothioate RNA have increased nuclease resistance and subsequently a longer half-life in a cellular environment. Phosphorothioate linked nucleic acids are expected to also reduce the innate immune response through weaker binding/activation of cellular innate immune molecules.

In certain embodiments it is desirable to intracellularly degrade a modified nucleic acid introduced into the cell, for example if precise timing of protein production is desired. Thus, the invention provides a modified nucleic acid containing a degradation domain, which is capable of being acted on in a directed manner within a cell.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1 -methyl-6-thio-guanosine, N2-methyl-6-thio-guanosine, and N2,N2-dimethyl-6-thio-guanosine.

### Optional Components of the Modified Nucleic Acids

In further embodiments, the modified nucleic acids may include other optional components, which can be beneficial in some embodiments. These optional components include, but are not limited to, untranslated regions, kozak sequences, intronic nucleotide sequences, internal ribosome entry site (IRES), caps and polyA tails. For example, a 5' untranslated region (UTR) and/or a 3' UTR may be provided, wherein either or both may independently contain one or more different nucleoside modifications. In such embodiments, nucleoside modifications may also be present in the translatable region. Also provided are nucleic acids containing a Kozak sequence.

Additionally, provided are nucleic acids containing one or more intronic nucleotide sequences capable of being excised from the nucleic acid.

### Untranslated Regions (UTRs)

Untranslated regions (UTRs) of a gene are transcribed but not translated. The 5'UTR starts at the transcription start site and continues to the start codon but does not include the start codon; whereas, the 3'UTR starts immediately following the stop codon and continues until the transcriptional termination signal. There is growing body of evidence about the regulatory roles played by the UTRs in terms of stability of the nucleic acid molecule and translation. The regulatory features of a UTR can be incorporated into the mRNA used in the present invention to increase the stability of the molecule. The specific features can also be incorporated to ensure controlled down-regulation of the transcript in case they are misdirected to undesired organs sites.

### 5 ' Capping

The 5' cap structure of an mRNA is involved in nuclear export, increasing mRNA stability and binds the mRNA Cap Binding Protein (CBP), which is responsible for mRNA stability in the cell and translation competency through the association of CBP with poly(A) binding protein to form the mature cyclic mRNA species. The cap further assists the removal of 5' proximal introns removal during mRNA splicing.

Endogenous mRNA molecules may be 5'-end capped generating a 5'-ppp-5'-triphosphate linkage between a terminal guanosine cap residue and the 5'-terminal transcribed sense nucleotide of the mRNA molecule. This 5'-guanylate cap may then be methylated to generate an N7-methyl-guanylate residue. The ribose sugars of the terminal and/or anteterminal transcribed nucleotides of the 5' end of the mRNA may optionally also be 2'-0-methylated. 5'-decapping through hydrolysis and cleavage of the guanylate cap structure may target a nucleic acid molecule, such as an mRNA molecule, for degradation. IRES Sequences

mRNA containing an internal ribosome entry site (IRES) are also useful in the practice of the present invention. An IRES may act as the sole ribosome binding site, or may serve as one of multiple ribosome binding sites of an mRNA. An mRNA containing more than one functional ribosome binding site may encode several peptides or polypeptides that are translated independently by the ribosomes ("multicistronic mRNA"). When mRNA are provided with an IRES, further optionally provided is a second translatable region. Examples of IRES sequences that can be used according to the invention include without limitation, those from picornaviruses (e.g. FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (S1V) or cricket paralysis viruses (CrPV).

### Poly-A tails

During RNA processing, a long chain of adenine nucleotides (poly-A tail) may be added to a polynucleotide such as an mRNA molecules in order to increase stability. Immediately after transcription, the 3' end of the transcript may be cleaved to free a 3' hydroxyl. Then poly-A polymerase adds a chain of adenine nucleotides to the RNA. The process, called polyadenylation, adds a poly-A tail that can be between 100 and 250 residues long.

Generally, the length of a poly-A tail is greater than 30 nucleotides in length. In another embodiment, the poly-A tail is greater than 35 nucleotides in length (e.g., at least or greater than about 35, 40, 45, 50, 55, 60, 70, 80, 90, 100, 120, 140, 160, 180, 200, 250, 300, 350, 400, 450, 500, 600, 700, 800, 900, 1000, 1100, 1200, 1300, 1400, 1500, 1600, 1700, 1800, 1900, 2,000, 2,500, and 3,000 nucleotides).

In this context the poly-A tail may be 10, 20, 30, 40, 50, 60, 70, 80, 90, or 100% greater in length than the modified mRNA. The poly-A tail may also be designed as a fraction of modified nucleic acids to which it belongs. In this context, the poly-A tail may be 10, 20, 30, 40, 50, 60, 70, 80, or 90% or more of the total length of the modified mRNA or the total length of the modified mRNA minus the poly-A tail.

### Generating mRNA Molecules

Methods for isolating RNA, synthesizing RNA, hybridizing nucleic acids, making and screening cDNA libraries, and performing PCR are well known in the art (*see, e.g.,* Gubler and Hoffman, Gene, 25:263-269 (1983); Sambrook et al., Molecular Cloning, A Laboratory Manual (2nd ed. 1989)); as are PCR methods (*see,* U.S. Patent Nos. 4,683,195 and 4,683,202; PCR Protocols: A Guide to Methods and Applications (Innis et al., eds, 1990)). Expression libraries are also well known to those of skill in the art. Additional basic texts disclosing the general methods of use in this invention include Kriegler, Gene Transfer and Expression: A Laboratory Manual (1990); and Current Protocols in Molecular Biology (Ausubel et al., eds., 1994).

### Lipid Particles

In certain aspects, the present invention provides lipid particles comprising one or more therapeutic mRNA molecules encapsulated within the lipid particles.

In some embodiments, the mRNA is fully encapsulated within the lipid portion of the lipid particle such that the mRNA in the lipid particle is resistant in aqueous solution to nuclease degradation. In other embodiments, the lipid particles described herein are substantially non-toxic to mammals such as humans. The lipid particles of the invention typically have a mean diameter of from about 30 nm to about 150 nm, from about 40 nm to about 150 nm, from about 50 nm to about 150 nm, from about 60 nm to about 130 nm, from about 70 nm to about 110 nm, or from about 70 to about 90 nm. The lipid particles of the invention also typically have a lipid:mRNA ratio (mass/mass ratio) of from about 1:1 to about 100:1, from about 1:1 to about 50:1, from about 2:1 to about 25:1, from about 3:1 to about 20:1, from about 5:1 to about 15:1, or from about 5:1 to about 10:1, or from about 10:1 to about 14:1, or from about 9:1 to about 20:1. In one embodiment, the lipid particles of the invention have a lipid: mRNA ratio (mass/mass ratio) of about 12:1, such as 12:1. In another embodiment, the lipid particles of the invention have a lipid: mRNA ratio (mass/mass ratio) of about 13:1, such as 13:1.

In preferred embodiments, the lipid particles of the invention are serum-stable nucleic acid-lipid particles (SNALP) which comprise an mRNA, a cationic lipid (*e.g.,* one or more cationic lipids of the present invention as set forth herein), a phospholipid, and a conjugated lipid that inhibits aggregation of the particles (*e.g*., one or more PEG-lipid conjugates). The lipid particles can also include cholesterol. The SNALP may comprise at least 1, 2, 3, 4, 5, 6, 7, 8, 9, 10, or more unmodified and/or modified mRNA that express one or more polypeptides. Nucleic acid-lipid particles and their method of preparation are described in, *e.g.,* U.S. Patent Nos. 5,753,613; 5,785,992; 5,705,385; 5,976,567; 5,981,501; 6,110,745; and 6,320,017; and PCT Publication No. WO 96/40964.

In the nucleic acid-lipid particles of the invention, the mRNA may be fully encapsulated within the lipid portion of the particle, thereby protecting the nucleic acid from nuclease degradation. In preferred embodiments, a SNALP comprising an mRNA is fully encapsulated within the lipid portion of the particle, thereby protecting the nucleic acid from nuclease degradation. In certain instances, the mRNA in the SNALP is not substantially degraded after exposure of the particle to a nuclease at 37°C for at least about 20, 30, 45, or 60 minutes. In certain other instances, the mRNA in the SNALP is not substantially degraded after incubation of the particle in serum at 37°C for at least about 30, 45, or 60 minutes or at least about 2, 3, 4, 5, 6, 7, 8, 9, 10, 12, 14, 16, 18, 20, 22, 24, 26, 28, 30, 32, 34, or 36 hours. In other embodiments, the mRNA is complexed with the lipid portion of the particle. One of the benefits of the formulations of the present invention is that the nucleic acid-lipid particle compositions are substantially non-toxic to mammals such as humans.

The term "fully encapsulated" indicates that the nucleic acid (mRNA) in the nucleic acid-lipid particle is not significantly degraded after exposure to serum or a nuclease assay that would significantly degrade free RNA. In a fully encapsulated system, preferably less than about 25% of the nucleic acid in the particle is degraded in a treatment that would normally degrade 100% of free nucleic acid, more preferably less than about 10%, and most preferably less than about 5% of the nucleic acid in the particle is degraded. "Fully encapsulated" also indicates that the nucleic acid-lipid particles are serum-stable, that is, that they do not rapidly decompose into their component parts upon *in vivo* administration.

In the context of nucleic acids, full encapsulation may be determined by performing a membrane-impermeable fluorescent dye exclusion assay, which uses a dye that has enhanced fluorescence when associated with nucleic acid. Specific dyes such as OliGreen® and RiboGreen® (Invitrogen Corp.; Carlsbad, CA) are available for the quantitative determination of plasmid DNA, single-stranded deoxyribonucleotides, and/or single- or double-stranded ribonucleotides. Encapsulation is determined by adding the dye to a liposomal formulation, measuring the resulting fluorescence, and comparing it to the fluorescence observed upon addition of a small amount of nonionic detergent. Detergent-mediated disruption of the liposomal bilayer releases the encapsulated nucleic acid, allowing it to interact with the membrane-impermeable dye. Nucleic acid encapsulation may be calculated as *E* = *(Iₒ* - *I)*/*Iₒ*, where *I* and *Iₒ* refer to the fluorescence intensities before and after the addition of detergent (*see,* Wheeler et al., Gene Ther., 6:271-281 (1999)).

In other embodiments, the present invention provides a nucleic acid-lipid particle (*e.g*., SNALP) composition comprising a plurality of nucleic acid-lipid particles.

In some instances, the SNALP composition comprises mRNA that is fully encapsulated within the lipid portion of the particles, such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the particles have the mRNA encapsulated therein.

Depending on the intended use of the lipid particles of the invention, the proportions of the components can be varied and the delivery efficiency of a particular formulation can be measured using, *e.g*., an endosomal release parameter (ERP) assay.

### Cationic Lipids

The cationic lipids include the (R) and/or (S) enantiomers thereof. Typically, the cationic lipids contain a portion (i.e. a hydrophobic moiety) that comprises unsaturated and/or saturated hydrocarbon chains.

General cationic lipids of Formula I having the following structure are: 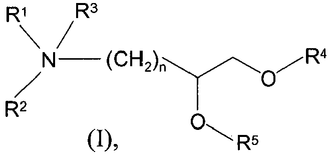 or salts thereof, wherein:
R¹ and R² are either the same or different and are independently hydrogen (H) or an optionally substituted C₁-C₆ alkyl, C₂-C₆ alkenyl, or C₂-C₆ alkynyl, or R¹ and R² may join to form an optionally substituted heterocyclic ring of 4 to 6 carbon atoms and 1 or 2 heteroatoms selected from the group consisting of nitrogen (N), oxygen (O), and mixtures thereof;
R³ is either absent or is hydrogen (H) or a C₁-C₆ alkyl to provide a quaternary amine; R⁴ and R⁵ are either the same or different and are independently an optionally substituted C₁₀-C₂₄ alkyl, C₁₀-C₂₄ alkenyl, C₁₀-C₂₄ alkynyl, or C₁₀-C₂₄ acyl, wherein at least one of R⁴ and R⁵ comprises at least two sites of unsaturation; and
n is 0, 1,2, 3, or 4.

In some embodiments of the general formula, R¹ and R² are independently an optionally substituted C₁-C₄ alkyl, C₂-C₄ alkenyl, or C₂-C₄ alkynyl. In one preferred embodiment of the general formula, R¹ and R² are both methyl groups. In other preferred embodiments of the general formula, n is 1 or 2. In other embodiments of the general formula, R³ is absent when the pH is above the pKₐ of the cationic lipid and R³ is hydrogen when the pH is below the pKₐ of the cationic lipid such that the amino head group is protonated. In an alternative embodiment of the general formula, R³ is an optionally substituted C₁-C₄ alkyl to provide a quaternary amine. In further embodiments of the general formula, R⁴ and R⁵ are independently an optionally substituted C₁₂-C₂₀ or C₁₄-C₂₂ alkyl, C₁₂-C₂₀ or C₁₄-C₂₂ alkenyl, C₁₂-C₂₀ or C₁₄-C₂₂ alkynyl, or C₁₂-C₂₀ or C₁₄-C₂₂ acyl, wherein at least one of R⁴ and R⁵ comprises at least two sites of unsaturation.

In certain embodiments of the general formula, R⁴ and R⁵ are independently selected from the group consisting of a dodecadienyl moiety, a tetradecadienyl moiety, a hexadecadienyl moiety, an octadecadienyl moiety, an icosadienyl moiety, a dodecatrienyl moiety, a tetradectrienyl moiety, a hexadecatrienyl moiety, an octadecatrienyl moiety, an icosatrienyl moiety, an arachidonyl moiety, and a docosahexaenoyl moiety, as well as acyl derivatives thereof (*e.g.,* linoleoyl, linolenoyl, γ-linolenoyl, *etc*.). In some instances, one of R⁴ and R⁵ comprises a branched alkyl group (*e.g*., a phytanyl moiety) or an acyl derivative thereof (*e.g*., a phytanoyl moiety). In certain instances, the octadecadienyl moiety is a linoleyl moiety. In certain other instances, the octadecatrienyl moiety is a linolenyl moiety or a γ-linolenyl moiety. In certain embodiments of the general formula, R⁴ and R⁵ are both linoleyl moieties, linolenyl moieties, or γ-linolenyl moieties. In particular embodiments of the general formula, the cationic lipid of Formula I is 1,2-dilinoleyloxy-N,N-dimethylaminopropane (DLinDMA), 1,2-dilinolenyloxy-N,N-dimethylaminopropane (DLenDMA), 1,2-dilinoleyloxy-(N,N-dimethyl)-butyl-4-amine (C2-DLinDMA), 1,2-dilinoleoyloxy-(N,N-dimethyl)-butyl-4-amine (C2-DLinDAP), or mixtures thereof.

In some embodiments of the general formula, the cationic lipid of Formula I forms a salt (preferably a crystalline salt) with one or more anions. In one particular embodiment of the general formula, the cationic lipid of Formula I is the oxalate (*e.g.*, hemioxalate) salt thereof, which is preferably a crystalline salt.

The synthesis of cationic lipids such as DLinDMA and DLenDMA, as well as additional cationic lipids, is described in U.S. Patent Publication No. 20060083780. The synthesis of cationic lipids such as C2-DLinDMA and C2-DLinDAP, as well as additional cationic lipids, is described in international patent application number WO2011/000106.

In general, cationic lipids of Formula II having the following structure (or salts thereof) are: 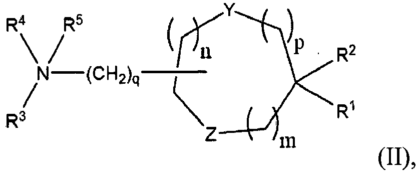 wherein R¹ and R² are either the same or different and are independently an optionally substituted C₁₂-C₂₄ alkyl, C₁₂-C₂₄ alkenyl, C₁₂-C₂₄ alkynyl, or C₁₂-C₂₄ acyl; R³ and R⁴ are either the same or different and are independently an optionally substituted C₁-C₆ alkyl, C₂-C₆ alkenyl, or C₂-C₆ alkynyl, or R³ and R⁴ may join to form an optionally substituted heterocyclic ring of 4 to 6 carbon atoms and 1 or 2 heteroatoms chosen from nitrogen and oxygen; R⁵ is either absent or is hydrogen (H) or a C₁-C₆ alkyl to provide a quaternary amine; m, n, and p are either the same or different and are independently either 0, 1, or 2, with the proviso that m, n, and p are not simultaneously 0; q is 0, 1, 2, 3, or 4; and Y and Z are either the same or different and are independently O, S, or NH. In a preferred embodiment of the general formula, q is 2.

In some embodiments of the general formula, the cationic lipid of Formula II is 2,2-dilinoleyl-4-(2-dimethylaminoethyl)-[1,3]-dioxolane, 2,2-dilinoleyl-4-(3-dimethylaminopropyl)-[1,3]-dioxolane, 2,2-dilinoleyl-4-(4-dimethylaminobutyl)-[1,3]-dioxolane, 2,2-dilinoleyl-5-dimethylaminomethyl-[1,3]-dioxane, 2,2-dilinoleyl-4-N-methylpepiazino-[1,3]-dioxolane, 2,2-dilinoleyl-4-dimethylaminomethyl-[1,3]-dioxolane, 2,2-dioleoyl-4-dimethylaminomethyl-[1,3]-dioxolane, 2,2-distearoyl-4-dimethylaminomethyl-[1,3]-dioxolane, 2,2-dilinoleyl-4-N-morpholino-[1,3]-dioxolane, 2,2-Dilinoleyl-4-trimethylamino-[1,3]-dioxolane chloride, 2,2-dilinoleyl-4,5-bis(dimethylaminomethyl)-[1,3]-dioxolane, 2,2-dilinoleyl-4-methylpiperzine-[1,3]-dioxolane, or mixtures thereof. In preferred embodiments of the general formula, the cationic lipid of Formula II is 2,2-dilinoleyl-4-(2-dimethylaminoethyl)-[1,3]-dioxolane.

In some embodiments of the general formula, the cationic lipid of Formula II forms a salt (preferably a crystalline salt) with one or more anions. In one particular embodiment of the general formula, the cationic lipid of Formula II is the oxalate (*e.g*., hemioxalate) salt thereof, which is preferably a crystalline salt.

The synthesis of cationic lipids such as 2,2-dilinoleyl-4-dimethylaminomethyl-[1,3]-dioxolane, as well as additional cationic lipids, is described in PCT Publication No. WO 09/086558 and in PCT Application No. PCT/US2009/060251, entitled "Improved Amino Lipids and Methods for the Delivery of Nucleic Acids,".

In general, cationic lipids of Formula III having the following structure are: 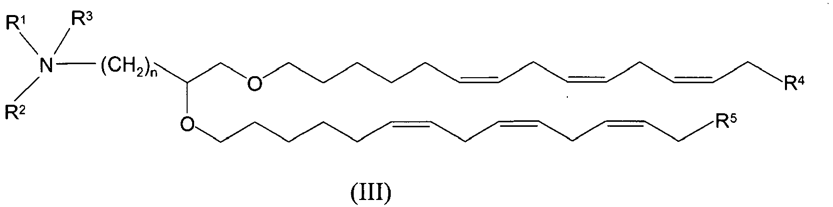 or salts thereof, wherein: R¹ and R² are either the same or different and are independently an optionally substituted C₁-C₆ alkyl, C₂-C₆ alkenyl, or C₂-C₆ alkynyl, or R¹ and R² may join to form an optionally substituted heterocyclic ring of 4 to 6 carbon atoms and 1 or 2 heteroatoms selected from the group consisting of nitrogen (N), oxygen (O), and mixtures thereof; R³ is either absent or is hydrogen (H) or a C₁-C₆ alkyl to provide a quaternary amine; R⁴ and R⁵ are either absent or present and when present are either the same or different and are independently an optionally substituted C₁-C₁₀ alkyl or C₂-C₁₀ alkenyl; and n is 0, 1, 2, 3, or 4.

In some embodiments of the general formula, R¹ and R² are independently an optionally substituted C₁-C₄ alkyl, C₂-C₄ alkenyl, or C₂-C₄ alkynyl. In a preferred embodiment of the general formula, R¹ and R² are both methyl groups. In another preferred embodiment of the general formula, R⁴ and R⁵ are both butyl groups. In yet another preferred embodiment of the general formula, n is 1. In other embodiments of the general formula, R³ is absent when the pH is above the pKₐ of the cationic lipid and R³ is hydrogen when the pH is below the pKₐ of the cationic lipid such that the amino head group is protonated. In an alternative embodiment of the general formula, R³ is an optionally substituted C₁-C₄ alkyl to provide a quaternary amine. In further embodiments of the general formula, R⁴ and R⁵ are independently an optionally substituted C₂-C₆ or C₂-C₄ alkyl or C₂-C₆ or C₂-C₄ alkenyl.

In an alternative embodiment of the general formula, the cationic lipid of Formula III comprises ester linkages between the amino head group and one or both of the alkyl chains. In some embodiments of the general formula, the cationic lipid of Formula III forms a salt (preferably a crystalline salt) with one or more anions. In one particular embodiment of the general formula, the cationic lipid of Formula III is the oxalate (*e.g*., hemioxalate) salt thereof, which is preferably a crystalline salt.

Although each of the alkyl chains in Formula III contains *cis* double bonds at positions 6, 9, and 12 (*i.e., cis,cis,cis*-Δ⁶,Δ⁹,Δ¹²), in an alternative embodiment of the general formula, one, two, or three of these double bonds in one or both alkyl chains may be in the *trans* configuration.

In one embodiment of the general formula, the cationic lipid of Formula III has the structure:

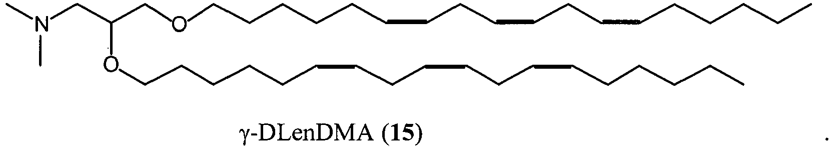

The synthesis of cationic lipids such as γ-DLenDMA, as well as additional cationic lipids, is described in International Patent Application WO2011/000106.

In particular embodiments of the general formula, a cationic lipid having the following structure is:

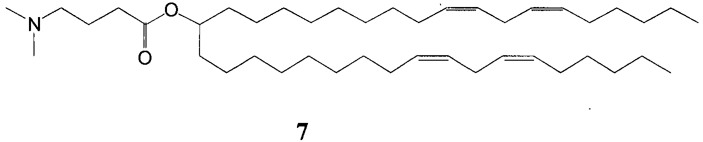

The synthesis of cationic lipids such as compound **7**, as well as additional cationic lipids, are described in U.S. patent serial number 8,158,601, and in International Patent Application serial number PCT/GB2011/000723.

Examples of other general cationic lipids or salts thereof which may be included in lipid particles include, but are not limited to, cationic lipids such as those described in WO2011/000106 as well as cationic lipids such as N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3 - (N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethy-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), 1,2-dioeylcarbamoyloxy-3-dimethylaminopropane (DO-C-DAP), 1,2-dimyristoleoyl-3-dimethylaminopropane (DMDAP), 1,2-dioleoyl-3-trimethylaminopropane chloride (DOTAP.Cl), dilinoleylmethyl-3-dimethylaminopropionate (DLin-M-C2-DMA; also known as DLin-M-K-DMA or DLin-M-DMA), and mixtures thereof. Additional general cationic lipids or salts thereof which may be included in lipid particles are described in U.S. Patent Publication No. 20090023673.

In general, a trialkyl cationic lipid can be used to prepare lipid particles. Such trialkyl cationic lipids typically comprise three saturated or unsaturated hydrocarbon chains having six or more carbons in each chain. Trialkyl cationic lipids that can be incorporated into general compositions can be prepared as described in International Patent Application Publication Number WO 2013/126803.

For example, a trialkyl cationic lipid of the following Formula B can be used to make general lipid particles:

X-A-Y-Z; (Formula B)

or salts thereof, wherein:
X is -N(H)R or -NR₂;
A is absent, C₁ to C₆alkyl, C₂ to C₆alkenyl, or C₂ to C₆alkynyl, which C₁ to C₆alkyl, C₂ to C₆alkenyl, and C₂ to C₆alkynyl is optionally substituted with one or more groups independently selected from oxo, halogen, heterocycle, -CN, -OR^{x}, -NR^{x}R^{y}, -NR^{x}C(=O)R^{y},-NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein each alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle;
Y is selected from the group consisting of absent, -C(=O)-, -O-, -OC(=O)-, -C(=O)O-, -N(R^{b})C(=O)-, -C(=O)N(R^{b})-, -N(R^{b})C(=O)O-, and -OC(=O)N(R^{b})-;
Z is a hydrophobic moiety comprising three chains wherein each of the chains is independently selected from C₈ to C₁₁alkyl, C₈ to C₁₁alkenyl, and C₈ to C₁₁alkynyl, which C₈ to C₁₁alkyl, C₈ to C₁₁alkenyl, and C₈ to C₁₁alkynyl is optionally substituted with one or more groups independently selected from oxo, halogen, heterocycle, -CN, -OR^{x}, -NR^{x}R^{y},-NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle,-NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle;
each R is independently alkyl, alkenyl, or alkynyl, that is optionally substituted with one or more groups independently selected from oxo, halogen, heterocycle, -CN, -OR^{x},-NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and-SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'},-C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle; and
each R^{b} is H or C₁ to C₆alkyl.

In some embodiments of the general formula, Z in Formula B has the structure: 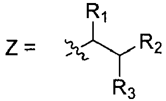 wherein, R₁, R₂, and R₃ are each independently C₈ to C₁₁alkyl, C₈ to C₁₁alkenyl, or C₈ to C₁₁alkynyl, which C₈ to C₁₁alkyl, C₈ to C₁₁alkenyl, and C₈ to C₁₁alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH,-CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle.

In the present invention, cationic lipids of the following Formula C are used to make lipid particles of the present invention:

X-A-Y-Z¹; (Formula C)

or salts thereof, wherein:
X is -N(H)R or -NR₂;
A is absent, C₁ to C₆alkyl, C₂ to C₆alkenyl, or C₂ to C₆alkynyl, which C₁ to C₆alkyl, C₂ to C₆alkenyl, and C₂ to C₆alkynyl is optionally substituted with one or more groups independently selected from oxo, halogen, heterocycle, -CN, -OR^{x}, -NR^{x}R^{y}, -NR^{x}C(=O)R^{y},-NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein each alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle;
Y is selected from the group consisting of absent, -C(=O)-, -O-, -OC(=O)-, -C(=O)O-, -N(R^{b})C(=O)-, -C(=O)N(R^{b})-, -N(R^{b})C(=O)O-, and -OC(=O)N(R^{b})-;
Z¹ is a C₁ to C₆alkyl that is subststuted with four R^{x} groups, wherein each R^{x} is independently selected from C₆ to C₁₁alkyl, C₆ to C₁₁alkenyl, and C₆ to C₁₁alkynyl, which C₆ to C₁₁alkyl, C₆ to C₁₁alkenyl, and C₆ to C₁₁alkynyl is optionally substituted with one or more groups independently selected from oxo, halogen, heterocycle, -CN, -OR^{x}, -NR^{x}R^{y},-NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle,-NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle;
each R is independently alkyl, alkenyl, or alkynyl, that is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(-O)R^{x'}, -C(=O)OR^{x'},-C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle; and
each R^{b} is H or C₁ to C₆alkyl.

In general, Z¹ in Formula C may have the structure: 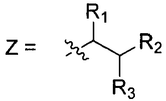 wherein, R₁, R₂, and R₃ are each independently C₈ to C₁₁alkyl, C₈ to C₁₁alkenyl, or C₈ to C₁₁alkynyl, which C₈ to C₁₁alkyl, C₈ to C₁₁alkenyl, and C₈ to C₁₁alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH,-CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle.

In general, Z¹ in Formula C may have the structure: 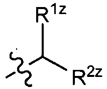 wherein one of R^{1z} and R^{2z} is selected from the group consisting of: 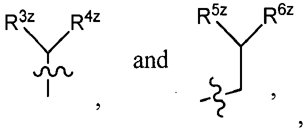 and
the other of R^{1z} and R^{2z} is selected from the group consisting of: 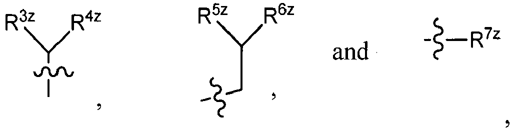 wherein each R^{3z}, R^{4z}, R^{5z}, R^{6z}, and R^{7z} is independently selected from C₆ to C₁₁alkyl, C₆ to C₁₁alkenyl, and C₆ to C₁₁alkynyl, which C₆ to C₁₁alkyl, C₆ to C₁₁alkenyl, and C₆ to C₁₁alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x},-C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'},-C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle.

In some embodiments of the present invention, Z¹ in Formula C has the structure: 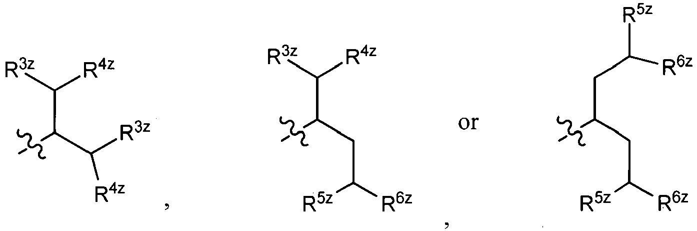 wherein each R^{3z}, R^{4z}, R^{5z}, and R^{6z} is independently selected from C₆ to C₁₁alkyl, C₆ to C₁₁alkenyl, and C₆ to C₁₁alkynyl, which C₆ to C₁₁alkyl, C₆ to C₁₁alkenyl, and C₆ to C₁₁alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x},-C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'},-C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle.

In some embodiments of the present invention the cationic lipid is selected from the group consisting of: 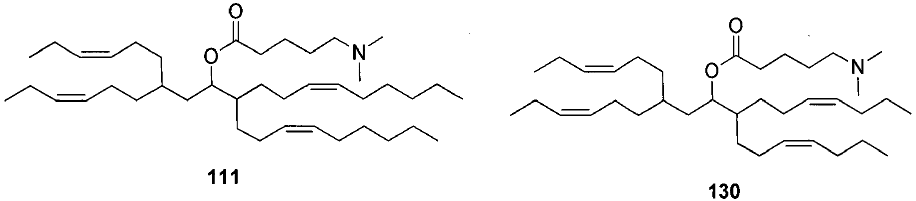 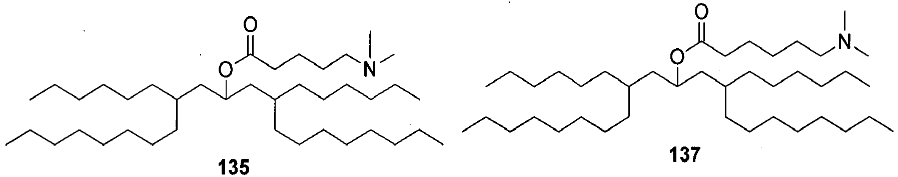 and 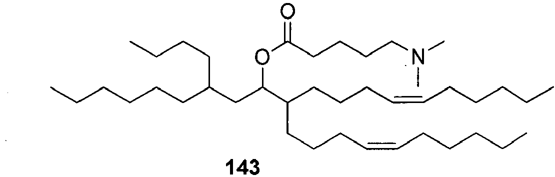 and salts thereof.

The synthesis of cationic lipids such as CLinDMA, as well as additional cationic lipids, is described in U.S. Patent Publication No. 20060240554. The synthesis of cationic lipids such as DLin-C-DAP, DLinDAC, DLinMA, DLinDAP, DLin-S-DMA, DLin-2-DMAP, DLinTMA.Cl, DLinTAP.Cl, DLinMPZ, DLinAP, DOAP, and DLin-EG-DMA, as well as additional cationic lipids, is described in PCT Publication No. WO 09/086558. The synthesis of cationic lipids such as DO-C-DAP, DMDAP, DOTAP.Cl, DLin-M-C2-DMA, as well as additional cationic lipids, is described in PCT Application No. PCT/US2009/060251, entitled "Improved Amino Lipids and Methods for the Delivery of Nucleic Acids," filed October 9, 2009. The synthesis of a number of other cationic lipids and related analogs has been described in U.S. Patent Nos. 5,208,036; 5,264,618; 5,279,833; 5,283,185; 5,753,613; and 5,785,992; and PCT Publication No. WO 96/10390. Additionally, a number of commercial preparations of general cationic lipids are, *e.g*., LIPOFECTIN® (including DOTMA and DOPE, available from Invitrogen); LIPOFECTAMINE® (including DOSPA and DOPE, available from Invitrogen); and TRANSFECTAM® (including DOGS, available from Promega Corp.).

In some embodiments, the cationic lipid comprises from about 50 mol % to about 90 mol %, from about 50 mol % to about 85 mol %, from about 50 mol % to about 80 mol %, from about 50 mol % to about 75 mol %, from about 50 mol % to about 70 mol %, from about 50 mol % to about 65 mol %, from about 50 mol % to about 60 mol %, from about 55 mol % to about 65 mol %, or from about 55 mol % to about 70 mol % (or any fraction thereof or range therein) of the total lipid present in the particle. In particular embodiments, the cationic lipid comprises about 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, or 65 mol % (or any fraction thereof) of the total lipid present in the particle.

In other embodiments, the cationic lipid comprises from about 2 mol % to about 60 mol %, from about 5 mol % to about 50 mol %, from about 10 mol % to about 50 mol %, from about 20 mol % to about 50 mol %, from about 20 mol % to about 40 mol %, from about 30 mol % to about 40 mol %, or about 40 mol % (or any fraction thereof or range therein) of the total lipid present in the particle.

Additional percentages and ranges of cationic lipids suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335.

It should be understood that the percentage of cationic lipid present in the lipid particles of the invention is a target amount, and that the actual amount of cationic lipid present in the formulation may vary, for example, by ± 5 mol %. For example, in the 1:57 lipid particle (*e.g.*, SNALP) formulation, the target amount of cationic lipid is 57.1 mol %, but the actual amount of cationic lipid may be ± 5 mol %, ± 4 mol %, ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

Examples of general cationic lipids include the following compounds: 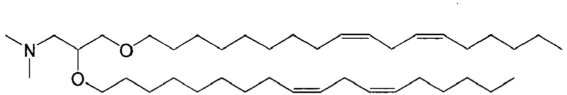 N,N-dimethyl-2,3-bis((9Z,12Z)-octadeca-9,12-dienyloxy)propan-1-amine (**5**) 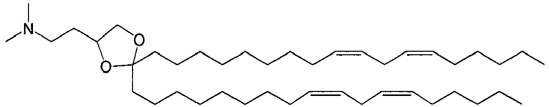 2-(2,2-di((9Z,12Z)-octadeca-9,12-dienyl)-1,3-dioxolan-4-yl)-N,N-dimethylethanamine (**6**) 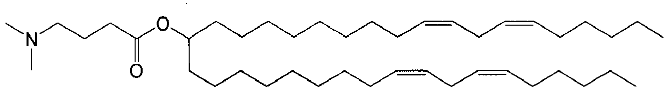 (6Z,9Z,28Z,31 Z)-heptatriaconta-6,9,28,3 1 -tetraen-19-yl 4-(dimethylamino)butanoate (**7**) 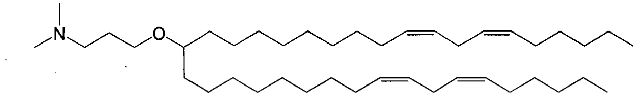 3-((6Z,9Z,28Z,31Z)-heptatriaconta-6,9,28,31-tetraen-19-yloxy)-N,N-dimethylpropan-1-amine (8) 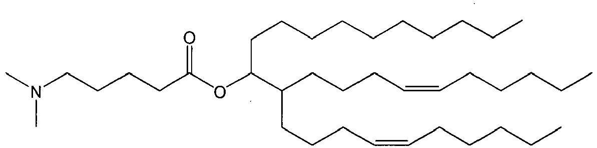 (Z)-12-((Z)-dec-4-enyl)docos-16-en-11-yl 5-(dimethylamino)pentanoate (**53**) 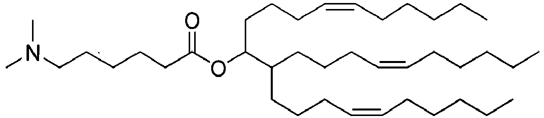 (6Z,16Z)-12-((Z)-dec-4-enyl)docosa-6,16-dien-11-yl 6-(dimethylamino)hexanoate (**11**) 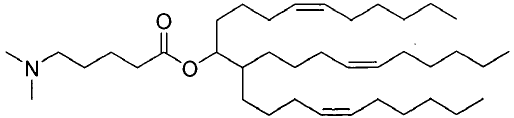 (6Z,16Z)-12-((Z)-dec-4-enyl)docosa-6,16-dien-11-yl 5-(dimethylamino)pentanoate (**13**) 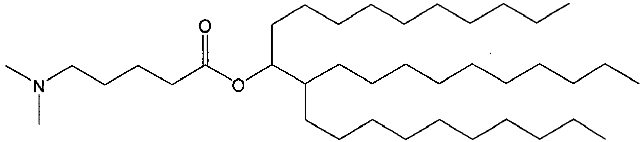 12-decyldocosan-11-yl 5-(dimethylamino)pentanoate (**14**) 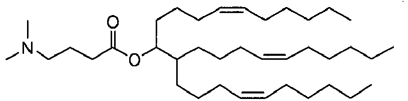 Compound **9**, 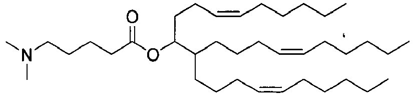 Compound **19**, 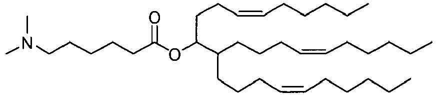 Compound **21**, 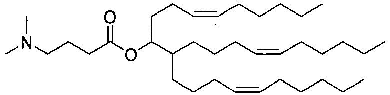 Compound **22**, 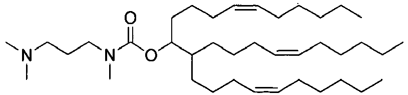 Compound **23**, 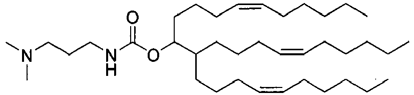 Compound **24**, 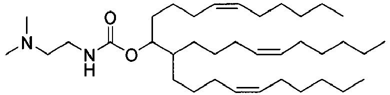 Compound **25**, 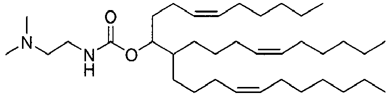 Compound **26**, 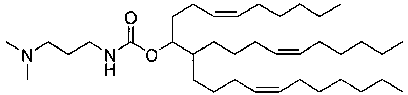 Compound **27**, 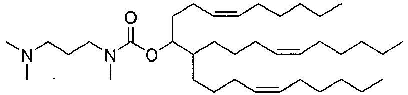 Compound **28**, 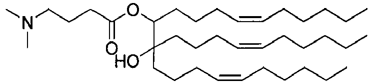 Compound **30**, 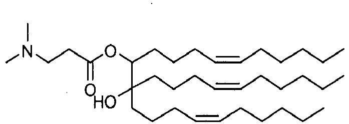 Compound **31**, 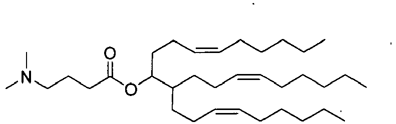 Compound **40**, 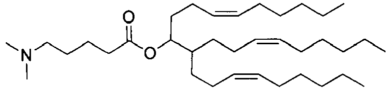 Compound **42**, 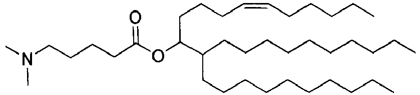 Compound **50**, 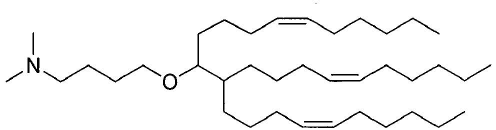 Compound **62**, 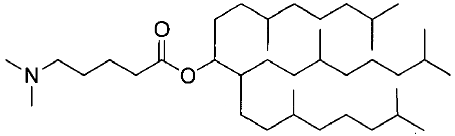 Compound **71**, 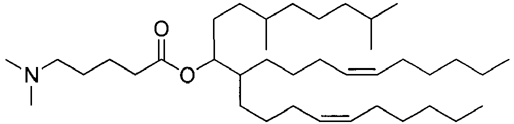 Compound **74**, 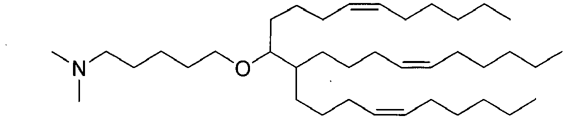 Compound **76**, 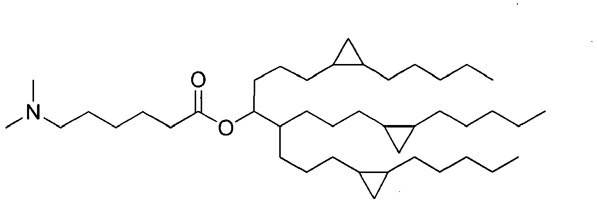 Compound **79**, 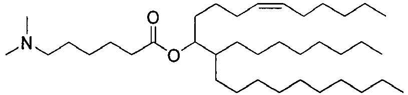 Compound **83**, 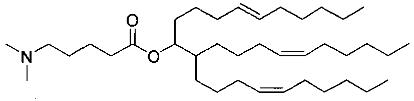 Compound **89**, and 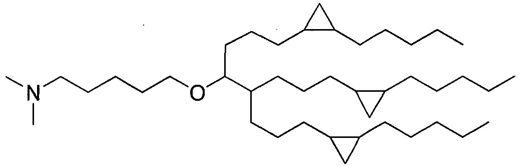 Compound **90**

### Non-cationic Lipids

The non-cationic lipids used in the lipid particles of the invention (*e.g*., SNALP) can be any of a variety of neutral uncharged, zwitterionic, or anionic lipids capable of producing a stable complex.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having C₁₀-C₂₄ carbon chains, *e.g*., lauroyl, myristoyl, palmitoyl, stearoyl, or oleoyl.

Additional examples of non-cationic lipids include sterols such as cholesterol and derivatives thereof. Non-limiting examples of cholesterol derivatives include polar analogues such as 5α-cholestanol, 5β-coprostanol, cholesteryl-(2'-hydroxy)-ethyl ether, cholesteryl-(4'-hydroxy)-butyl ether, and 6-ketocholestanol; non-polar analogues such as 5α-cholestane, cholestenone, 5α-cholestanone, 5β-cholestanone, and cholesteryl decanoate; and mixtures thereof. In preferred embodiments, the cholesterol derivative is a polar analogue such as cholesteryl-(4'-hydroxy)-butyl ether. The synthesis of cholesteryl-(2'-hydroxy)-ethyl ether is described in PCT Publication No. WO 09/127060.

In some embodiments, the non-cationic lipid present in the lipid particles (*e.g*., SNALP) comprises or consists of a mixture of one or more phospholipids and cholesterol or a derivative thereof. In other embodiments, the non-cationic lipid present in the lipid particles (*e.g.,* SNALP) comprises or consists of one or more phospholipids, *e.g.,* a cholesterol-free lipid particle formulation. In yet other embodiments, the non-cationic lipid present in the lipid particles (*e.g.,* SNALP) comprises or consists of cholesterol or a derivative thereof, *e.g.,* a phospholipid-free lipid particle formulation.

Other examples of non-cationic lipids suitable for use in the present invention include nonphosphorous containing lipids such as, *e.g*., stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide, ceramide, sphingomyelin, and the like.

In some embodiments, the non-cationic lipid comprises from about 10 mol % to about 60 mol %, from about 20 mol % to about 55 mol %, from about 20 mol % to about 45 mol %, from about 20 mol % to about 40 mol %, from about 25 mol % to about 50 mol %, from about 25 mol % to about 45 mol %, from about 30 mol % to about 50 mol %, from about 30 mol % to about 45 mol %, from about 30 mol % to about 40 mol %, from about 35 mol % to about 45 mol %, from about 37 mol % to about 42 mol %, or about 35 mol %, 36 mol %, 37 mol %, 38 mol %, 39 mol %, 40 mol %, 41 mol %, 42 mol %, 43 mol %, 44 mol %, or 45 mol % (or any fraction thereof or range therein) of the total lipid present in the particle.

In embodiments where the lipid particles contain a mixture of phospholipid and cholesterol or a cholesterol derivative, the mixture may comprise up to about 40 mol %, 45 mol %, 50 mol %, 55 mol %, or 60 mol % of the total lipid present in the particle.

In some embodiments, the phospholipid component in the mixture may comprise from about 2 mol % to about 20 mol %, from about 2 mol % to about 15 mol %, from about 2 mol % to about 12 mol %, from about 4 mol % to about 15 mol %, or from about 4 mol % to about 10 mol % (or any fraction thereof or range therein) of the total lipid present in the particle. In certain preferred embodiments, the phospholipid component in the mixture comprises from about 5 mol % to about 10 mol %, from about 5 mol % to about 9 mol %, from about 5 mol % to about 8 mol %, from about 6 mol % to about 9 mol %, from about 6 mol % to about 8 mol %, or about 5 mol %, 6 mol %, 7 mol %, 8 mol %, 9 mol %, or 10 mol % (or any fraction thereof or range therein) of the total lipid present in the particle. As a non-limiting example, a 1:57 lipid particle formulation comprising a mixture of phospholipid and cholesterol may comprise a phospholipid such as DPPC or DSPC at about 7 mol % (or any fraction thereof), *e.g.,* in a mixture with cholesterol or a cholesterol derivative at about 34 mol % (or any fraction thereof) of the total lipid present in the particle. As another non-limiting example, a 7:54 lipid particle formulation comprising a mixture of phospholipid and cholesterol may comprise a phospholipid such as DPPC or DSPC at about 7 mol % (or any fraction thereof), *e.g*., in a mixture with cholesterol or a cholesterol derivative at about 32 mol % (or any fraction thereof) of the total lipid present in the particle.

In other embodiments, the cholesterol component in the mixture may comprise from about 25 mol % to about 45 mol %, from about 25 mol % to about 40 mol %, from about 30 mol % to about 45 mol %, from about 30 mol % to about 40 mol %, from about 27 mol % to about 37 mol %, from about 25 mol % to about 30 mol %, or from about 35 mol % to about 40 mol % (or any fraction thereof or range therein) of the total lipid present in the particle. In certain preferred embodiments, the cholesterol component in the mixture comprises from about 25 mol % to about 35 mol %, from about 27 mol % to about 35 mol %, from about 29 mol % to about 35 mol %, from about 30 mol % to about 35 mol %, from about 30 mol % to about 34 mol %, from about 31 mol % to about 33 mol %, or about 30 mol %, 31 mol %, 32 mol %, 33 mol %, 34 mol %, or 35 mol % (or any fraction thereof or range therein) of the total lipid present in the particle. Typically, a 1:57 lipid particle formulation comprising a mixture of phospholipid and cholesterol may comprise cholesterol or a cholesterol derivative at about 34 mol % (or any fraction thereof), *e.g.,* in a mixture with a phospholipid such as DPPC or DSPC at about 7 mol % (or any fraction thereof) of the total lipid present in the particle. Typically, a 7:54 lipid particle formulation comprising a mixture of phospholipid and cholesterol may comprise cholesterol or a cholesterol derivative at about 32 mol % (or any fraction thereof), *e.g*., in a mixture with a phospholipid such as DPPC or DSPC at about 7 mol % (or any fraction thereof) of the total lipid present in the particle.

In embodiments where the lipid particles are phospholipid-free, the cholesterol or derivative thereof may comprise up to about 25 mol %, 30 mol %, 35 mol %, 40 mol %, 45 mol %, 50 mol %, 55 mol %, or 60 mol % of the total lipid present in the particle.

In some embodiments, the cholesterol or derivative thereof in the phospholipid-free lipid particle formulation may comprise from about 25 mol % to about 45 mol %, from about 25 mol % to about 40 mol %, from about 30 mol % to about 45 mol %, from about 30 mol % to about 40 mol %, from about 31 mol % to about 39 mol %, from about 32 mol % to about 38 mol %, from about 33 mol % to about 37 mol %, from about 35 mol % to about 45 mol %, from about 30 mol % to about 35 mol %, from about 35 mol % to about 40 mol %, or about 30 mol %, 31 mol %, 32 mol %, 33 mol %, 34 mol %, 35 mol %, 36 mol %, 37 mol %, 38 mol %, 39 mol %, or 40 mol % (or any fraction thereof or range therein) of the total lipid present in the particle. As a non-limiting example, a 1:62 lipid particle formulation may comprise cholesterol at about 37 mol % (or any fraction thereof) of the total lipid present in the particle. As another non-limiting example, a 7:58 lipid particle formulation may comprise cholesterol at about 35 mol % (or any fraction thereof) of the total lipid present in the particle.

In other embodiments, the non-cationic lipid comprises from about 5 mol % to about 90 mol %, from about 10 mol % to about 85 mol %, from about 20 mol % to about 80 mol %, about 10 mol % (*e.g.,* phospholipid only), or about 60 mol % (*e.g.,* phospholipid and cholesterol or derivative thereof) (or any fraction thereof or range therein) of the total lipid present in the particle.

Additional percentages and ranges of non-cationic lipids suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335.

It should be understood that the percentage of non-cationic lipid present in the lipid particles of the invention is a target amount, and that the actual amount of non-cationic lipid present in the formulation may vary, for example, by ± 5 mol %. For example, in the 1:57 lipid particle (*e.g*., SNALP) formulation, the target amount of phospholipid is 7.1 mol % and the target amount of cholesterol is 34.3 mol %, but the actual amount of phospholipid may be ± 2 mol %, ± 1.5 mol %, ± 1 mol %, ± 0.75 mol %, ± 0.5 mol %, ± 0.25 mol %, or ± 0.1 mol % of that target amount, and the actual amount of cholesterol may be ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle). Similarly, in the 7:54 lipid particle (*e.g*., SNALP) formulation, the target amount of phospholipid is 6.75 mol % and the target amount of cholesterol is 32.43 mol %, but the actual amount of phospholipid may be ± 2 mol %, ± 1.5 mol %, ± 1 mol %, ± 0.75 mol %, ± 0.5 mol %, ± 0.25 mol %, or ± 0.1 mol % of that target amount, and the actual amount of cholesterol may be ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

### Lipid Conjugates

In addition to cationic and non-cationic lipids, the lipid particles of the invention (*e.g*., SNALP) may further comprise a lipid conjugate. The conjugated lipid is useful in that it prevents the aggregation of particles. Suitable conjugated lipids include, but are not limited to, PEG-lipid conjugates, POZ-lipid conjugates, ATTA-lipid conjugates, cationic-polymer-lipid conjugates (CPLs), and mixtures thereof. In certain embodiments, the particles comprise either a PEG-lipid conjugate or an ATTA-lipid conjugate together with a CPL.

In a preferred embodiment, the lipid conjugate is a PEG-lipid. Examples of PEG-lipids include, but are not limited to, PEG coupled to dialkyloxypropyls (PEG-DAA) as described in, *e.g.,* PCT Publication No. WO 05/026372, PEG coupled to diacylglycerol (PEG-DAG) as described in, *e.g.,* U.S. Patent Publication Nos. 20030077829 and 2005008689, PEG coupled to phospholipids such as phosphatidylethanolamine (PEG-PE), PEG conjugated to ceramides as described in, *e.g.,* U.S. Patent No. 5,885,613, PEG conjugated to cholesterol or a derivative thereof, and mixtures thereof. Additional PEG-lipids suitable for use in the invention include, without limitation, mPEG2000-1,2-di-O-alkyl-*sn*3-carbomoylglyceride (PEG-C-DOMG). The synthesis of PEG-C-DOMG is described in PCT Publication No. WO 09/086558. Yet additional suitable PEG-lipid conjugates include, without limitation, 1-[8'-(1,2-dimyristoyl-3-propanoxy)-carboxamido-3',6'-dioxaoctanyl]carbamoyl-ω-methyl-poly(ethylene glycol) (2KPEG-DMG). The synthesis of 2KPEG-DMG is described in U.S. Patent No. 7,404,969.

PEG is a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, but are not limited to, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), monomethoxypolyethylene glycol-imidazolyl-carbonyl (MePEG-IM), as well as such compounds containing a terminal hydroxyl group instead of a terminal methoxy group (*e.g.,* HO-PEG-S, HO-PEG-S-NHS, HO-PEG-NH₂, *etc.*)*.* Other PEGs such as those described in U.S. Patent Nos. 6,774,180 and 7,053,150 (*e.g.,* mPEG (20 KDa) amine) are also useful for preparing the PEG-lipid conjugates of the present invention. In addition, monomethoxypolyethyleneglycol-acetic acid (MePEG-CH₂COOH) is particularly useful for preparing PEG-lipid conjugates including, *e.g*., PEG-DAA conjugates.

The PEG moiety of the PEG-lipid conjugates described herein may comprise an average molecular weight ranging from about 550 daltons to about 10,000 daltons. In certain instances, the PEG moiety has an average molecular weight of from about 750 daltons to about 5,000 daltons (*e.g*., from about 1,000 daltons to about 5,000 daltons, from about 1,500 daltons to about 3,000 daltons, from about 750 daltons to about 3,000 daltons, from about 750 daltons to about 2,000 daltons, *etc.*). In preferred embodiments, the PEG moiety has an average molecular weight of about 2,000 daltons or about 750 daltons.

In certain instances, the PEG can be optionally substituted by an alkyl, alkoxy, acyl, or aryl group. The PEG can be conjugated directly to the lipid or may be linked to the lipid via a linker moiety. Any linker moiety suitable for coupling the PEG to a lipid can be used including, *e.g*., non-ester containing linker moieties and ester-containing linker moieties. In a preferred embodiment, the linker moiety is a non-ester containing linker moiety. As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, as well as combinations thereof (such as a linker containing both a carbamate linker moiety and an amido linker moiety). In a preferred embodiment, a carbamate linker is used to couple the PEG to the lipid.

In other embodiments, an ester containing linker moiety is used to couple the PEG to the lipid. Suitable ester containing linker moieties include, *e.g*., carbonate (-OC(O)O-), succinoyl, phosphate esters (-O-(O)POH-O-), sulfonate esters, and combinations thereof.

Phosphatidylethanolamines having a variety of acyl chain groups of varying chain lengths and degrees of saturation can be conjugated to PEG to form the lipid conjugate. Such phosphatidylethanolamines are commercially available, or can be isolated or synthesized using conventional techniques known to those of skilled in the art. Phosphatidylethanolamines containing saturated or unsaturated fatty acids with carbon chain lengths in the range of C₁₀ to C₂₀ are preferred. Phosphatidylethanolamines with mono- or diunsaturated fatty acids and mixtures of saturated and unsaturated fatty acids can also be used. Suitable phosphatidylethanolamines include, but are not limited to, dimyristoyl-phosphatidylethanolamine (DMPE), dipalmitoyl-phosphatidylethanolamine (DPPE), dioleoylphosphatidylethanolamine (DOPE), and distearoyl-phosphatidylethanolamine (DSPE).

The term "ATTA" or "polyamide" includes, without limitation, compounds described in U.S. Patent Nos. 6,320,017 and 6,586,559. These compounds include a compound having the formula: 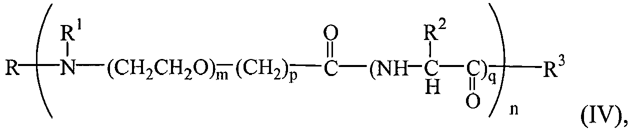 wherein R is a member selected from the group consisting of hydrogen, alkyl and acyl; R¹ is a member selected from the group consisting of hydrogen and alkyl; or optionally, R and R¹ and the nitrogen to which they are bound form an azido moiety; R² is a member of the group selected from hydrogen, optionally substituted alkyl, optionally substituted aryl and a side chain of an amino acid; R³ is a member selected from the group consisting of hydrogen, halogen, hydroxy, alkoxy, mercapto, hydrazino, amino and NR⁴R⁵, wherein R⁴ and R⁵ are independently hydrogen or alkyl; n is 4 to 80; m is 2 to 6; p is 1 to 4; and q is 0 or 1. It will be apparent to those of skill in the art that other polyamides can be used in the compounds of the present invention.

The term "diacylglycerol" or "DAG" includes a compound having 2 fatty acyl chains, R¹ and R², both of which have independently between 2 and 30 carbons bonded to the 1- and 2-position of glycerol by ester linkages. The acyl groups can be saturated or have varying degrees of unsaturation. Suitable acyl groups include, but are not limited to, lauroyl (C₁₂), myristoyl (C₁₄), palmitoyl (C₁₆), stearoyl (C₁₈), and icosoyl (C₂₀). In preferred embodiments, R¹ and R² are the same, *i.e.,* R¹ and R² are both myristoyl (*i.e.,* dimyristoyl), R¹ and R² are both stearoyl (*i.e.,* distearoyl), *etc.* Diacylglycerols have the following general formula:

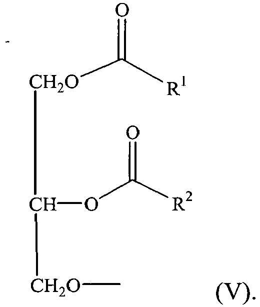

The term "dialkyloxypropyl" or "DAA" includes a compound having 2 alkyl chains, R¹ and R², both of which have independently between 2 and 30 carbons. The alkyl groups can be saturated or have varying degrees of unsaturation. Dialkyloxypropyls have the following general formula:

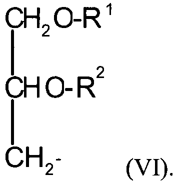

In a preferred embodiment, the PEG-lipid is a PEG-DAA conjugate having the following formula: 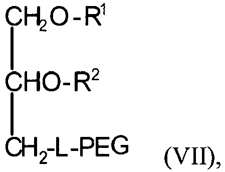 wherein R¹ and R² are independently selected and are long-chain alkyl groups having from about 10 to about 22 carbon atoms; PEG is a polyethyleneglycol; and L is a non-ester containing linker moiety or an ester containing linker moiety as described above. The long-chain alkyl groups can be saturated or unsaturated. Suitable alkyl groups include, but are not limited to, decyl (C₁₀), lauryl (C₁₂), myristyl (C₁₄), palmityl (C₁₆), stearyl (C₁₈), and icosyl (C₂₀). In preferred embodiments, R¹ and R² are the same, *i.e.,* R¹ and R² are both myristyl (*i.e.,* dimyristyl), R¹ and R² are both stearyl (*i.e.,* distearyl), *etc.*

In Formula VII above, the PEG has an average molecular weight ranging from about 550 daltons to about 10,000 daltons. In certain instances, the PEG has an average molecular weight of from about 750 daltons to about 5,000 daltons (*e.g*., from about 1,000 daltons to about 5,000 daltons, from about 1,500 daltons to about 3,000 daltons, from about 750 daltons to about 3,000 daltons, from about 750 daltons to about 2,000 daltons, *etc.*). In preferred embodiments, the PEG has an average molecular weight of about 2,000 daltons or about 750 daltons. The PEG can be optionally substituted with alkyl, alkoxy, acyl, or aryl groups. In certain embodiments, the terminal hydroxyl group is substituted with a methoxy or methyl group.

In a preferred embodiment, "L" is a non-ester containing linker moiety. Suitable non-ester containing linkers include, but are not limited to, an amido linker moiety, an amino linker moiety, a carbonyl linker moiety, a carbamate linker moiety, a urea linker moiety, an ether linker moiety, a disulphide linker moiety, a succinamidyl linker moiety, and combinations thereof. In a preferred embodiment, the non-ester containing linker moiety is a carbamate linker moiety (*i.e.,* a PEG-C-DAA conjugate). In another preferred embodiment, the non-ester containing linker moiety is an amido linker moiety (*i.e.,* a PEG-*A*-DAA conjugate). In yet another preferred embodiment, the non-ester containing linker moiety is a succinamidyl linker moiety (*i.e.,* a PEG-S-DAA conjugate).

In particular embodiments, the PEG-lipid conjugate is selected from: 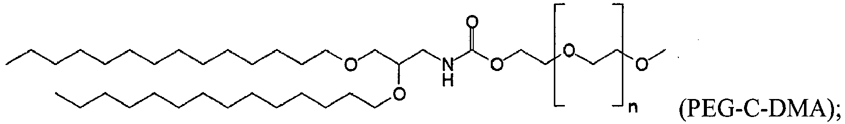 and

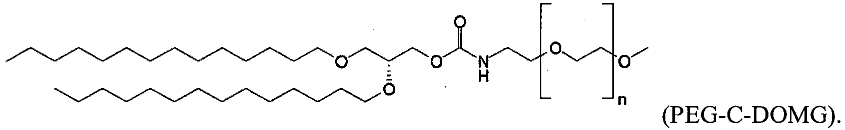

The PEG-DAA conjugates are synthesized using standard techniques and reagents known to those of skill in the art. It will be recognized that the PEG-DAA conjugates will contain various amide, amine, ether, thio, carbamate, and urea linkages. Those of skill in the art will recognize that methods and reagents for forming these bonds are well known and readily available. *See, e.g.,* March, ADVANCED ORGANIC CHEMISTRY (Wiley 1992); Larock, COMPREHENSIVE ORGANIC TRANSFORMATIONS (VCH 1989); and Furniss, VOGEL'S TEXTBOOK OF PRACTICAL ORGANIC CHEMISTRY, 5th ed. (Longman 1989). It will also be appreciated that any functional groups present may require protection and deprotection at different points in the synthesis of the PEG-DAA conjugates. Those of skill in the art will recognize that such techniques are well known. *See, e.g.,* Green and Wuts, PROTECTIVE GROUPS IN ORGANIC SYNTHESIS (Wiley 1991).

Preferably, the PEG-DAA conjugate is a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, or a PEG-distearyloxypropyl (C₁₈) conjugate. In these embodiments, the PEG preferably has an average molecular weight of about 750 or about 2,000 daltons. In one particularly preferred embodiment, the PEG-lipid conjugate comprises PEG2000-C-DMA, wherein the "2000" denotes the average molecular weight of the PEG, the "C" denotes a carbamate linker moiety, and the "DMA" denotes dimyristyloxypropyl. In another particularly preferred embodiment, the PEG-lipid conjugate comprises PEG750-C-DMA, wherein the "750" denotes the average molecular weight of the PEG, the "C" denotes a carbamate linker moiety, and the "DMA" denotes dimyristyloxypropyl. In particular embodiments, the terminal hydroxyl group of the PEG is substituted with a methyl group. Those of skill in the art will readily appreciate that other dialkyloxypropyls can be used in the PEG-DAA conjugates of the present invention.

In addition to the foregoing, it will be readily apparent to those of skill in the art that other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses such as hydroxymethylcellulose or hydroxyethylcellulose.

In addition to the foregoing components, the lipid particles (*e.g*., SNALP) of the present invention can further comprise cationic poly(ethylene glycol) (PEG) lipids or CPLs (*see, e.g.,* Chen et al., Bioconj. Chem., 11:433-437 (2000); U.S. Patent No. 6,852,334; PCT Publication No. WO 00/62813).

Suitable CPLs include compounds of Formula VIII:

A-W-Y (VIII),

wherein A, W, and Y are as described below.

With reference to Formula VIII, "A" is a lipid moiety such as an amphipathic lipid, a neutral lipid, or a hydrophobic lipid that acts as a lipid anchor. Suitable lipid examples include, but are not limited to, diacylglycerolyls, dialkylglycerolyls, N-N-dialkylaminos, 1,2-diacyloxy-3-aminopropanes, and 1,2-dialkyl-3-aminopropanes.

"W" is a polymer or an oligomer such as a hydrophilic polymer or oligomer. Preferably, the hydrophilic polymer is a biocompatable polymer that is nonimmunogenic or possesses low inherent immunogenicity. Alternatively, the hydrophilic polymer can be weakly antigenic if used with appropriate adjuvants. Suitable nonimmunogenic polymers include, but are not limited to, PEG, polyamides, polylactic acid, polyglycolic acid, polylactic acid/polyglycolic acid copolymers, and combinations thereof. In a preferred embodiment, the polymer has a molecular weight of from about 250 to about 7,000 daltons.

"Y" is a polycationic moiety. The term polycationic moiety refers to a compound, derivative, or functional group having a positive charge, preferably at least 2 positive charges at a selected pH, preferably physiological pH. Suitable polycationic moieties include basic amino acids and their derivatives such as arginine, asparagine, glutamine, lysine, and histidine; spermine; spermidine; cationic dendrimers; polyamines; polyamine sugars; and amino polysaccharides. The polycationic moieties can be linear, such as linear tetralysine, branched or dendrimeric in structure. Polycationic moieties have between about 2 to about 15 positive charges, preferably between about 2 to about 12 positive charges, and more preferably between about 2 to about 8 positive charges at selected pH values. The selection of which polycationic moiety to employ may be determined by the type of particle application which is desired.

The charges on the polycationic moieties can be either distributed around the entire particle moiety, or alternatively, they can be a discrete concentration of charge density in one particular area of the particle moiety *e.g.,* a charge spike. If the charge density is distributed on the particle, the charge density can be equally distributed or unequally distributed. All variations of charge distribution of the polycationic moiety are encompassed by the present invention.

The lipid "A" and the nonimmunogenic polymer "W" can be attached by various methods and preferably by covalent attachment. Methods known to those of skill in the art can be used for the covalent attachment of "A" and "W." Suitable linkages include, but are not limited to, amide, amine, carboxyl, carbonate, carbamate, ester, and hydrazone linkages. It will be apparent to those skilled in the art that "A" and "W" must have complementary functional groups to effectuate the linkage. The reaction of these two groups, one on the lipid and the other on the polymer, will provide the desired linkage. For example, when the lipid is a diacylglycerol and the terminal hydroxyl is activated, for instance with NHS and DCC, to form an active ester, and is then reacted with a polymer which contains an amino group, such as with a polyamide (*see, e.g.,* U.S. Patent Nos. 6,320,017 and 6,586,559), an amide bond will form between the two groups.

In certain instances, the polycationic moiety can have a ligand attached, such as a targeting ligand or a chelating moiety for complexing calcium. Preferably, after the ligand is attached, the cationic moiety maintains a positive charge. In certain instances, the ligand that is attached has a positive charge. Suitable ligands include, but are not limited to, a compound or device with a reactive functional group and include lipids, amphipathic lipids, carrier compounds, bioaffinity compounds, biomaterials, biopolymers, biomedical devices, analytically detectable compounds, therapeutically active compounds, enzymes, peptides, proteins, antibodies, immune stimulators, radiolabels, fluorogens, biotin, drugs, haptens, DNA, RNA, polysaccharides, liposomes, virosomes, micelles, immunoglobulins, functional groups, other targeting moieties, or toxins.

In some embodiments, the lipid conjugate (*e.g*., PEG-lipid) comprises from about 0.1 mol % to about 2 mol %, from about 0.5 mol % to about 2 mol %, from about 1 mol % to about 2 mol %, from about 0.6 mol % to about 1.9 mol %, from about 0.7 mol % to about 1.8 mol %, from about 0.8 mol % to about 1.7 mol %, from about 0.9 mol % to about 1.6 mol %, from about 0.9 mol % to about 1.8 mol %, from about 1 mol % to about 1.8 mol %, from about 1 mol % to about 1.7 mol %, from about 1.2 mol % to about 1.8 mol %, from about 1.2 mol % to about 1.7 mol %, from about 1.3 mol % to about 1.6 mol %, or from about 1.4 mol % to about 1.5 mol % (or any fraction thereof or range therein) of the total lipid present in the particle.

In other embodiments, the lipid conjugate (*e.g*., PEG-lipid) comprises from about 0 mol % to about 20 mol %, from about 0.5 mol % to about 20 mol %, from about 2 mol % to about 20 mol %, from about 1.5 mol % to about 18 mol %, from about 2 mol % to about 15 mol %, from about 4 mol % to about 15 mol %, from about 2 mol % to about 12 mol %, from about 5 mol % to about 12 mol %, or about 2 mol % (or any fraction thereof or range therein) of the total lipid present in the particle.

In further embodiments, the lipid conjugate (*e.g*., PEG-lipid) comprises from about 4 mol % to about 10 mol %, from about 5 mol % to about 10 mol %, from about 5 mol % to about 9 mol %, from about 5 mol % to about 8 mol %, from about 6 mol % to about 9 mol %, from about 6 mol % to about 8 mol %, or about 5 mol %, 6 mol %, 7 mol %, 8 mol %, 9 mol %, or 10 mol % (or any fraction thereof or range therein) of the total lipid present in the particle.

Additional percentages and ranges of lipid conjugates suitable for use in the lipid particles of the present invention are described in PCT Publication No. WO 09/127060, U.S. Published Application No. US 2011/0071208, PCT Publication No. WO2011/000106, and U.S. Published Application No. US 2011/0076335.

It should be understood that the percentage of lipid conjugate (*e.g*., PEG-lipid) present in the lipid particles of the invention is a target amount, and that the actual amount of lipid conjugate present in the formulation may vary, for example, by ± 2 mol %. For example, in the 1:57 lipid particle (*e.g.*, SNALP) formulation, the target amount of lipid conjugate is 1.4 mol %, but the actual amount of lipid conjugate may be ± 0.5 mol %, ± 0.4 mol %, ± 0.3 mol %, ± 0.2 mol %, ± 0.1 mol %, or ± 0.05 mol % of that target amount, with the balance of the formulation being made up of other lipid components (adding up to 100 mol % of total lipids present in the particle). Similarly, in the 7:54 lipid particle (*e.g*., SNALP) formulation, the target amount of lipid conjugate is 6.76 mol %, but the actual amount of lipid conjugate may be ± 2 mol %, ± 1.5 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

One of ordinary skill in the art will appreciate that the concentration of the lipid conjugate can be varied depending on the lipid conjugate employed and the rate at which the lipid particle is to become fusogenic.

By controlling the composition and concentration of the lipid conjugate, one can control the rate at which the lipid conjugate exchanges out of the lipid particle and, in turn, the rate at which the lipid particle becomes fusogenic. For instance, when a PEG-DAA conjugate is used as the lipid conjugate, the rate at which the lipid particle becomes fusogenic can be varied, for example, by varying the concentration of the lipid conjugate, by varying the molecular weight of the PEG, or by varying the chain length and degree of saturation of the alkyl groups on the PEG-DAA conjugate. In addition, other variables including, for example, pH, temperature, ionic strength, *etc.* can be used to vary and/or control the rate at which the lipid particle becomes fusogenic. Other methods which can be used to control the rate at which the lipid particle becomes fusogenic will become apparent to those of skill in the art upon reading this disclosure. Also, by controlling the composition and concentration of the lipid conjugate, one can control the lipid particle (*e.g*., SNALP) size.

### Preparation of Lipid Particles

The lipid particles of the present invention, *e.g*., SNALP, in which an mRNA is entrapped within the lipid portion of the particle and is protected from degradation, can be formed by any method known in the art including, but not limited to, a continuous mixing method, a direct dilution process, and an in-line dilution process.

In certain embodiments, the present invention provides nucleic acid-lipid particles (*e.g.,* SNALP) produced via a continuous mixing method, *e.g*., a process that includes providing an aqueous solution comprising an mRNA in a first reservoir, providing an organic lipid solution in a second reservoir (wherein the lipids present in the organic lipid solution are solubilized in an organic solvent, *e.g.*, a lower alkanol such as ethanol), and mixing the aqueous solution with the organic lipid solution such that the organic lipid solution mixes with the aqueous solution so as to substantially instantaneously produce a lipid vesicle (*e.g*., liposome) encapsulating the nucleic acid within the lipid vesicle. This process and the apparatus for carrying out this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a lipid vesicle substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (*i.e*., aqueous solution) to produce a nucleic acid-lipid particle.

The nucleic acid-lipid particles formed using the continuous mixing method typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

In another embodiment, the present invention provides nucleic acid-lipid particles (*e.g.,* SNALP) produced via a direct dilution process that includes forming a lipid vesicle (*e.g.,* liposome) solution and immediately and directly introducing the lipid vesicle solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of lipid vesicle solution introduced thereto. As a non-limiting example, a lipid vesicle solution in 45% ethanol when introduced into the collection vessel containing an equal volume of dilution buffer will advantageously yield smaller particles.

In yet another embodiment, the present invention provides nucleic acid-lipid particles (*e.g.,* SNALP) produced via an in-line dilution process in which a third reservoir containing dilution buffer is fluidly coupled to a second mixing region. In this embodiment, the lipid vesicle (*e.g.,* liposome) solution formed in a first mixing region is immediately and directly mixed with dilution buffer in the second mixing region. In preferred aspects, the second mixing region includes a T-connector arranged so that the lipid vesicle solution and the dilution buffer flows meet as opposing 180° flows; however, connectors providing shallower angles can be used, *e.g.,* from about 27° to about 180° (*e.g.,* about 90°). A pump mechanism delivers a controllable flow of buffer to the second mixing region. In one aspect, the flow rate of dilution buffer provided to the second mixing region is controlled to be substantially equal to the flow rate of lipid vesicle solution introduced thereto from the first mixing region. This embodiment advantageously allows for more control of the flow of dilution buffer mixing with the lipid vesicle solution in the second mixing region, and therefore also the concentration of lipid vesicle solution in buffer throughout the second mixing process. Such control of the dilution buffer flow rate advantageously allows for small particle size formation at reduced concentrations.

These processes and the apparatuses for carrying out these direct dilution and in-line dilution processes are described in detail in U.S. Patent Publication No. 20070042031.

The nucleic acid-lipid particles formed using the direct dilution and in-line dilution processes typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

If needed, the lipid particles of the invention (*e.g.,* SNALP) can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Patent No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In other embodiments, the methods may further comprise adding non-lipid polycations which are useful to effect the lipofection of cells using the present compositions. Examples of suitable non-lipid polycations include, hexadimethrine bromide (sold under the brand name POLYBRENE®, from Aldrich Chemical Co., Milwaukee, Wisconsin, USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

In some embodiments, the nucleic acid to lipid ratios (mass/mass ratios) in a formed nucleic acid-lipid particle (*e.g.,* SNALP) will range from about 0.01 to about 0.2, from about 0.05 to about 0.2, from about 0.02 to about 0.1, from about 0.03 to about 0.1, or from about 0.01 to about 0.08. The ratio of the starting materials (input) also falls within this range. In other embodiments, the particle preparation uses about 400 µg nucleic acid per 10 mg total lipid or a nucleic acid to lipid mass ratio of about 0.01 to about 0.08 and, more preferably, about 0.04, which corresponds to 1.25 mg of total lipid per 50 µg of nucleic acid. In other preferred embodiments, the particle has a nucleic acid:lipid mass ratio of about 0.08.

In other embodiments, the lipid to nucleic acid ratios (mass/mass ratios) in a formed nucleic acid-lipid particle (*e.g.,* SNALP) will range from about 1 (1:1) to about 100 (100:1), from about 5 (5:1) to about 100 (100:1), from about 1 (1:1) to about 50 (50:1), from about 2 (2:1) to about 50 (50:1), from about 3 (3:1) to about 50 (50:1), from about 4 (4:1) to about 50 (50:1), from about 5 (5:1) to about 50 (50:1), from about 1 (1:1) to about 25 (25:1), from about 2 (2:1) to about 25 (25:1), from about 3 (3:1) to about 25 (25:1), from about 4 (4:1) to about 25 (25:1), from about 5 (5:1) to about 25 (25:1), from about 5 (5:1) to about 20 (20:1), from about 5 (5:1) to about 15 (15:1), from about 5 (5:1) to about 10 (10:1), or about 5 (5:1), 6 (6:1), 7 (7:1), 8 (8:1), 9 (9:1), 10 (10:1), 11 (11:1), 12 (12:1), 13 (13:1), 14 (14:1), 15 (15:1), 16 (16:1), 17 (17:1), 18 (18:1), 19 (19:1), 20 (20:1), 21 (21:1), 22 (22:1), 23 (23:1), 24 (24:1), or 25 (25:1), or any fraction thereof or range therein. The ratio of the starting materials (input) also falls within this range.

As previously discussed, the conjugated lipid may further include a CPL. A variety of general methods for making SNALP-CPLs (CPL-containing SNALP) are discussed herein. Two general techniques include the "post-insertion" technique, that is, insertion of a CPL into, for example, a pre-formed SNALP, and the "standard" technique, wherein the CPL is included in the lipid mixture during, for example, the SNALP formation steps. The post-insertion technique results in SNALP having CPLs mainly in the external face of the SNALP bilayer membrane, whereas standard techniques provide SNALP having CPLs on both internal and external faces. The method is especially useful for vesicles made from phospholipids (which can contain cholesterol) and also for vesicles containing PEG-lipids (such as PEG-DAAs and PEG-DAGs). Methods of making SNALP-CPLs are taught, for example, in U.S. Patent Nos. 5,705,385; 6,586,410; 5,981,501; 6,534,484; and 6,852,334; U.S. Patent Publication No. 20020072121; and PCT Publication No. WO 00/62813.

### Administration of Lipid Particles

Once formed, the lipid particles of the invention (*e.g.,* SNALP) are particularly useful for the introduction of mRNA into cells. Accordingly, the present invention also provides a lipid particle for use in methods for introducing an mRNA into a cell. In particular embodiments, the mRNA is introduced into an infected cell such as reticuloendothelial cells (*e.g.,* macrophages, monocytes, *etc*.) as well as other cell types, including fibroblasts, endothelial cells (such as those lining the interior surface of blood vessels), and/or platelet cells. The methods may be carried out *in vitro* or *in vivo* by first forming the particles as described herein and then contacting the particles with the cells for a period of time sufficient for delivery of the mRNA to the cells to occur.

The lipid particles of the invention (*e.g.,* SNALP) can be adsorbed to almost any cell type with which they are mixed or contacted. Once adsorbed, the particles can either be endocytosed by a portion of the cells, exchange lipids with cell membranes, or fuse with the cells. Transfer or incorporation of the mRNA portion of the particle can take place via any one of these pathways. In particular, when fusion takes place, the particle membrane is integrated into the cell membrane and the contents of the particle combine with the intracellular fluid.

The lipid particles of the invention (*e.g.,* SNALP) can be administered either alone or in a mixture with a pharmaceutically acceptable carrier (*e.g.,* physiological saline or phosphate buffer) selected in accordance with the route of administration and standard pharmaceutical practice. Generally, normal buffered saline (*e.g.,* 135-150 mM NaCl) will be employed as the pharmaceutically acceptable carrier. Other suitable carriers include, *e.g*., water, buffered water, 0.4% saline, 0.3% glycine, and the like, including glycoproteins for enhanced stability, such as albumin, lipoprotein, globulin, *etc.* Additional suitable carriers are described in, *e.g*., REMINGTON'S PHARMACEUTICAL SCIENCES, Mack Publishing Company, Philadelphia, PA, 17th ed. (1985). As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The phrase "pharmaceutically acceptable" refers to molecular entities and compositions that do not produce an allergic or similar untoward reaction when administered to a human.

The pharmaceutically acceptable carrier is generally added following lipid particle formation. Thus, after the lipid particle (*e.g.,* SNALP) is formed, the particle can be diluted into pharmaceutically acceptable carriers such as normal buffered saline.

The concentration of particles in the pharmaceutical formulations can vary widely, *i.e.*, from less than about 0.05%, usually at or at least about 2 to 5%, to as much as about 10 to 90% by weight, and will be selected primarily by fluid volumes, viscosities, *etc.*, in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well-known sterilization techniques. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol, and water-soluble iron-specific chelators, such as ferrioxamine, are suitable.

In some embodiments, the lipid particles of the invention (*e.g.,* SNALP) are particularly useful in methods for the therapeutic delivery of one or more mRNA.

### In vivo Administration

Systemic delivery for *in vivo* therapy, *e.g*., delivery of a therapeutic nucleic acid to a distal target cell via body systems such as the circulation, has been achieved using nucleic acid-lipid particles such as those described in PCT Publication Nos. WO 05/007196, WO 05/121348, WO 05/120152, and WO 04/002453. The present invention also provides fully encapsulated lipid particles that protect the nucleic acid from nuclease degradation in serum, are non-immunogenic, are small in size, and are suitable for repeat dosing.

For *in vivo* administration, administration can be in any manner known in the art, *e.g*., by injection, oral administration, inhalation (*e.g*., intransal or intratracheal), transdermal application, or rectal administration. Administration can be accomplished via single or divided doses. The pharmaceutical compositions can be administered parenterally, *i.e*., intraarticularly, intravenously, intraperitoneally, subcutaneously, or intramuscularly. In some embodiments, the pharmaceutical compositions are administered intravenously or intraperitoneally by a bolus injection (*see*, *e.g*., U.S. Patent No. 5,286,634). Intracellular nucleic acid delivery has also been discussed in Straubringer et al., Methods Enzymol., 101:512 (1983); Mannino et al., Biotechniques, 6:682 (1988); Nicolau et al., Crit. Rev. Ther. Drug Carrier Syst., 6:239 (1989); and Behr, Acc. Chem. Res., 26:274 (1993). Still other methods of administering lipid-based therapeutics are described in, for example, U.S. Patent Nos. 3,993,754; 4,145,410; 4,235,871; 4,224,179; 4,522,803; and 4,588,578. The lipid particles can be administered by direct injection at the site of disease or by injection at a site distal from the site of disease (*see*, *e.g.*, Culver, HUMAN GENE THERAPY, MaryAnn Liebert, Inc., Publishers, New York. pp.70-71(1994)).

In embodiments where the lipid particles of the present invention (e.g., SNALP) are administered intravenously, at least about 5%, 10%, 15%, 20%, or 25% of the total injected dose of the particles is present in plasma about 8, 12, 24, 36, or 48 hours after injection. In other embodiments, more than about 20%, 30%, 40% and as much as about 60%, 70% or 80% of the total injected dose of the lipid particles is present in plasma about 8, 12, 24, 36, or 48 hours after injection. In certain instances, more than about 10% of a plurality of the particles is present in the plasma of a mammal about 1 hour after administration. In certain other instances, the presence of the lipid particles is detectable at least about 1 hour after administration of the particle. In some embodiments, the presence of a therapeutic nucleic acid such as an mRNA molecule is detectable in cells at about 8, 12, 24, 36, 48, 60, 72 or 96 hours after administration. In other embodiments, expression of a polypeptide encoded by an mRNA introduced into a living body in accordance with the present invention is detectable at about 8, 12, 24, 36, 48, 60, 72 or 96 hours after administration. In further embodiments, the presence or effect of an mRNA in cells at a site proximal or distal to the site of administration is detectable at about 12, 24, 48, 72, or 96 hours, or at about 6, 8, 10, 12, 14, 16, 18, 19, 20, 22, 24, 26, or 28 days after administration. In additional embodiments, the lipid particles (e.g., SNALP) of the invention are administered parenterally or intraperitoneally.

The compositions of the present invention, either alone or in combination with other suitable components, can be made into aerosol formulations (*i.e*., they can be "nebulized") to be administered via inhalation (*e.g*., intranasally or intratracheally) (*see*, Brigham et al., Am. J. Sci., 298:278 (1989)). Aerosol formulations can be placed into pressurized acceptable propellants, such as dichlorodifluoromethane, propane, nitrogen, and the like.

In certain embodiments, the pharmaceutical compositions may be delivered by intranasal sprays, inhalation, and/or other aerosol delivery vehicles. Methods for delivering nucleic acid compositions directly to the lungs via nasal aerosol sprays have been described, *e.g.,* in U.S. Patent Nos. 5,756,353 and 5,804,212. Likewise, the delivery of drugs using intranasal microparticle resins and lysophosphatidyl-glycerol compounds (U.S. Patent 5,725,871) are also known in the pharmaceutical arts. Similarly, transmucosal drug delivery in the form of a polytetrafluoroetheylene support matrix is described in U.S. Patent No. 5,780,045.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions are preferably administered, for example, by intravenous infusion, orally, topically, intraperitoneally, intravesically, or intrathecally.

Generally, when administered intravenously, the lipid particle formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, PA, 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, *etc.* Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, *etc.* These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

In certain applications, the lipid particles disclosed herein may be delivered via oral administration to the individual. The particles may be incorporated with excipients and used in the form of ingestible tablets, buccal tablets, troches, capsules, pills, lozenges, elixirs, mouthwash, suspensions, oral sprays, syrups, wafers, and the like (*see, e.g.,* U.S. Patent Nos. 5,641,515, 5,580,579, and 5,792,451). These oral dosage forms may also contain the following: binders, gelatin; excipients, lubricants, and/or flavoring agents. When the unit dosage form is a capsule, it may contain, in addition to the materials described above, a liquid carrier. Various other materials may be present as coatings or to otherwise modify the physical form of the dosage unit. Of course, any material used in preparing any unit dosage form should be pharmaceutically pure and substantially non-toxic in the amounts employed.

Typically, these oral formulations may contain at least about 0.1% of the lipid particles or more, although the percentage of the particles may, of course, be varied and may conveniently be between about 1% or 2% and about 60% or 70% or more of the weight or volume of the total formulation. Naturally, the amount of particles in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Formulations suitable for oral administration can consist of: (a) liquid solutions, such as an effective amount of a packaged therapeutic mRNA suspended in diluents such as water, saline, or PEG 400; (b) capsules, sachets, or tablets, each containing a predetermined amount of a therapeutic mRNA, as liquids, solids, granules, or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise a therapeutic mRNA in a flavor, *e.g*., sucrose, as well as pastilles comprising the therapeutic nucleic acid in an inert base, such as gelatin and glycerin or sucrose and acacia emulsions, gels, and the like containing, in addition to the therapeutic nucleic acid, carriers known in the art.

In another example of their use, lipid particles can be incorporated into a broad range of topical dosage forms. For instance, a suspension containing nucleic acid-lipid particles such as SNALP can be formulated and administered as gels, oils, emulsions, topical creams, pastes, ointments, lotions, foams, mousses, and the like.

When preparing pharmaceutical preparations of the lipid particles of the invention, it is preferable to use quantities of the particles which have been purified to reduce or eliminate empty particles or particles with therapeutic agents such as nucleic acid associated with the external surface.

The methods of the present invention may be practiced in a variety of hosts. Preferred hosts include mammalian species, such as primates (*e.g*., humans and chimpanzees as well as other nonhuman primates), canines, felines, equines, bovines, ovines, caprines, rodents (*e.g*., rats and mice), lagomorphs, and swine.

The amount of particles administered will depend upon the ratio of therapeutic mRNA to lipid, the particular therapeutic nucleic acid used, the disease or disorder being treated, the age, weight, and condition of the patient, and the judgment of the clinician, but will generally be between about 0.01 and about 50 mg per kilogram of body weight, preferably between about 0.1 and about 5 mg/kg of body weight, or about 10⁸-10¹⁰ particles per administration (*e.g.*, injection).

### In vitro Administration

For *in vitro* applications, the delivery of therapeutic mRNA can be to any cell grown in culture, whether of plant or animal origin, vertebrate or invertebrate, and of any tissue or type. In preferred embodiments, the cells are animal cells, more preferably mammalian cells, and most preferably human cells.

Contact between the cells and the lipid particles, when carried out *in vitro*, takes place in a biologically compatible medium. The concentration of particles varies widely depending on the particular application, but is generally between about 1 µmol and about 10 mmol. Treatment of the cells with the lipid particles is generally carried out at physiological temperatures (about 37°C) for periods of time of from about 1 to 48 hours, preferably of from about 2 to 4 hours.

In one group of preferred embodiments, a lipid particle suspension is added to 60-80% confluent plated cells having a cell density of from about 10³ to about 10⁵ cells/ml, more preferably about 2 x 10⁴ cells/ml. The concentration of the suspension added to the cells is preferably of from about 0.01 to 0.2 µg/ml, more preferably about 0.1 µg/ml.

To the extent that tissue culture of cells may be required, it is well-known in the art. For example, Freshney, Culture of Animal Cells, a Manual of Basic Technique, 3rd Ed., Wiley-Liss, New York (1994), Kuchler et al., Biochemical Methods in Cell Culture and Virology, Dowden, Hutchinson and Ross, Inc. (1977), and the references cited therein provide a general guide to the culture of cells. Cultured cell systems often will be in the form of monolayers of cells, although cell suspensions are also used.

Using an Endosomal Release Parameter (ERP) assay, the delivery efficiency of the SNALP or other lipid particle of the invention can be optimized. An ERP assay is described in detail in U.S. Patent Publication No. 20030077829. More particularly, the purpose of an ERP assay is to distinguish the effect of various cationic lipids and helper lipid components of SNALP or other lipid particle based on their relative effect on binding/uptake or fusion with/destabilization of the endosomal membrane. This assay allows one to determine quantitatively how each component of the SNALP or other lipid particle affects delivery efficiency, thereby optimizing the SNALP or other lipid particle. Usually, an ERP assay measures expression of a reporter protein (*e.g*., luciferase, β-galactosidase, green fluorescent protein (GFP), *etc*.), and in some instances, a SNALP formulation optimized for an expression plasmid will also be appropriate for encapsulating an mRNA. By comparing the ERPs for each of the various SNALP or other lipid particles, one can readily determine the optimized system, *e.g*., the SNALP or other lipid particle that has the greatest uptake in the cell.

### Cells for Delivery of Lipid Particles

The present invention can be practiced on a wide variety of cell types from any vertebrate species, including mammals, such as, *e.g,* canines, felines, equines, bovines, ovines, caprines, rodents (*e.g*., mice, rats, and guinea pigs), lagomorphs, swine, and primates (*e.g*. monkeys, chimpanzees, and humans).

### Detection of Lipid Particles

In some embodiments, the lipid particles of the present invention (*e.g.,* SNALP) are detectable in the subject at about 1, 2, 3, 4, 5, 6, 7, 8 or more hours. In other embodiments, the lipid particles of the present invention (*e.g.,* SNALP) are detectable in the subject at about 8, 12, 24, 48, 60, 72, or 96 hours, or about 6, 8, 10, 12, 14, 16, 18, 19, 22, 24, 25, or 28 days after administration of the particles. The presence of the particles can be detected in the cells, tissues, or other biological samples from the subject. The particles may be detected, *e.g*., by direct detection of the particles, and/or detection of an mRNA sequence encapsulated within the lipid particles, and/or detection of a polypeptide expressed from an mRNA.

### Detection of Particles

Lipid particles of the invention such as SNALP can be detected using any method known in the art. For example, a label can be coupled directly or indirectly to a component of the lipid particle using methods well-known in the art. A wide variety of labels can be used, with the choice of label depending on sensitivity required, ease of conjugation with the lipid particle component, stability requirements, and available instrumentation and disposal provisions. Suitable labels include, but are not limited to, spectral labels such as fluorescent dyes (*e.g*., fluorescein and derivatives, such as fluorescein isothiocyanate (FITC) and Oregon Green™; rhodamine and derivatives such Texas red, tetrarhodimine isothiocynate (TRITC), *etc*., digoxigenin, biotin, phycoerythrin, AMCA, CyDyes™, and the like; radiolabels such as ³H, ¹²⁵I, ³⁵S, ¹⁴C, ³²P, ³³P, *etc*.; enzymes such as horse radish peroxidase, alkaline phosphatase, *etc*.; spectral colorimetric labels such as colloidal gold or colored glass or plastic beads such as polystyrene, polypropylene, latex, *etc.* The label can be detected using any means known in the art.

### Detection of Nucleic Acids

Nucleic acids (*e.g*., mRNA) are detected and quantified herein by any of a number of means well-known to those of skill in the art. The detection of nucleic acids may proceed by well-known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), and hyperdiffusion chromatography may also be employed.

The selection of a nucleic acid hybridization format is not critical. A variety of nucleic acid hybridization formats are known to those skilled in the art. For example, common formats include sandwich assays and competition or displacement assays. Hybridization techniques are generally described in, *e.g*., "Nucleic Acid Hybridization, A Practical Approach," Eds. Hames and Higgins, IRL Press (1985).

The sensitivity of the hybridization assays may be enhanced through the use of a nucleic acid amplification system which multiplies the target nucleic acid being detected. *In vitro* amplification techniques suitable for amplifying sequences for use as molecular probes or for generating nucleic acid fragments for subsequent subcloning are known. Examples of techniques sufficient to direct persons of skill through such *in vitro* amplification methods, including the polymerase chain reaction (PCR), the ligase chain reaction (LCR), Qβ-replicase amplification, and other RNA polymerase mediated techniques (*e.g*., NASBA™) are found in Sambrook et al., In Molecular Cloning: A Laboratory Manual, Cold Spring Harbor Laboratory Press (2000); and Ausubel et al., SHORT PROTOCOLS IN MOLECULAR BIOLOGY, eds., Current Protocols, Greene Publishing Associates, Inc. and John Wiley & Sons, Inc. (2002); as well as U.S. Patent No. 4,683,202; PCR Protocols, A Guide to Methods and Applications (Innis et al. eds.) Academic Press Inc. San Diego, CA (1990); Amheim & Levinson (October 1, 1990), C&EN 36; The Journal Of NIH Research, 3:81 (1991); Kwoh et al., Proc. Natl. Acad. Sci. USA, 86:1173 (1989); Guatelli et al., Proc. Natl. Acad. Sci. USA, 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990); and Sooknanan and Malek, Biotechnology, 13:563 (1995). Improved methods of cloning *in vitro* amplified nucleic acids are described in U.S. Pat. No. 5,426,039. Other methods described in the art are the nucleic acid sequence based amplification (NASBA™, Cangene, Mississauga, Ontario) and Qβ-replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

Nucleic acids for use as probes, *e.g*., in *in vitro* amplification methods, for use as gene probes, or as inhibitor components are typically synthesized chemically according to the solid phase phosphoramidite triester method described by Beaucage et al., Tetrahedron Letts., 22:1859 1862 (1981), *e.g*., using an automated synthesizer, as described in Needham VanDevanter et al., Nucleic Acids Res., 12:6159 (1984). Purification of polynucleotides, where necessary, is typically performed by either native acrylamide gel electrophoresis or by anion exchange HPLC as described in Pearson et al., J. Chrom., 255:137 149 (1983). The sequence of the synthetic polynucleotides can be verified using the chemical degradation method of Maxam and Gilbert (1980) in Grossman and Moldave (eds.) Academic Press, New York, Methods in Enzymology, 65:499.

An alternative means for determining the level of transcription is *in situ* hybridization. *In situ* hybridization assays are well-known and are generally described in Angerer et al., Methods Enzymol., 152:649 (1987). In an *in situ* hybridization assay, cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### Examples

The present invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

### Example 1.

This Example describes expression of mRNA encoding a luciferase reporter gene in mice. The mRNA was encapsulated within nucleic acid-lipid particles (referred to as LNP) which were injected into mice.

### LNP Preparation

The experiments reported in this example used a firefly luciferase mRNA, fully modified with pseudouridine and 5-methylcytidine replacing uridine and cytidine, respectively. The mRNA was formulated into LNP with lipids at a lipid-to-drug ratio of 13:1. The LNP formulation used in these studies had the following lipid composition: PEG-lipid (PEG2000-C-DMA or PEG2000-C-DSA) (1.6 mol%); general Dilinoleylmethoxypropyl-*N*,*N*-dimethylamine (1-B11) (54.6 mol%); cholesterol (32.8 mol%); and DSPC (10.9 mol%). LNP were prepared at a 3.5 mg (mRNA) scale, using a method adapted from Jeffs et al, Pharmaceutical Research, Vol.22, No.3, 362-372 (2005*).* Buffer exchange was then performed via tangential flow ultrafiltration (TFU). LNP were concentrated to ∼5 mL and then diafiltered against 60 mL of PBS (12 wash volumes). The LNP were further concentrated to approx 3 mL, discharged, and sterile filtered. Concentration of the LNP was determined using RiboGreen and a Varian Cary Eclipse Fluorimeter. Particle size and polydispersity were determined using a Malvern Nano Series Zetasizer.

### In Vivo Protocols

In the experiments reported herein, two *in vivo* mouse models were used to demonstrate tissue luciferase expression following the intravenous injection of LNP. The liver model (for analysis of 1.6:55 (PEG2000-C-DMA) LNP) used naive female Balb/C mice. The distal subcutaneous tumor model (for analysis of 1.6:55 (PEG2000-C-DSA) LNP) used female scid/beige mice seeded in the hind flank with Hep3B cells.

In the liver model, LNP were dosed at 0.5 mg/kg in the naive Balb/C mice (n=5). Six hours following injection, the mice were euthanized and the livers were removed, weighed, and placed in lysing matrix tubes (containing one ceramic bead) on ice.

In the subcutaneous tumor model, Scid mice (n=4) were seeded with 3x10⁶ Hep3B cells subcutaneously in the hind flank. After ∼20 days, LNP were dosed at 1.0 mg/kg. At time points of 2, 4, 6, 8, 16, 24, and 48 hours, the mice were euthanized and the livers, spleens, lungs, kidneys, hearts, and tumors were removed, weighed, and placed in lysing matrix tubes (containing one ceramic bead) on ice.

In both models, PBS treatment groups were included to determine the background level of quenching of luciferase activity caused by the tissue homogenate in the Luciferase assay. Following collection, the tissues from these PBS treated animals were spiked with 50µL of 100ng/mL Luciferase solution in 1xCCLR (Cell Culture Lysis Reagent). In all cases, tissues were stored at -80°C until Luciferase assay was performed.

### Luciferase Assay Procedure

All sample processing procedures were performed on ice. Tissues were homogenized and centrifuged in an Eppendorf microfuge at 3000rpm at 4°C for 10 minutes. After centrifugation, 20 µL of homogenate was aliquoted in duplicate into a white 96-well luminometer plate. Firefly luciferase fluorescence was then analyzed on the EG&G Berthold Microplate Luminometer LB using the Promega Luciferase Assay System. The luciferase activity within the wells was determined by comparing the resulting Relative Light Units (RLUs) for the homogenates to a standard curve prepared from 20 µL of firefly luciferase. A quenching factor was assigned to each tissue to control for natural quenching from the homogenates. This was determined using luciferase-spiked tissue from PBS treated mice.

### Results

Table 1 shows the luciferase activity in various tissues of Balb/C mice at 6 hours after injection with general LNP comprising luciferase mRNA. The results show that expression was highest in liver and spleen.

| **Tissue** | **Luciferase Activity (pg Luc/g Tissue)** | **Std Dev** |
|---|---|---|
| Liver | 577766 | 25428 |
| Spleen | 35387 | 1978 |
| Lung | 1257 | 102 |
| Kidney | 2614 | 445 |
| Heart | 900 | 278 |

Tables 2 and 3 show luciferase expression in various tissues of Scid mice, seeded with Hep3B cells, at various times after injection with general LNP comprising luciferase mRNA. The results show that luciferase expression was initially highest in liver, spleen and Hep3B tumor, but that after 48 hours most expression was seen in Hep3B tumor.

**Table 2**

| | **Liver** | | **Spleen** | | **Lung** | | **Kidney** | |
|---|---|---|---|---|---|---|---|---|
| **Time (h)** | **Luciferase Activity (pg Luc/g Tissue)** | **Std Dev** | **Luciferase Activity (pg Luc/g Tissue)** | **Std Dev** | **Luciferase Activity (pg Luc/g Tissue)** | **Std Dev** | **Luciferase Activity (pg Luc/g Tissue)** | **Std Dev** |
| 2 | 199428 | 10538 | 154817 | 19119 | 1068 | 118 | 345 | 64 |
| 4 | 450250 | 18561 | 188207 | 30129 | 1382 | 237 | 834 | 63 |
| 6 | 446597 | 11590 | 127239 | 13749 | 2512 | 862 | 965 | 118 |
| 8 | 231417 | 13666 | 49089 | 10184 | 1438 | 202 | 694 | 140 |
| 16 | 38167 | 1438 | 7413 | 1626 | 846 | 226 | 509 | 85 |
| 24 | 9765 | 2379 | 5013 | 469 | 523 | 32 | 339 | 50 |
| 48 | 790 | 67 | 1058 | 223 | 260 | 37 | 90 | 9 |

**Table 3**

| **Heart** | | | **Tumor** | |
|---|---|---|---|---|
| **Time (h)** | **Luciferase Activity (pg Luc/g Tissue)** | **Std Dev** | **Luciferase Activity (pg Luc/g Tissue)** | **Std Dev** |
| 2 | 201 | 30 | 11603 | 2830 |
| 4 | 548 | 93 | 78513 | 14353 |
| 6 | 569 | 20 | 175858 | 35313 |
| 8 | 666 | 130 | 562934 | 93205 |
| 16 | 391 | 51 | 371712 | 33607 |
| 24 | 386 | 56 | 245899 | 15769 |
| 48 | 158 | 27 | 116748 | 14115 |

### Example 2

### General Procedures

### LNP Preparation:

The experiments described in this Example 2 used a firefly luciferase mRNA ("mLuc"), fully modified with pseudouridine and 5-methylcytidine replacing uridine and cytidine respectively. The LNP formulation used in these studies have the following general lipid composition (molar ratios): PEG-lipid (PEG2000-C-DMA (1.6 mol%); appropriate aminolipid (54.6 mol%); cholesterol (32.8 mol%); and DSPC (10.9 mol%). A lipid stock was prepared with the appropriate lipids dissolved in ethanol (12.6 mM). The mLuc stock was made up in a 40 mM EDTA buffer at 0.366 mg/mL in 40 mM EDTA. The two stocks were combined using the Jeffs et al method (Pharm. Research (2005), 22(3), pages 362-372), blending in a t-connector and diluted into Phosphate Buffered Saline, pH 7.4. Buffer exchange was then performed via overnight bag dialysis against 10x volume of PBS. After dialysis, LNP were concentrated by centrifugation in Vivaspin-6 or Vivaspin-20 units (MWCO 100k) from GE Healthcare. The LNP were then sterile filtered (0.2 µm filter). Concentration of the LNP was determined using RiboGreen and a Varian Cary Eclipse Fluorimeter. Particle size and polydispersity were detennined using a Malvern Nano Series Zetasizer.

### In vivo protocol (luciferase liver model)

LNP containing a luciferase mRNA payload were dosed at 0.5 mg/kg in naive Balb/C mice (n=3). Six hours following injection, the mice were euthanized and livers were removed, weighed, and placed in lysing matrix tubes (containing one ceramic bead) on ice. PBS treatment groups were included to determine the background level of quenching of luciferase activity caused by the tissue homogenate in the Luciferase assay. Following collection, the tissues from these PBS treated animals were spiked with 50µL of 100ng/mL Luciferase solution in 1xCCLR (Cell Culture Lysis Reagent). Tissues were stored at -80°C until Luciferase assay was performed.

### Luciferase Assay Procedure:

All sample processing procedures were performed on ice. Tissues were homogenized and centrifuged in an Eppendorf microfuge at 3000rpm & 4°C for 10 minutes. After centrifugation, 20 µL of homogenate was aliquoted in duplicate into a white 96-well luminometer plate. Firefly luciferase fluorescence was then analyzed on the EG&G Berthold Microplate Luminometer LB using the Promega Luciferase Assay System. The luciferase activity within the wells was determined by comparing the resulting RLUs for the homogenates to a standard curve prepared from 20 µL of firefly luciferase. A quenching factor was assigned to each tissue to control for natural quenching from the homogenates. This was determined using luciferase-spiked tissue from PBS treated mice.

### Relative formulability and potency

### Formulability

The effect of the choice of aminolipid on parameters such as formulability and potency in the mRNA formulations was examined. As benchmarks, we selected the general dilinoleyl lipids **5**, **6**, **7**, and **8.** These lipids have been shown to be extremely effective at mediating delivery of siRNA oligonucleotides to liver *in vivo.* A common feature of their structures is that they all possess two linoleyl chains (18 carbons with two cis double bonds) as their hydrophobic domain. We also selected a range of lipids with hydrophobic domains consisting of multiple (3 or 4) alkyl chains; **10**, **9**, **11**, **13**, **14**, **17**, and **18**, wherein the compounds with 3 alkyl chains represent reference examples and the compounds with 4 alkyl chains examples of the present invention. Formulations were prepared as described above, using a 12.6 mM lipid stock. The basic physicochemical characterization data is in Table 4, wherein: Zavg is the average lipid particle diameter; PDI is the polydispersity index that is a measure of the distribution of lipid particle diameters (a lower PDI value indicates a more homogenous population of particles); and % Encaps is the Percentage Encapsulation which is a measure of the amount of RNA encapsulated within the lipid particles (a higher Percentage Encapsulation value indicates that more mRNA was encapsulated).

**Table 4**

| LNP used for luciferase expression assay | | | |
|---|---|---|---|
| **Amino Lipid** | **Zavg (nm)** | **PDI** | **% Encaps** |
| **8** | 144 | 0.13 | 57 |
| **13** | 86 | 0.10 | 95 |
| **5** | 124 | 0.07 | 58 |
| **7** | 158 | 0.11 | 50 |
| **18** | 117 | 0.08 | 88 |
| **6** | 128 | 0.10 | 66 |
| **11** | 79 | 0.06 | 97 |
| **19** | 138 | 0.10 | 78 |
| **17** | 124 | 0.07 | 73 |
| **14** | 111 | 0.07 | 86 |
| **9** | 95 | 0.06 | 62 |

### Table 1

It is noteworthy that the 'multiple alkyl chain' (i.e. 3 or more) aminolipids (e.g., **13**) were found to formulate the mRNA payload much better than the benchmark linoleyl lipids (**5**, **6**, **7**, and **8**). In particular, the multiple alkyl chain lipids with short alkyl chains yielded formulations possessing either a smaller particle size, better encapsulation, or both.

Better encapsulation is advantageous for various reasons; a more efficient process is more cost effective, protects the payload more completely, helps avoid unwanted payload interaction with components of the blood, is more homogenous/reproducible and therefore far more appropriate for use as a pharmaceutical product.

Small particle size is important for in vivo applications for a couple of reasons. First, LNP often rely on the 'Enhanced Permeation and Retention' effect, whereby passive accumulation in target tissue is mediated by the particles passing through small fenestrae in the local vasculature. The diameter of liver fenestrae in humans has been reported to be 107 ± 1.5 nm, and in mice as 141 ± 1.5 nm. The formation of smaller particles should therefore improve liver penetration and accumulation, ultimately producing more effective formulations. Further, larger liposomal formulations are known to be rapidly eliminated from the blood and also have the potential for immune stimulation.

### Liver Activity Results

The various formulations described were also assessed for potency in the mouse liver model. As an additional control, the use of the commercially available delivery reagent TransIT (Mirus) has been described in the literature for delivery of mRNA. A 0.05 mg/mL solution of TransIT-mRNA complex was prepared as follows. 50 µL of 1 mg/mL mRNA was added to 860 µL of DMEM, and to this solution the reagents from the TransIT-mRNA kit (Mirus) were added. In sequential order, 35 µL of the mRNA Boost reagent was added followed by 55 µL of the TransIT-mRNA reagent. The final solution was then incubated for 2-5 minutes before intravenous or intraperitoneal dosing.

All formulations were administered intravenously at a dose of 0.5 mg/kg total mRNA. The TransIT formulation was additionally administered intraperitoneally, as described by Kariko et al. As shown in Table 5, formulations containing aminolipids with multiple short alkyl domains (e.g., 13-B43) were clearly more potent than those with the benchmark linoleyl alkyl domains. It was notable that those with good encapsulation, appropriate pKa (typically 6.1 - 6.3), and more double bonds in the hydrophobic domain were the most effective lipids.

**Table 5**

| **Treatment** | **Luciferase protein in ng per g of liver** |
|---|---|
| **5** | 30.28 |
| **6** | 199.06 |
| **8** | 264.90 |
| **7** | 369.85 |
| **10** | 203.47 |
| **17** | 365.04 |
| **14** | 864.54 |
| **9** | 968.83 |
| **18** | 1388.39 |
| **11** | 1604.99 |
| **13** | 2937.24 |
| TransIT-mLuc (IV) | 1.11 |
| TransIT-mLuc (IP) | 0.12 |

### Cryo Transmission Electron Microscopy

The following LNP formulation was used to prepare nucleic acid-lipid particles that were examined using Cryo-TEM: PEG-lipid (PEG2000-C-DMA) (1.6 mol%); 13-B43 lipid (54.6 mol%); cholesterol (32.8 mol%); and DSPC (10.9 mol%). LNP were prepared at a 0.95 mg (mRNA) scale, using the general procedures described above. The lipid stock was prepared in ethanol (12.6 mM total lipid). The mRNA stock was prepared at 0.366 mg/mL in 40 mM EDTA. The two stocks were combined and diluted into 4.9 mL of PBS. Buffer exchange was then performed via overnight bag dialysis against 10x volume of PBS. Samples were then concentrated to ∼1 mg/mL mRNA by centrifugation in Vivaspin-20 units (MWCO 100k) from GE Healthcare. The LNP were then sterile filtered. Concentration of the LNP was determined using RiboGreen and a Varian Cary Eclipse Fluorimeter. Particle size and polydispersity were determined using a Malvern Nano Series Zetasizer.

Nucleic acid-lipid particles prepared in accordance with the teachings of the foregoing paragraph were visualized by Cryo Transmission Electron Microscopy (Cryo-TEM). Analysis was performed at Uppsala University, Sweden, using a Zeiss EM 902A. Samples were incubated in a climate chamber (25°C, 98% humidity) for 20-30 minutes prior to use. Sample solution (0.5 µL) was then deposited on a copper grid, excess removed by blotting, and sample vitrified in liquid ethane. Images at 100,000X total magnification were captured, and diametrical size of particles calculated by number averaging. FIGURE 1 of this patent application shows a representative Cryo-TEM image of the lipid particles.

### Reproducibility of lipid particle formulation

Two aminolipids were selected to examine the reproducibility of lipid particle formulation. The general benchmark dilinoleyl aminolipid MC3 was compared to the short general trialkyl lipid 13-B43.

Multiple formulations were made, using the following composition (expressed as molar percentages): PEG-lipid (PEG2000-C-DMA) (1.6 mol%); amino lipid (54.6 mol%); cholesterol (32.8 mol%); and DSPC (10.9 mol%), according to the general procedures described above. The lipid stock was prepared in ethanol (ranging from 8.3 - 19.0 mM total lipid). The mRNA stock was prepared at 0.366 mg/mL in 40 mM EDTA. Buffer exchange was then performed via overnight bag dialysis against 10x volume of PBS. Samples were then concentrated to ∼1 mg/mL mRNA by centrifugation in Vivaspin-20 units (MWCO 100k) from GE Healthcare. The LNP were then sterile filtered. Concentration of the LNP was determined using RiboGreen and a Varian Cary Eclipse Fluorimeter. Particle size and polydispersity were determined using a Malvern Nano Series Zetasizer.

As shown in Tables 6 and 7, the compound **13** particles (Table 6) were consistently smaller, and showed better encapsulation, than the particles made with compound **7** (Table 7).

**Table 6**

| **Composition** | **Particle size (nm)** | **Polydispersity Index** | **% Encaps of mLuc** |
|---|---|---|---|
| **(13)** | 82 | 0.1 | 93 |
| | 82 | 0.11 | 92 |
| | 86 | 0.10 | 95 |
| | 83 | 0.07 | 96 |
| | 94 | 0.07 | 96 |
| | 86 | 0.09 | 97 |
| | 91 | 0.10 | 98 |

**Table 7**

| **Composition** | **Particle size (nm)** | **Polydispersity Index** | **% Encaps of mLuc** |
|---|---|---|---|
| (7) | 151 | 0.08 | 49 |
| | 158 | 0.11 | 50 |
| | 119 | 0.14 | 69 |
| | 134 | 0.12 | 52 |
| | 134 | 0.11 | 56 |
| | 134 | 0.07 | 63 |

The structures of the PEG lipids and some additional aminolipids used herein are shown below.

### PEG-Lipids

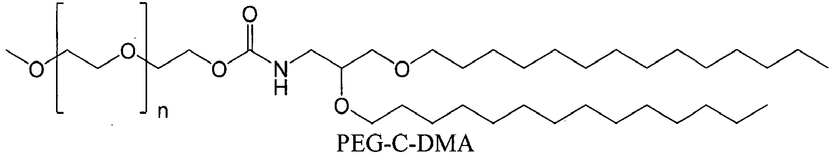

### Cationic Lipid

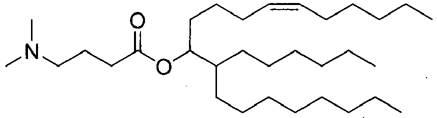

### (Z)-9-hexylicos-14-en-10-yl 4-(dimethylamino)butanoate 17

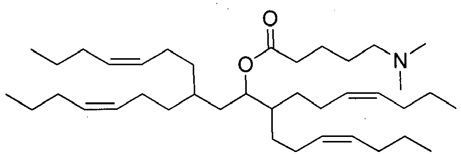

### (4Z,14Z)-8,11-di((Z)-hept-3-enyl)octadeca-4,14-dien-9-yl 5-(dimethylamino)pentanoate 18

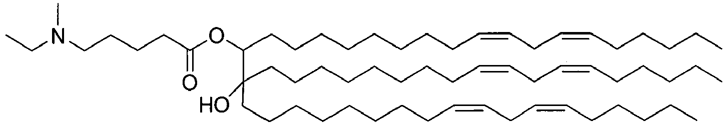

### (6Z,9Z,29Z,32Z)-20-hydroxy-20-((9Z,12Z)-octadeca-9,12-dienyl)octatriaconta-6,9,29,32-tetraen-19-yl 5-(ethyl(methyl)amino)pentanoate 10

### Example 3 Preparation of Cationic Lipid (111)

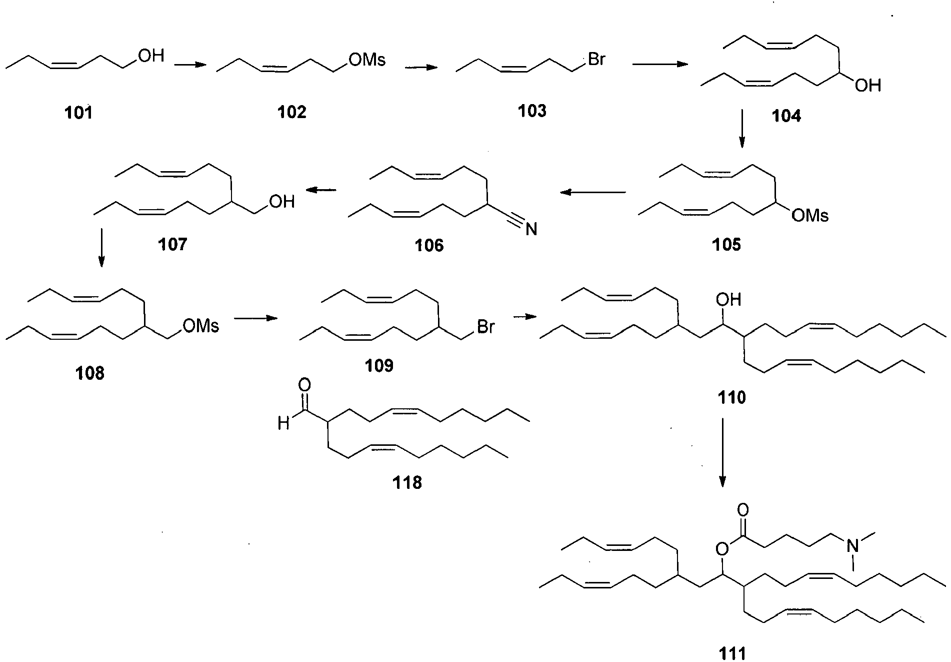

A solution of (3Z, 13Z)-7-((Z)-hex-3-en-1-yl)-10-((Z)-non-3-en-1-yl)nonadeca-3,13-dien-9-ol (**110**, 700 mg, 1.44 mmol) in CH₂Cl₂ (10 mL) was successively treated with 5-bromovaleric acid (390 mg, 2.16 mmol), EDC (413 mg, 2.2 mmol) and DMAP (10 mg) and stirred (30°C, 18H). The solution was diluted with CH₂Cl₂ and washed with saturated NaHCO₃ and brine, dried (MgSO₄), filtered and concentrated. The crude material was taken-up in dimethylamine in ethanol (10mL as a 2M solution) placed in a sealed vessel and heated (80°C, 5H). Once complete the solution was concentrated and the crude material was subjected to chromatography (EtOAc) to yield **111** (294 mg, 22%) as a pale yellow oil. ¹H NMR (400MHz, CDCl₃, δ_{H}) 5.54-5.28 (m, 8 H), 5.15-5.05 (m, 1 H), 2.27-2.22 (m, 4 H), 2.20 (s, 6 H), 2.10-1.96 (m, 16 H), 1.71-1.44 (m, 9 H), 1.43-1.25 (23 H), 0.95 (t, 6 H), 0.87 (t, 6 H).

The intermediate compound (3Z,13Z)-7-((Z)-hex-3-en-1-yl)-10-((Z)-non-3-en-1-yl)nonadeca-3,13-dien-9-ol **110** was prepared as described below.
a. Synthesis of (Z)-hex-3-en-1-yl methanesulfonate **102** 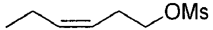
   To a solution of *cis*-3-hexen-1-ol **101** (12.9 g, 129 mmol) in dichloromethane (300 mL) was added triethylamine (50 mL). The solution was cooled to 0 °C and methanesulfonyl chloride (20 mL, 258 mmol) was added. The solution was stirred for 1.5 hours at room temperature and was then washed with saturated NaHCO₃, and then the aqueous layer was back extracted with dichloromethane. The combined organic extracts were dried (MgSO₄), filtered and concentrated *in vacuo* to dryness. The residue was filtered through a pad of silica (100% DCM) to afford (Z)-hex-3-en-1-yl methanesulfonate **102** as a pale yellow crude oil (22.6g, 98%). Rf 0.7 (CH₂Cl₂).
b. Synthesis of (Z)-1-bromohex-3-ene **103** 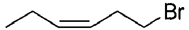
   A solution of (Z)-hex-3-en-1-yl methanesulfonate 2 (22.6 g, 127 mmol) in 2-methyltetrahydrofuran (300 mL) was heated to 80 °C and subsequently treated with tetrabutylammonium bromide (53.1 g, 165 mmol). After stirring (40 min) the mixture was cooled to 20°C and washed with ice water. The aqueous layer was back extracted with EtOAc (3 x) and the combined organics were washed with brine, dried (MgSO₄), filtered and concentrated. The crude product was filtered through a pad of silica rinsing with hexanes and then concentrated to yield (Z)-1-bromohex-3-ene **103** (20 g, 97%) as a yellow oil. Rf (0.8, hexanes).
c. Synthesis of (3Z,10Z)-trideca-3,10-dien-7-ol **104** 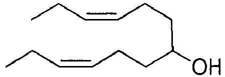
   A suspension of magnesium turnings (1.82 g, 74.8 mmol) in anhydrous tetrahydrofuran (10 mL) under nitrogen was treated with a solution of (Z)-1-bromohex-3-ene **103** (11.4 g, 69.9 mmol) in tetrahydrofuran (15 mL). The reaction was stirred at 45°C for 2 hours. The solution was then cooled to 5-10°C and a solution of ethyl formate (5.8 mL, 72 mmol) in tetrahydrofuran (15 mL) was added drop wise. The solution was stirred at room temperature for 15 minutes, then cooled to 5°C and quenched with water (30 mL) followed by the slow addition of 6M hydrochloric acid (30 mL). The solution was stirred at room temperature until all the magnesium had dissolved then treated with hexanes (50mL) and water (50mL). The combined hexanes extracts were dried (MgSO₄), filtered and concentrated *in vacuo* to dryness. The residue was dissolved in ethanol (45 mL) then a solution of potassium hydroxide (3.53 g, 62.9 mol) in water (15 mL) was added. The solution was stirred vigorously for 5 minutes at room temperature then concentrated *in vacuo* to remove the ethanol. The solution was made acidic with 6M HCl (40 mL) and water was added (40 mL) to dissolve the KCl then it was extracted with hexanes. The combined hexanes extracts were dried (MgSO₄), filtered and concentrated *in vacuo* to dryness. The product was purified by column chromatography (100% hexanes to 5% ethyl acetate in hexanes) to afford (3Z,10Z)-trideca-3,10-dien-7-ol **104** as a pale yellow oil (4.75 g, 65%). Rf 0.4 (10% EtOAc-Hexanes).
d. Synthesis of (3Z,10Z)-trideca-3,10-dien-7-yl methanesulfonate **105** 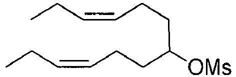
   A solution of the alcohol **104** (4.75 g, 22.7 mmol) in dichloromethane (100 mL) with triethylamine (20 mL) was cooled to 0°C and treated with methanesulfonyl chloride (3.5 mL, 45.3 mmol). The solution was stirred for 1 hour at room temperature and was then washed with saturated sodium bicarbonate and water:brine (1:1). The organics were then dried (MgSO₄), filtered, concentrated to dryness. The crude residue was filtered through a pad of silica (CH₂Cl₂) to afford (3Z,10Z)-trideca-3,10-dien-7-yl methanesulfonate **105** (5.95 g, 91%). Rf 0.9 (CH₂Cl₂).
e. Synthesis of (Z)-2-((Z)-hex-3-en-1-yl)oct-5-enenitrile **106** 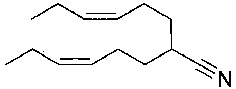
   A solution of the mesylate **105** (5.95 g, 20.6 mmol) in N,N dimethylformamide (125 mL) was treated with powdered sodium cyanide (2.53 g, 51.6 mmol) and stirred overnight at 60°C. The reaction mixture was cooled to 20°C and treated with water to dissolve the excess NaCN and stirred for 30 minutes. The solution was then separated into two layers by the addition of brine and EtOAc. The aqueous layer was back extracted with EtOAc and the combined organics were washed with brine (3 x), dried (MgSO₄), filtered and concentrated. The crude residue was filtered through a pad of silica rinsing with hexanes:CH₂Cl₂ (1:1) to yield (Z)-2-((Z)-hex-3-en-1-yl)oct-5-enenitrile **106** (3.11 g, 69%). Rf 0.5 (1:1 hexanes:CH₂Cl₂).
f. Synthesis of (Z)-2-((Z)-hex-3-en-1-yl)oct-5-en-1-ol **107** 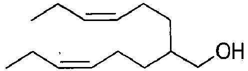
   A solution of the nitrile **106** (3.11 g, 14.2 mmol) in dichloromethane (80 mL) was cooled to -8 °C and slowly treated with DIBAL (28.3 mL, 28.3 mmol; as a 1M solution in CH₂Cl₂). After stirring (2h) the reaction was quenched by the addition of 5% HCl (25 mL) and extracted with CH₂Cl₂ and concentrated. The residue was taken up in methanol (80 mL), cooled (0°C) and treated with NaBH₄ (1.07g, 28.3mmol). After stirring (30min) the reaction was quenched by the addition of 5% HCl and extracted with CH₂Cl₂. The organics were washed with water and brine, dried (MgSO₄), filtered and concentrated. The crude material was purified via chromatography (hexanes → 5 % EtOAc-hexanes) to yield (Z)-2-((Z)-hex-3-en-1-yl)oct-5-en-1-ol **107** (2.8g, 88%). Rf 0.75 (10% EtOAc-hexanes).
g. Synthesis of (Z)-2-((Z)-hex-3-en-1-yl)oct-5-en-1-yl methanesulfonate **108** 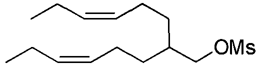
   To a solution of **107** (2.8 g, 12.5 mmol) in dichloromethane (40 mL) was added triethylamine (5 mL). The solution was cooled to 0 °C and methanesulfonyl chloride (1.93 mL, 25 mmol) was added. The solution was stirred for 1.5 hours at room temperature and was then washed with saturated NaHCO₃, and then the aqueous layer was back extracted with dichloromethane. The combined organic extracts were dried (MgSO₄), filtered and concentrated *in vacuo* to dryness. The residue was filtered through a pad of silica (100% DCM) to afford (Z)-2-((Z)-hex-3-en-1-yl)oct-5-en-1-yl methanesulfonate **108** as a pale yellow crude oil (3.6g, 95%). Rf 0.75 (CH₂Cl₂).
h. Synthesis of (3Z,10Z)-7-(bromomethyl)trideca-3,10-diene **109** 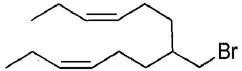
   A solution of **108** in 2-methyltetrahydrofuran (30 mL) was heated to 80°C and subsequently treated with tetrabutylammonium bromide (5 g, 15.5 mmol). After stirring (40 min) the mixture was cooled to 20°C and washed with ice water. The aqueous layer was back extracted with EtOAc (3 x) and the combined organics were washed with brine, dried (MgSO₄), filtered and concentrated. The crude product was filtered through a pad of silica rinsing with hexanes and then concentrated to yield (3Z,10Z)-7-(bromomethyl)trideca-3,10-diene **109** (2.13 g, 62%) as a yellow oil. Rf (0.8, hexanes).
i. Synthesis of (3Z,13Z)-7-((Z)-hex-3-en-1-yl)-10-((Z)-non-3-en-1-yl)nonadeca-3,13-dien-9-ol **110** 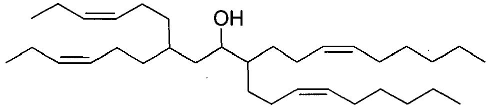
   A mixture of Mg (193 mg, 7.94 mmol) in THF (2 mL) was treated with a solution of the bromide **109** (2.13 g, 7.42 mmol) in THF (4 mL) and heated (45°C, 4 h). The Grignard reagent was then cooled (20°C) and treated with a solution of the aldehyde (**118**, 4.65 g, 15.9 mmol) in THF (10mL). After stirring (2h) the mixture was cooled (5°C) and quenched by the addition of water (5 mL) then 6M HCl (5 mL) and stir until the excess magnesium was consumed then treated with hexanes and water. The organic layer was dried (MgSO₄), filtered, concentrated and purified via chromatography (hexanes → 2% → 5% EtOAc-hexanes) to yield (3Z,13Z)-7-((Z)-hex-3-en-1-yl)-10-((Z)-non-3-en-1-yl)nonadeca-3,13-dien-9-ol **110** (700 mg, 19%). Rf 0.6(10% EtOAc-hexanes).
   The intermediate compound (Z)-2-((Z)-non-3-en-1-yl)undec-5-enal **118** was prepared as illustrated and described below. 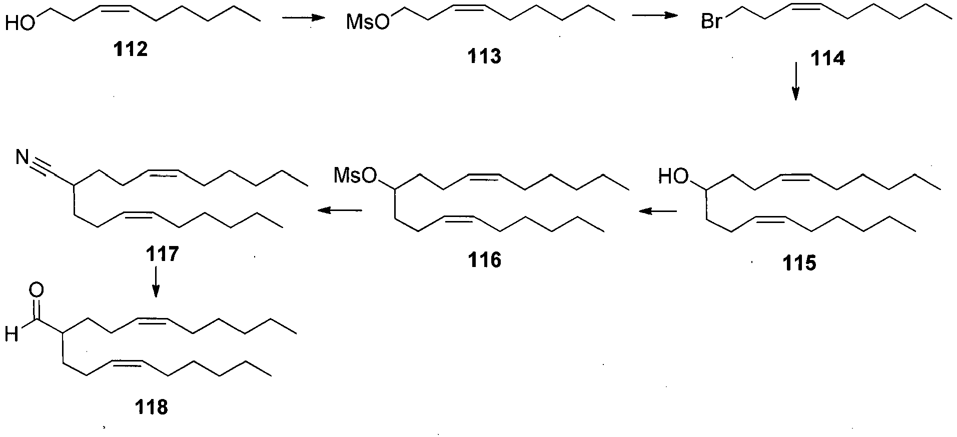
j. Synthesis of (Z)-non-3-en-1-yl methanesulfonate **113.** In the same manner as Compound **102**, Compound **113** was prepared from Z-non-3-en-1-ol (65.5 g, 461 mmol), methanesulphonyl chloride (39 mL, 507 mmol), triethylamine (78 mL, 576 mmol).
k. Synthesis of (Z)-1-bromonon-3-ene **114**. In the same manner as Compound **103**, Compound **114** was prepared from (Z)-non-3-en-1-yl methanesulfonate (101 g, 459 mmol) and TBAB (183 g, 569 mmol). Yield of 14 (92 g, 97%).
l. Synthesis of (6Z,13Z)-nonadeca-6,13-dien-10-ol **115.** In the same manner as Compound **104**, Compound **115** was prepared from (Z)-1-bromonon-3-ene 14 (45 g, 219.5 mmol), magnesium (5.9 g, 241.5 mmol), ethylformate (17.1 g, 230.5 mmol) and potassium hydroxide (17.1 g, 230.5 mmol). Yield (11.2 g, 37%).
m. Synthesis of (6Z,13Z)-nonadeca-6,13-dien-10-yl methanesulfonate **116.** In the same manner as Compound **105**, Compound **116** was prepared from (6Z,13Z)-nonadeca-6,13-dien-10-ol 11.2 g, 40 mmol), methanesulphonyl chloride (3.4 mL, 44 mmol) and triethylamine (8.7 mL, 60 mmol). Yield (14.4 g, quantitative).
n. Synthesis of (Z)-2-((Z)-non-3-en-1-yl)undec-5-enenitrile **117**. In the same manner as Compound **106**, Compound **116** was prepared from (6Z,13Z)-nonadeca-6,13-dien-10-yl methanesulfonate 14.4 g, 40 mmol) and potassium cyanide (6.6 g, 100 mmol). Yield (10 g, 87%).
o. Synthesis of (Z)-2-((Z)-non-3-en-1-yl)undec-5-enal **118**. A solution of the nitrile **117** (10 g, 34.5 mmol) in dichloromethane (350 mL) was cooled to -8 °C and slowly treated with DIBAL (86.3 mL, 86.3 mmol; as a 1M solution in CH₂Cl₂). After stirring (2h) the reaction was quenched by the addition of 5% HCl (25 mL) and extracted with CH₂Cl₂ and concentrated. The residue was subjected to chromatography (2% EtOAc-hexanes) to yield Compound **118** (5.1 g, 50%).

### Example 4 Preparation of (3Z,13Z)-10-((Z)-hept-3-en-1-yl)-7-((Z)-hex-3-en-1-yl)heptadeca-3,13-dien-9-yl 5-(dimethylamino)pentanoate 130

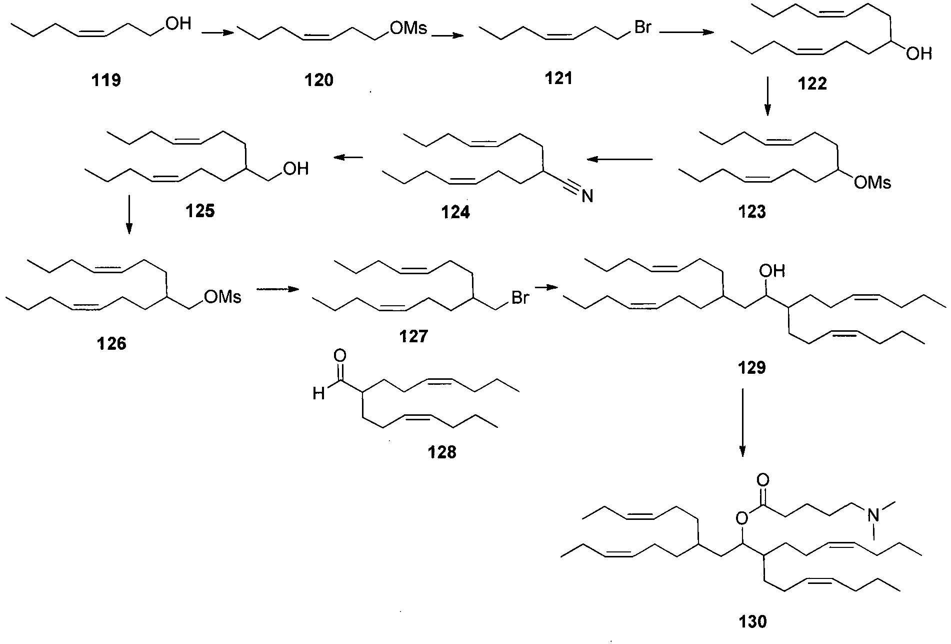

In the same manner as Compound **111**, Compound **130** was prepared from (4Z,14Z)-8,11-di((Z)-hept-3-en-1-yl)octadeca-4,14-dien-9-ol **129** (223 mg, 0.49 mmol), 5-bromovaleric acid (264 mg, 14.6 mmol), EDC (279 mg, 14.6 mmol) then dimethylamine. Yield of Compound **130** (138 mg, 51%, 2 steps). ¹H NMR (400MHz, CDCl₃, δ_{H}) 5.41-5.29 (m, 8 H), 5.13-5.07 (m, 1 H), 2.38-2.24 9M, 4 h), 2.22 (s, 6 H), 2.14-1.93 (m, 16 H), 1.67-1.45 (m, 6 H), 1.45-1.23 (m, 19 H), 0.92 (t, 6 H).

The intermediate (4Z,14Z)-8,11-di((Z)-hept-3-en-1-yl)octadeca-4,14-dien-9-ol **129** was prepared as described below.
a. Synthesis of (Z)-hept-3-en-1-yl methanesulfonate **120.** In the same manner as Compound **102**, Compound **120** was prepared from Z-3-hepten-1-ol (52 g, 455 mmol), triethylamine (80 mL) and methanesulphonyl chloride (70.5 mL, 911 mmol). Yield (87.4 g, quantitative).
b. Synthesis of (Z)-1-bromohept-3-ene **21**. In the same manner as Compound **103**, Compound **121** was prepared from (Z)-hept-3-en-1-yl methanesulfonate **120** and TBAB (190.7 g, 592 mmol). Yield (60.9 g, 76 %).
c. Synthesis of (4Z,11Z)-pentadeca-4,11-dien-8-ol **122**. In the same manner as Compound **104**, Compound **122** was prepared from (Z)-1-bromohept-3-ene 21 (22 g, 124 mmol), magnesium (3.23 g, 13.3 mmol), ethyl formate (10.3 mL, 128 mmol) and potassium hydroxide (6.28 g, 112 mmol). Yield of Compound **122** (11.83 g, 85%).
d. Synthesis of (4Z,11Z)-pentadeca-4,11-dien-8-yl methanesulfonate **123.** In the same manner as Compound **105**, Compound **123** was prepared from (4Z,11Z)-pentadeca-4,11-dien-8-ol 22 (11.8g, 50 mmol), triethylamine (15 mL) and methanesulphonyl chloride (7.7 mL, 100 mmol). Yield of Compound **123** (15.1 g, quantitative).
e. Synthesis of (Z)-2-((Z)-hept-3-en-1-yl)non-5-enenitrile **124**. n the same manner as Compound **106**, Compound **124** was prepared from (4Z,11Z)-pentadeca-4,11-dien-8-yl methanesulfonate **123** (15.1 g, 50 mmol) and sodium cyanide (6.4 g, 130 mmol). Yield of Compound **124** (9.87 g, 85%).
f. Synthesis of (Z)-2-((Z)-hept-3-en-1-yl)non-5-en-1-ol (**125**). In the same manner as Compound **107**, Compound **125** was prepared from (Z)-2-((Z)-hept-3-en-1-yl)non-5-enenitrile **124** (9.87 g, 42 mmol), DIBAL (85 mL, 85 mmol) then NaBH₄ (3.2 g, 85 mmol). Yield of Compound **125** (6 g, 60%).
g. Synthesis of (Z)-2-((Z)-hept-3-en-1-yl)non-5-en-1-yl methanesulfonate **126.** In the same manner as Compound **108**, Compound **126** was prepared from (Z)-2-((Z)-hept-3-en-1-yl)non-5-en-1-ol **125** (6 g, 25 mmol), triethylamine (10 mL) and methanesulphonyl chloride (3.9 mL, 50.3 mmol). Yield of Compound **126** (7.8 g, 97%).
h. Synthesis of (4Z,11Z)-8-(bromomethyl)pentadeca-4,11-diene **127.** In the same manner as Compound **109**, Compound **127** was prepared from (Z)-2-((Z)-hept-3-en-1-yl)non-5-en-1-yl methanesulfonate **126** (7.8 g, 24.5 mmol) and TBAB (10.3 g, 31.8 mmol). Yield of Compound **127** (7 g, 95%).
i. Synthesis of (Z)-2-((Z)-hept-3-en-1-yl)non-5-enal **128.** In the same manner as Compound **118,** Compound **128** was prepared from (Z)-2-((Z)-hept-3-en-1-yl)non-5-enenitrile **124** (8.6 g, 36.8 mmol) and DIBAL (44.2 mL, 44.2 mmol). Yield of Compound **128** (5.8 g, 67%).
j. Synthesis of (4Z,14Z)-8,11-di((Z)-hept-3-en-1-yl)octadeca-4,14-dien-9-ol **129**. In the same manner as Compound **110**, Compound **129** was prepared from (4Z,11Z)-8-(bromomethyl)pentadeca-4,11-diene (3.8 g, 12.6 mmol), magnesium and (Z)-2-((Z)-hept-3-en-1-yl)non-5-enal **128** (642 mg, 2.7 mmol). Yield of Compound **129** (223 mg, 18%).

### Example 5 Preparation of 9,13-dihexylhenicosan-11-yl 5-(dimethylamino)pentanoate 135

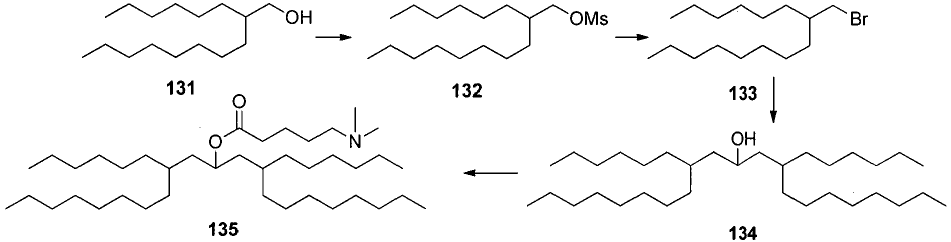

In the same manner as Compound **111**, Compound **135** was prepared from 9,13-dihexylhenicosan-11-ol **134** (1.6 g, 3.3 mmol), 5-bromovaleric acid (1.81 g, 9.9 mmol), EDC (1.92 g, 9.9 mmol) and DIPEA (1.74 mL, 9.9 mmol) then dimethylamine in ethanol (8mL). Yield (1.3 g, 65%). ¹H NMR (400MHz, CDCl₃, δ_{H}) 5.08-5.00 (m, 1 H), 2.34-2.23 (m, 4 H), 2.24 (s, 6 H), 1.68-1.59 (m, 4 H), 1.54-1.44 (m, 4 H), 1.40-1.16 (m, 54 H), 0.87 (t, 12 H).

The intermediate 9,13-dihexylhenicosan-11-ol **134** was prepared as described below.
a. Synthesis of 2-hexyldecyl methanesulfonate **132.** In the same manner as Compound **102**, Compound **132** was prepared from 2-hexyldecan-1-ol (10 g, 41 mmol), triethylamine (6.4 mL, 48 mmol) and methanesulphonyl chloride (6.35 mL, 82 mmol). Yield (13 g, quantitative).
b. Synthesis of 7-(bromomethyl)pentadecane **133.** In the same manner as Compound **103**, Compound **133** was prepared from 2-hexyldecyl methanesulfonate 13.1 g, 41 mmol) and TBAB (17.2 g, 53.3 mmol). Yield (11.9 g, 95%).
c. Synthesis of 9,13-dihexylhenicosan-11-ol **134**. In the same manner as Compound **104**, Compound **134** was prepared from 7-(bromomethyl)pentadecane Compound 133 (11.9 g, 39 mmol), magnesium (1.05 g, 43 mmol), ethyl formate (3.3 mL, 41 mmol) and potassium hydroxide (1.98 g, 35 mmol). Yield (6.1 g, 67%).

### Example 6 Preparation of 9,13-dihexylhenicosan-11-yl 6-(dimethylamino)hexanoate 137

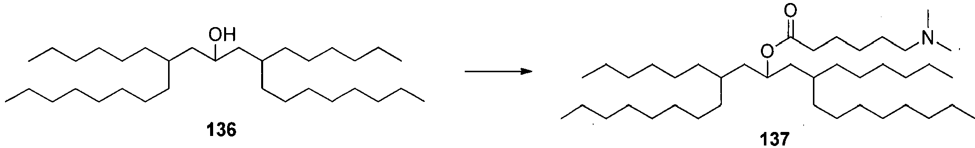

In the same manner as Compound **111, 137** was prepared from 9,13-dihexylhenicosan-11-ol 34 (1.6 g, 3.3 mmol), 6-bromocaproic acid (1.95 g, 9.9 mmol), EDC (1.92 g, 9.9 mmol) and DIPEA (1.74 mL, 9.9 mmol) then dimethylamine in ethanol (8mL). Yield (1.2 g, 59%).%). ¹H NMR (400MHz, CDCl₃, δ_{H}) 5.08-5.00 (m, 1 H), 2.30-2.23 (m, 4 H), 2.22 (s, 6 H), 1.68-1.59 (m, 4 H), 1.57-1.43 (m, 4 H), 1.39-1.15 (m, 56 H), 0.87 (t, 12 H).

### Example 7 Preparation of (Z)-7-butyl-10-((Z)-dec-4-en-1-yl)icos-14-en-9-yl 5-(dimethylamino)pentanoate 143

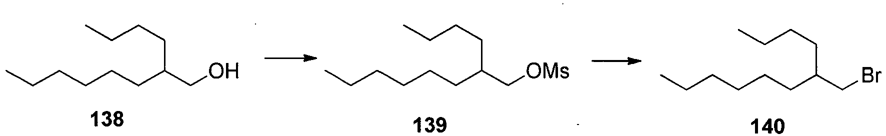

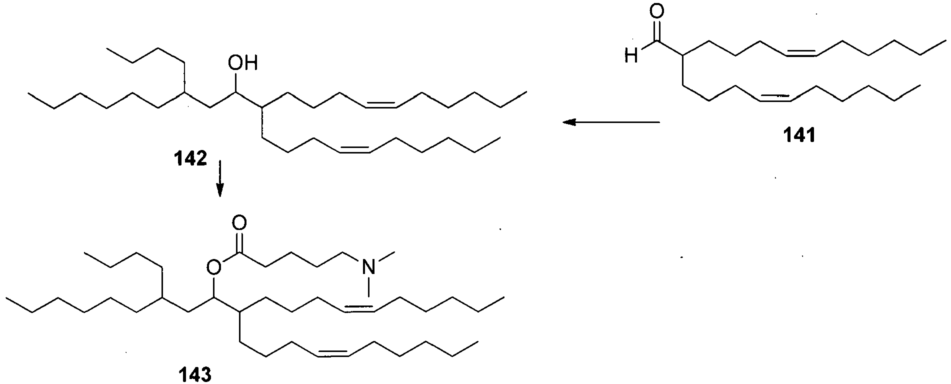

In the same manner as Compound **111**, Compound **143** was prepared from (Z)-7-butyl-10-((Z)-dec-4-en-1-yl)icos-14-en-9-ol (**142**, 132 mg, 0.027mmol), N,N,Dimethyl-aminobutyric acid hydrochloride (136 mg, 0.81 mmol), EDC (155 mg, 0.81 mmol) and DIPEA (200µL). Yield (100 mg, 58%).

The intermediate (Z)-7-butyl-10-((Z)-dec-4-en-1-yl)icos-14-en-9-ol **142** was prepared as described below.
a. Synthesis of 2-butyloctyl methanesulfonate **139.** In the same manner as Compound **102**, Compound **139** was prepared from 2-butyloctan-1-ol **138** (3 g, 16.1 mmol), triethylamine (8 mL), methanesulphonyl chloride (2.49 mL, 32.2 mmol). Yield (4.2 g, 99%).
b. Synthesis of 5-(bromomethyl)undecane **140.** In the same manner as Compound **103**, Compound **140** was prepared from 2-butyloctyl methanesulfonate **139** (4.2 g, 16 mmol) and TBAB (6.75 g, 20.9 mmol). Yield (3.67 g, 92%).
c. Synthesis of (Z)-7-butyl-10-((Z)-dec-4-en-1-yl)icos-14-en-9-ol **142**. In the same manner as Compound **110**, Compound **142** was prepared from 5-(bromomethyl)undecane **140** (3.67 g, 14.7 mmol) and (Z)-2-((Z)-dec-4-en-1-yl)dodec-6-enal **141** (1.6 g, 5 mmol). Yield (132 mg, 5%).

### Example 8

The data in Table 8 demonstrates that particles comprising representative tetraalkyl lipids provide greater luciferase activity than particles comprising a general dialkyl lipid control compound **8** when they are utilized in the assay described in Example 1. In particular, the tetraalkyl lipids mediated an enhanced level of delivery of luciferase mRNA to living cells than the established benchmark dialkyl lipid **8** shown in Table 8.

**Table 8**

| **Compound number** | **Structure** | **Activity in luciferase assay (pg/g liver tissue)** |
|---|---|---|
| **8** | 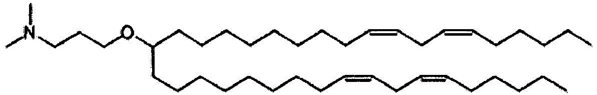 | 774,159 |
| **11** | 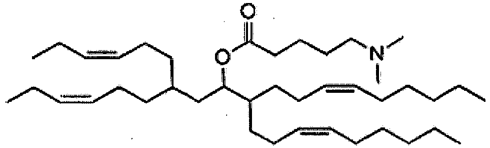 | 927,344 |
| **30** | 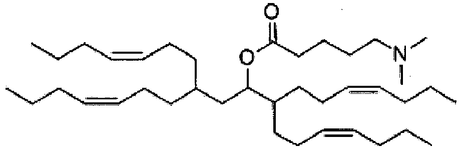 | 883,124 |
| **35** | 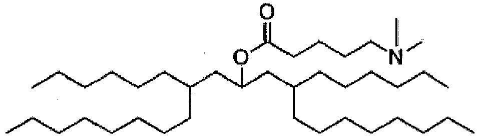 | 1,412,464 |
| **37** | 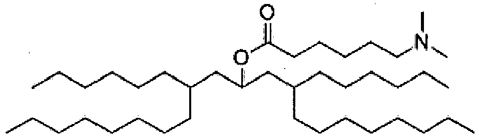 | 2,228,526 |
| **43** | 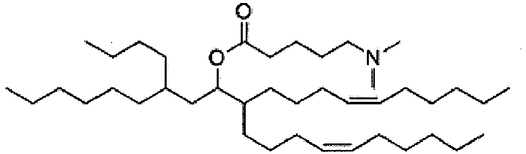 | 919,590 |

### Example 9.

This Example shows that lipid particles that are formulated with general trialkyl lipid **13** have a smaller average particle diameter (Zavg), over a wider range of lipid:mRNA ratios, compared to lipid particles that are formulated with reference dialkyl lipid **8.** Smaller particles size is typically desirable for *in vivo* administration of lipid particles of the present invention.

Lipid particles were made using the general formulating procedure described in Example 2. Formulations with a lipid to drug (mRNA) ratio of 20:1 employed a 12.6 mM lipid stock. Formulations with lower lipidtodrug ratios (13:1, 9:1, 6:1) used an appropriately diluted lipid stock. Lipid particles comprising the general short chain trialkyl compound **13** were diluted into a volume of phosphate buffered saline (PBS) such that the concentration of ethanol at this point was 17%. As shown in Table 9, smaller lipid particles were readily obtained when formulating with compound **13** and using a range of total lipid:mRNA ratios. When employing the dilinoleyl lipid **8**-however, a higher concentration of ethanol was required (25%), and therefore the nascent particles were diluted into a smaller quantity of PBS. As the lipid-to-drug ratio was reduced, particle sizes were larger; an unwanted property for in vivo applications because larger particle size is often associated with unwanted toxicities. The short trialkyl lipid **13**-consistently yielded smaller particles than the longer chain dilinoleyl lipid **8**. Compound**13** produced lipid particles having Zavg below 100 nm for lipid-to-drug ratios as low as 9:1.

**Table 9**

| **Formulation** | **L:D** | **[EDTA]** | **Blend Rate** | **Final % EtOH** | **Payload** | **Zavg** |
|---|---|---|---|---|---|---|
| 1.6:55 **13** | **20:1** | **40 mM** | **250 mL/min** | **17%** | **mLuc** | **78** |
| | **13:1** | | | | | **80** |
| | **9:1** | | | | | **95** |
| | **6:1** | | | | | **119** |
| 1.6:55 **8** | **20:1** | | | **25%** | | **89** |
| | **13:1** | | | | | **114** |
| | **9:1** | | | | | **120** |
| | **6:1** | | | | | **122** |

## Claims

1. A compound having structural formula C:
X-A-Y-Z¹; (Formula C)
or a salt thereof, wherein:
X is -N(H)R or -NR₂;
A is absent, C₁ to C₆alkyl, C₂ to C₆alkenyl, or C₂ to C₆alkynyl, which C₁ to C₆alkyl, C₂ to C₆alkenyl, and C₂ to C₆alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein each alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}. -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle;
Y is selected from the group consisting of absent, -C(=O)-, -O-, -OC(=O)-, -C(=O)O-, -N(R^{b})C(=O)-, -C(=O)N(R^{b})-, -N(R^{b})C(=O)O-, and -OC(=O)N(R^{b})-;
Z¹ is a C₁ to C₆alkyl that is substituted with four R^{x} groups, wherein each R^{x} is independently selected from C₆ to C₁₁alkyl, C₆ to C₁₁alkenyl, and C₆ to C₁₁alkynyl, which C₆ to C₁₁alkyl, C₆ to C₁₁alkenyl, and C₆ to C₁₁alkynyl is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SOₙNR^{x'}R^{y'}, wherein n' is 0, 1, or 2, and R^{x'} and R^{y'} are each independently hydrogen, alkyl, or heterocycle;
each R is independently alkyl, alkenyl, or alkynyl, that is optionally substituted with one or more groups independently selected from oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'}, and -SO_{n'}NR^{x'}R^{y'}, wherein n' is 0, 1, or 2, and R^{x'} and R^{y'} are each independently hydrogen, alkyl, or heterocycle; and
each R^{b} is H or C₁ to C₆alkyl.

2. The compound of claim 1 wherein Z¹ has the structure: 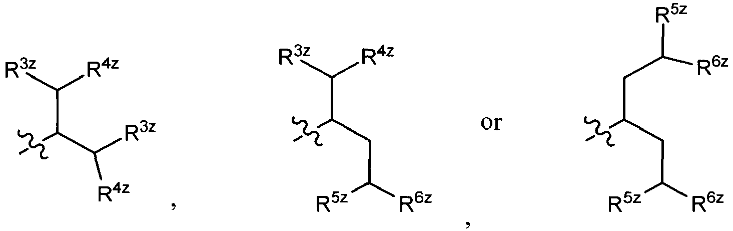 wherein each R^{3z}, R^{4z}, R^{5z}, and R^{6z} is independently selected from C₆ to C₁₁alkyl, C₆ to C₁₁alkenyl, and C₆ to C₁₁alkynyl, which C₆ to C₁₁alkyl, C₆ to C₁₁alkenyl, and C₆ to C₁₁alkynyl is optionally substituted with one or more groups independently selected from oxo, halogen, heterocycle, -CN, -OR^{x}, -NR^{x}R^{y}, -NR^{x}C(=O)R^{y}. -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, and R^{x} and R^{y} are each independently hydrogen, alkyl, or heterocycle, wherein any alkyl and heterocycle of R^{x} and R^{y} may be further substituted with one or more groups independently selected from oxo, halogen, -OH, -CN, alkyl, -OR^{x'}, heterocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SOₙR^{x}, and -SO_{n'}NR^{x'}R^{y'}, wherein n' is 0, 1, or 2, and R^{x'} and R^{y'} are each independently hydrogen, alkyl, or heterocycle.

3. The compound of claim 1 that is selected from the group consisting of: 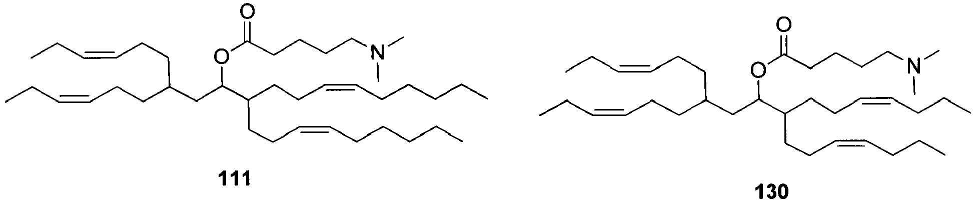 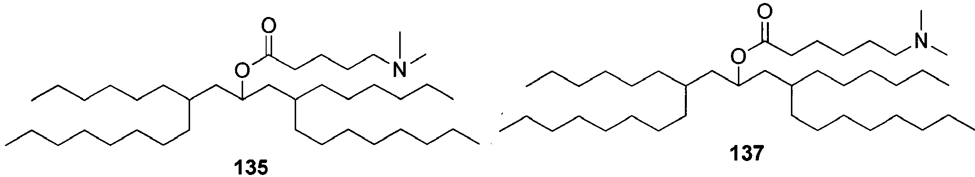 and

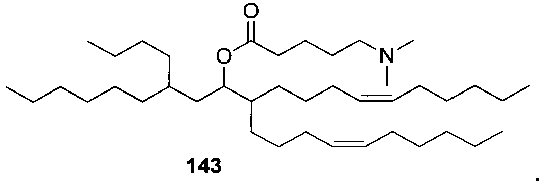

4. A lipid particle comprising a compound of any one of claims 1 to 3, a non-cationic lipid, and an mRNA molecule that is encapsulated within the lipid particle.

5. The lipid particle of claim 4, wherein the non-cationic lipid is selected from a PEG-lipid conjugate and a phospholipid.

6. The lipid particle of claim 4 or claim 5, wherein the lipid particle further comprises cholesterol.

7. The lipid particle of claim 5, wherein the phospholipid comprises dipalmitoylphosphatidylcholine (DPPC), distearoylphosphatidylcholine (DSPC), or a mixture thereof; and wherein the PEG-lipid conjugate is selected from the group consisting of a PEG-diacylglycerol (PEG-DAG) conjugate, a PEG-dialkyloxypropyl (PEG-DAA) conjugate, a PEG-phospholipid conjugate, a PEG-ceramide (PEG-Cer) conjugate, a mixture thereof, PEG-DMA, and PEG-DSA.

8. The lipid particle of any one of claims 4 to 7, wherein the particle has a lipid:mRNA mass ratio of from about 9:1 to about 20:1.

9. The lipid particle of any one of claims 4 to 6, wherein the compound of any one of claims 1 to 4 comprises from about 50 mol% to about 65 mol% of the total lipid present in the particle.

10. The lipid particle of any one of claims 5 to 9, wherein the phospholipid comprises from about 4 mol% to about 15 mol% of the total lipid present in the particle.

11. The lipid particle of any one of claims 6, and 8 to 10, wherein the cholesterol comprises from about 30 mol% to about 40 mol% of the total lipid present in the particle.

12. The lipid particle of any one of claims 5 to 11, wherein the PEG-lipid comprises from about 0.5 mol% to about 2 mol% of the total lipid present in the particle.

13. The lipid particle of any one of claims 5 to 12, wherein the lipid particle comprises an electron dense core and wherein the mRNA is located within the electron dense core.

14. The lipid particle of claim 13, wherein the electron dense core comprises an aqueous component and a lipid component, wherein the amount of the aqueous component is less than the amount of the lipid component.

15. A pharmaceutical composition comprising a lipid particle of any one of claims 5 to 14 and a pharmaceutically acceptable carrier.

16. A lipid particle of any one of claims 5 to 14 for use in a method for introducing an mRNA that encodes a protein into a cell, wherein the cell is in a mammal.

17. A lipid particle of any one of claims 5 to 14 for use in a method for treating and/or ameliorating one or more symptoms associated with a disease, in a human, caused by impaired expression of a protein in the human, wherein the mRNA encapsulated within the lipid particle encodes the protein.

18. A lipid particle comprising (a) one or more mRNA molecule(s); (b) a compound of any one of claims 1 to 3 comprising from about 30 mol% to about 50 mol% of the total lipid present in the particle; (c) a mixture of a phospholipid and cholesterol or a derivative thereof comprising from about 47 mol% to about 69 mol% of the total lipid present in the particle; and (d) a PEG-lipid conjugate comprising from about 1 mol% to about 3 mol% of the total lipid present in the particle.

## Patentansprüche

1. Verbindung mit Strukturformel C:
X-A-Y-Z¹; (Formel C)
oder ein Salz davon, wobei:
X -N(H)R oder -NR₂ darstellt;
A abwesend ist, C₁- bis C₆-Alkyl, C₂- bis C₆-Alkenyl oder C₂- bis C₆-Alkynyl darstellt, wobei C₁- bis C₆-Alkyl, C₂- bis C₆-Alkenyl und C₂- bis C₆-Alkynyl optional mit einer oder mehreren Gruppen, unabhängig ausgewählt aus Oxo, Halogen, Heterocyclus, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} und -SOₙNR^{x}R^{y}, substituiert ist, wobei n 0, 1 oder 2 beträgt und R^{x} und R^{y} jeweils unabhängig voneinander Wasserstoff, Alkyl oder Heterocyclus darstellen, wobei jedes/r Alkyl und Heterocyclus von R^{x} und R^{y} weiter mit einer oder mehreren Gruppen, unabhängig ausgewählt aus Oxo, Halogen, -OH, -CN, Alkyl, -OR^{x'}, Heterocyclus, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} und -SO_{n'}NR^{x'}R^{y'}, substituiert sein kann, wobei n' 0, 1 oder 2 beträgt und R^{x'} und R^{y'} jeweils unabhängig voneinander Wasserstoff, Alkyl oder Heterocyclus darstellen;
Y aus der Gruppe, bestehend aus
abwesend, -C(=O)-, -O-, -OC(=O)-, -C(=O)O-, -N(R^{b})C(=O)-, -C(=O)N(R^{b})-, -N(R^{b})C(=O)O- und -OC(=O)N(R^{b})-, ausgewählt ist;
Z¹ ein C₁- bis C₆-Alkyl darstellt, das mit vier R^{x}-Gruppen substituiert ist, wobei jedes R^{x} unabhängig aus C₆- bis C₁₁-Alkyl, C₆- bis C₁₁-Alkenyl und C₆- bis C₁₁-Alkynyl ausgewählt ist, wobei C₆- bis C₁₁-Alkyl, C₆- bis C₁₁-Alkenyl und C₆- bis C₁₁-Alkynyl optional mit einer oder mehreren Gruppen, unabhängig ausgewählt aus Oxo, Halogen, Heterocyclus, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} und -SOₙNR^{x}R^{y}, substituiert ist, wobei n 0, 1 oder 2 beträgt und R^{x} und R^{y} jeweils unabhängig voneinander Wasserstoff, Alkyl oder Heterocyclus darstellen, wobei jedes/r Alkyl und Heterocyclus von R^{x} und R^{y} weiter mit einer oder mehreren Gruppen, unabhängig ausgewählt aus Oxo, Halogen, -OH, -CN, Alkyl, -OR^{x'}, Heterocyclus, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} und -SO_{n'}NR^{x'}R^{y'}, substituiert sein kann, wobei n' 0, 1 oder 2 beträgt und R^{x'} und R^{y'} jeweils unabhängig voneinander Wasserstoff, Alkyl oder Heterocyclus darstellen;
jedes R unabhängig Alkyl, Alkenyl oder Alkynyl darstellt, das optional mit einer oder mehreren Gruppen, unabhängig ausgewählt aus Oxo, Halogen, Heterocyclus, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} und -SOₙNR^{x}R^{y}, substituiert ist, wobei n 0, 1 oder 2 beträgt und R^{x} und R^{y} jeweils unabhängig voneinander Wasserstoff, Alkyl oder Heterocyclus darstellen, wobei jedes/r Alkyl und Heterocyclus von R^{x} und R^{y} weiter mit einer oder mehreren Gruppen, unabhängig ausgewählt aus Oxo, Halogen, -OH, -CN, Alkyl, -OR^{x'}, Heterocyclus, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} und -SO_{n'}NR^{x'}R^{y'}, substituiert sein kann, wobei n' 0, 1 oder 2 beträgt und R^{x'} und R^{y'} jeweils unabhängig voneinander Wasserstoff, Alkyl oder Heterocyclus darstellen; und
jedes R^{b} H oder C₁- bis C₆-Alkyl darstellt.

2. Verbindung nach Anspruch 1, wobei Z¹ die Struktur aufweist: 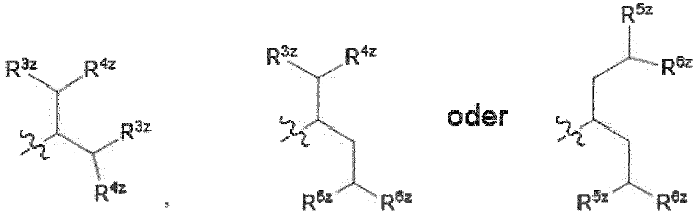 wobei jedes R^{3z}, R^{4z}, R^{5z} und R^{6z} unabhängig aus C₆- bis C₁₁-Alkyl, C₆- bis C₁₁-Alkenyl und C₆- bis C₁₁-Alkynyl ausgewählt ist, wobei C₆- bis C₁₁-Alkyl, C₆- bis C₁₁-Alkenyl und C₆- bis C₁₁-Alkynyl optional mit einer oder mehreren Gruppen, unabhängig ausgewählt aus Oxo, Halogen, Heterocyclus, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} und -SOₙNR^{x}R^{y}, substituiert ist, wobei n 0, 1 oder 2 beträgt und R^{x} und R^{y} jeweils unabhängig voneinander Wasserstoff, Alkyl oder Heterocyclus darstellen, wobei jedes/r Alkyl und Heterocyclus von R^{x} und R^{y} weiter mit einer oder mehreren Gruppen, unabhängig ausgewählt aus Oxo, Halogen, -OH, -CN, Alkyl, -OR^{x'}, Heterocyclus, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} und -SO_{n'}NR^{x'}R^{y'}, substituiert sein kann, wobei n' 0, 1 oder 2 beträgt und R^{x'} und R^{y'} jeweils unabhängig voneinander Wasserstoff, Alkyl oder Heterocyclus darstellen.

3. Verbindung nach Anspruch 1, die ausgewählt ist aus der Gruppe, bestehend aus: 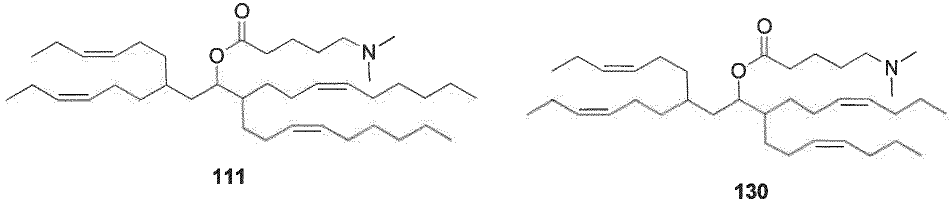 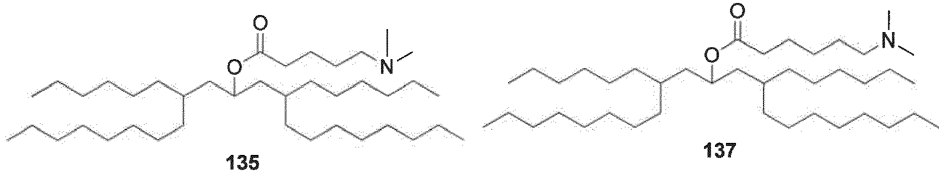 und

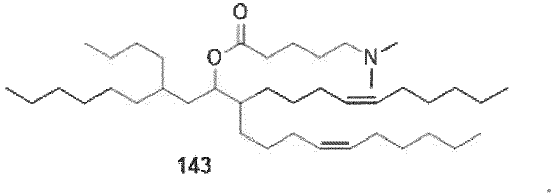

4. Lipidteilchen, umfassend eine Verbindung nach einem der Ansprüche 1 bis 3, ein nichtkationisches Lipid und ein mRNA-Molekül, das in dem Lipidteilchen eingekapselt ist.

5. Lipidteilchen nach Anspruch 4, wobei das nichtkationische Lipid aus einem PEG-Lipid-Konjugat und einem Phospholipid ausgewählt ist.

6. Lipidteilchen nach Anspruch 4 oder Anspruch 5, wobei das Lipidteilchen weiter Cholesterin umfasst.

7. Lipidteilchen nach Anspruch 5, wobei das Phospholipid Dipalmitoylphosphatidylcholin (DPPC), Distearoylphosphatidylcholin (DSPC) oder ein Gemisch davon umfasst; und wobei das PEG-Lipid-Konjugat aus der Gruppe, bestehend aus einem PEG-Diacylglycerol (PEG-DAG)-Konjugat, einem PEG-Dialkyloxypropyl (PEG-DAA)-Konjugat, einem PEG-Phospholipid-Konjugat, einem PEG-Ceramid (PEG-Cer)-Konjugat, einem Gemisch davon, PEG-DMA und PEG-DSA, ausgewählt ist.

8. Lipidteilchen nach einem der Ansprüche 4 bis 7, wobei das Teilchen ein Lipid:mRNA-Massenverhältnis von etwa 9:1 bis etwa 20:1 aufweist.

9. Lipidteilchen nach einem der Ansprüche 4 bis 6, wobei die Verbindung nach einem der Ansprüche 1 bis 4 etwa 50 Mol-% bis etwa 65 Mol-% des gesamten Lipids, vorhanden in dem Teilchen, umfasst.

10. Lipidteilchen nach einem der Ansprüche 5 bis 9, wobei das Phospholipid von etwa 4 Mol-% bis etwa 15 Mol-% des gesamten Lipids, vorhanden in dem Teilchen, umfasst.

11. Lipidteilchen nach einem der Ansprüche 6 und 8 bis 10, wobei das Cholesterin von etwa 30 Mol-% bis etwa 40 Mol-% des gesamten Lipids, vorhanden in dem Teilchen, umfasst.

12. Lipidteilchen nach einem der Ansprüche 5 bis 11, wobei das PEG-Lipid von etwa 0,5 Mol-% bis etwa 2 Mol-% des gesamten Lipids, vorhanden in dem Teilchen, umfasst.

13. Lipidteilchen nach einem der Ansprüche 5 bis 12, wobei das Lipidteilchen einen elektronendichten Kern umfasst und wobei sich die mRNA innerhalb des elektronendichten Kerns befindet.

14. Lipidteilchen nach Anspruch 13, wobei der elektronendichte Kern eine wässrige Komponente und eine Lipidkomponente umfasst, wobei die Menge der wässrigen Komponente geringer als die Menge der Lipidkomponente ist.

15. Pharmazeutische Zusammensetzung, umfassend ein Lipidteilchen nach einem der Ansprüche 5 bis 14 und einen pharmazeutisch verträglichen Träger.

16. Lipidteilchen nach einem der Ansprüche 5 bis 14 zur Verwendung in einem Verfahren zum Einführen einer mRNA, die ein Protein codiert, in eine Zelle, wobei sich die Zelle in einem Säugetier befindet.

17. Lipidteilchen nach einem der Ansprüche 5 bis 14 zur Verwendung in einem Verfahren zur Behandlung und/oder Besserung eines oder mehrerer mit einer Krankheit assoziierter Symptome in einem Menschen, verursacht durch beeinträchtigte Expression eines Proteins in dem Menschen, wobei die mRNA, eingekapselt innerhalb des Lipidteilchens, das Protein codiert.

18. Lipidteilchen, umfassend (a) ein oder mehrere mRNA-Molekül(e); (b) eine Verbindung nach einem der Ansprüche 1 bis 3, umfassend von etwa 30 Mol-% bis etwa 50 Mol-% des gesamten Lipids, vorhanden in dem Teilchen; (c) ein Gemisch von einem Phospholipid und Cholesterin oder einem Derivat davon, umfassend von etwa 47 Mol-% bis etwa 69 Mol-% des gesamten Lipids, vorhanden in dem Teilchen; und (d) ein PEG-Lipid-Konjugat, umfassend von etwa 1 Mol-% bis etwa 3 Mol-% des gesamten Lipids, vorhanden in dem Teilchen.

## Revendications

1. Composé ayant la formule développée C :
X-A-Y-Z¹ ; (Formule C)
ou un sel de celui-ci, dans lequel :
X est -N(H)R ou -NR₂ ;
A est absent, un alkyle en C₁ à C₆, un alcényle en C₂ à C₆ ou un alcynyle en C₂ à C₆, lequel alkyle en C₁ à C₆, alcényle en C₂ à C₆ ou alcynyle en C₂ à C₆ est facultativement substitué par un ou plusieurs groupes indépendamment sélectionnés parmi les groupes oxo, halogène, hétérocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} et -SOₙNR^{x}R^{y}, dans lequel n est égal à 0, 1 ou 2, et R^{x} et R^{y} sont chacun indépendamment un hydrogène, un alkyle ou un hétérocycle, dans lequel chaque alkyle et hétérocycle de R^{x} et R^{y} peut être en outre substitué par un ou plusieurs groupes indépendamment sélectionnés parmi les groupes oxo, halogène, -OH, -CN, alkyle, -OR^{x'}, hétérocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} et -SO_{n'}NR^{x'}R^{y'}, dans lequel n' est égal à 0, 1 ou 2, et R^{x'} et R^{y'} sont chacun indépendamment un hydrogène, un alkyle ou un hétérocycle ;
Y est sélectionné parmi le groupe constitué par une absence, -C(=O)-, -O-, -OC(=O)-, -C(=O)O-, -N(R^{b})C(=O)-, -C(=O)N(R^{b})-, -N(R^{b})C(=O)O-, et -OC(=O)N(R^{b})- ;
Z¹ est un groupe alkyle en C₁ à C₆ qui est substitué par quatre groupes R^{x}, dans lequel chaque groupe R^{x} est indépendamment sélectionné parmi un alkyle en C₆ à C₁₁, un alcényle en C₆ à C₁₁ et un alcynyle en C₆ à C₁₁, lequel alkyle en C₆ à C₁₁, alcényle en C₆ à C₁₁ ou alcynyle en C₆ à C₁₁ est facultativement substitué par un ou plusieurs groupes indépendamment sélectionnés parmi les groupes oxo, halogène, hétérocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} et -SOₙNR^{x}R^{y}, dans lequel n est égal à 0, 1 ou 2, et R^{x} et R^{y} sont chacun indépendamment un hydrogène, un alkyle ou un hétérocycle, dans lequel tout alkyle et hétérocycle de R^{x} et R^{y} peut être en outre substitué par un ou plusieurs groupes indépendamment sélectionnés parmi les groupes oxo, halogène, -OH, -CN, alkyle, -OR^{x'}, hétérocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} et -SO_{n'}NR^{x'}R^{y'}, dans lequel n' est égal à 0, 1 ou 2, et R^{x'} et R^{y'} sont chacun indépendamment un hydrogène, un alkyle ou un hétérocycle ;
chaque R est indépendamment un alkyle, un alcényle ou un alcynyle, qui est facultativement substitué par un ou plusieurs groupes indépendamment sélectionnés parmi les groupes oxo, halogène, hétérocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} et -SOₙNR^{x}R^{y}, dans lequel n est égal à 0, 1 ou 2, et R^{x} et R^{y} sont chacun indépendamment un hydrogène, un alkyle ou un hétérocycle, dans lequel tout alkyle et hétérocycle de R^{x} et R^{y} peut être en outre substitué par un ou plusieurs groupes indépendamment sélectionnés parmi les groupes oxo, halogène, -OH, -CN, alkyle, -OR^{x'}, hétérocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} et -SO_{n'}NR^{x'}R^{y'}, dans lequel n' est égal à 0, 1 ou 2, et R^{x'} et R^{y'} sont chacun indépendamment un hydrogène, un alkyle ou un hétérocycle ; et
chaque R^{b} est H ou un alkyle en C₁ à C₆.

2. Composé selon la revendication 1, dans lequel Z¹ possède la structure : 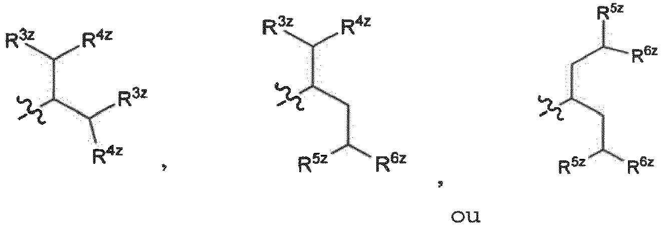
dans lequel chaque R^{3z}, R^{4z}, R^{5z} et R^{6z} est indépendamment sélectionné parmi un alkyle en C₆ à C₁₁, un alcényle en C₆ à C₁₁ et un alcynyle en C₆ à C₁₁, lequel alkyle en C₆ à C₁₁, alcényle en C₆ à C₁₁ et alcynyle en C₆ à C₁₁
est facultativement substitué par un ou plusieurs groupes indépendamment sélectionnés parmi les groupes oxo, halogène, hétérocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x} et -SOₙNR^{x}R^{y}, dans lequel n est égal à 0, 1 ou 2, et R^{x} et R^{y} sont chacun indépendamment un hydrogène, un alkyle ou un hétérocycle, dans lequel tout alkyle et hétérocycle de R^{x} et R^{y} peut être en outre substitué par un ou plusieurs groupes indépendamment sélectionnés parmi les groupes oxo, halogène, -OH, -CN, alkyle, -OR^{x'}, hétérocycle, -NR^{x'}R^{y'}, -NR^{x'}C(=O)R^{y'}, -NR^{x'}SO₂R^{y'}, -C(=O)R^{x'}, -C(=O)OR^{x'}, -C(=O)NR^{x'}R^{y'}, -SO_{n'}R^{x'} et -SO_{n'}NR^{x'}R^{y'}, dans lequel n' est égal à 0, 1 ou 2, et R^{x'} et R^{y'} sont chacun indépendamment un hydrogène, un alkyle ou un hétérocycle.

3. Composé selon la revendication 1 qui est sélectionné parmi le groupe constitué par : 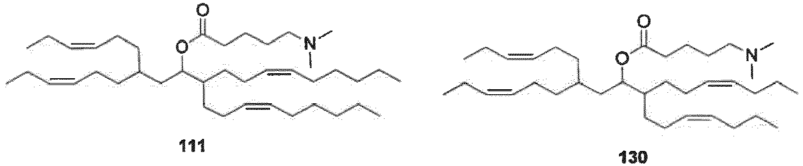 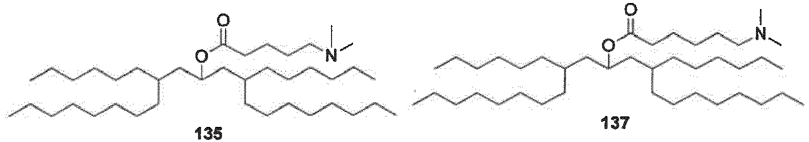 et

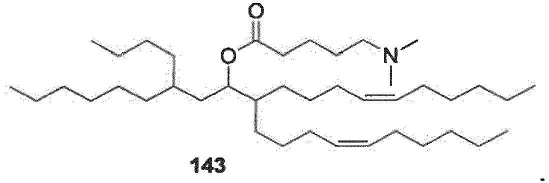

4. Particule lipidique comprenant un composé selon l'une quelconque des revendications 1 à 3, un lipide non cationique et une molécule d'ARNm qui est encapsulée à l'intérieur de la particule lipidique.

5. Particule lipidique selon la revendication 4, dans laquelle le lipide non cationique est sélectionné parmi un conjugué PEG-lipide et un phospholipide.

6. Particule lipidique selon la revendication 4 ou la revendication 5, dans laquelle la particule lipidique comprend en outre du cholestérol.

7. Particule lipidique selon la revendication 5, dans laquelle le phospholipide comprend la dipalmitoylphosphatidylcholine (DPPC), la distéaroylphosphatidylcholine (DSPC) ou un mélange de celles-ci ; et dans laquelle le conjugué PEG-lipide est sélectionné parmi le groupe constitué par un conjugué PEG-diacylglycérol (PEG-DAG), un conjugué PEG-dialkyloxypropyle (PEG-DAA), un conjugué PEG-phospholipide, un conjugué PEG-céramide (PEG-Cer), un mélange de ceux-ci, le PEG-DMA et le PEG-DSA.

8. Particule lipidique selon l'une quelconque des revendications 4 à 7, dans laquelle la particule possède un rapport massique lipide:ARNm d'environ 9:1 à environ 20:1.

9. Particule lipidique selon l'une quelconque des revendications 4 à 6, dans laquelle le composé selon l'une quelconque des revendications 1 à 4 comprend d'environ 50 % en moles à environ 65 % en moles des lipides totaux présents dans la particule.

10. Particule lipidique selon l'une quelconque des revendications 5 à 9, dans laquelle le phospholipide comprend d'environ 4 % en moles à environ 15 % en moles des lipides totaux présents dans la particule.

11. Particule lipidique selon l'une quelconque des revendications 6, et 8 à 10, dans laquelle le cholestérol comprend d'environ 30 % en moles à environ 40 % en moles des lipides totaux présents dans la particule.

12. Particule lipidique selon l'une quelconque des revendications 5 à 11, dans laquelle le PEG-lipide comprend d'environ 0,5 % en moles à environ 2 % en moles des lipides totaux présents dans la particule.

13. Particule lipidique selon l'une quelconque des revendications 5 à 12, dans laquelle la particule lipidique comprend un cœur dense en électrons et dans laquelle l'ARNm est situé à l'intérieur du cœur dense en électrons.

14. Particule lipidique selon la revendication 13, dans laquelle le cœur dense en électrons comprend un constituant aqueux et un constituant lipidique, dans laquelle la quantité du constituant aqueux est inférieure à la quantité du constituant lipidique.

15. Composition pharmaceutique comprenant une particule lipidique selon l'une quelconque des revendications 5 à 14 et un vecteur pharmaceutiquement acceptable.

16. Particule lipidique selon l'une quelconque des revendications 5 à 14 pour une utilisation dans un procédé d'introduction d'un ARNm qui code pour une protéine dans une cellule, dans laquelle la cellule est au sein d'un mammifère.

17. Particule lipidique selon l'une quelconque des revendications 5 à 14, pour une utilisation dans un procédé de traitement et/ou d'amélioration d'un ou de plusieurs symptômes associés à une maladie, chez un être humain, provoquée par l'expression déficiente d'une protéine chez l'être humain, dans laquelle l'ARNm encapsulé à l'intérieur de la particule lipidique code pour la protéine.

18. Particule lipidique comprenant (a) une ou plusieurs molécules d'ARNm ; (b) un composé selon l'une quelconque des revendications 1 à 3 comprenant d'environ 30 % en moles à environ 50 % en moles des lipides totaux présents dans la particule ; (c) un mélange d'un phospholipide et de cholestérol ou d'un dérivé de ceux-ci comprenant d'environ 47 % en moles à environ 69 % en moles des lipides totaux présents dans la particule ; et (d) un conjugué PEG-lipide comprenant d'environ 1 % en moles à environ 3 % en moles des lipides totaux présents dans la particule.
